(19) Europäisches Patentamt
European Patent Office
Office européen des brevets

(11) EP 3 936 150 A1

(12) EUROPEAN PATENT APPLICATION
published in accordance with Art. 153(4) EPC

(43) Date of publication:
**12.01.2022 Bulletin 2022/02**

(21) Application number: **20765639.8**

(22) Date of filing: **05.03.2020**

(51) Int Cl.:
*A61K 47/68* (2017.01) *A61K 47/65* (2017.01)
*A61K 31/5517* (2006.01) *A61P 35/00* (2006.01)
*C07K 16/28* (2006.01) *A61K 45/06* (2006.01)

(86) International application number:
**PCT/KR2020/003100**

(87) International publication number:
**WO 2020/180121 (10.09.2020 Gazette 2020/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.03.2019 KR 20190025987**
**04.03.2020 KR 20200027373**

(71) Applicants:
- **LegoChem Biosciences, Inc.**
  **Daejeon 34302 (KR)**
- **Y-Biologics Inc.**
  **Daejeon 34014 (KR)**

(72) Inventors:
- **PARK, Chang Sik**
  **Daejeon 34302 (KR)**
- **CHOI, Min Ji**
  **Daejeon 34302 (KR)**
- **JANG, Tae Ik**
  **Daejeon 34302 (KR)**
- **PARK, Yun-Hee**
  **Daejeon 34302 (KR)**
- **SONG, Ho Young**
  **Daejeon 34302 (KR)**
- **BAEK, Juyuel**
  **Daejeon 34302 (KR)**
- **KIM, Sung Min**
  **Daejeon 34302 (KR)**
- **LEE, Hyeun Joung**
  **Daejeon 34302 (KR)**
- **LEE, Ju Young**
  **Daejeon 34302 (KR)**
- **KIM, Hyoung Rae**
  **Daejeon 34302 (KR)**
- **LEE, Kun Jung**
  **Daejeon 34302 (KR)**
- **KIM, Yong Zu**
  **Daejeon 34302 (KR)**
- **LEE, Chang Sun**
  **Daejeon 34302 (KR)**
- **CHAE, Jeiwook**
  **Daejeon 34302 (KR)**
- **LEE, Sang Pil**
  **Daejeon 34014 (KR)**
- **SHIN, Ji-young**
  **Daejeon 34014 (KR)**
- **YOON, Sunha**
  **Daejeon 34014 (KR)**
- **CHOI, Yunseon**
  **Daejeon 34014 (KR)**
- **PARK, Jae Eun**
  **Daejeon 34014 (KR)**
- **LEE, Jisu**
  **Daejeon 34014 (KR)**
- **PARK, Bum-Chan**
  **Daejeon 34014 (KR)**
- **PARK, Young Woo**
  **Daejeon 34014 (KR)**

(74) Representative: **Vossius & Partner**
**Patentanwälte Rechtsanwälte mbB**
**Siebertstraße 3**
**81675 München (DE)**

# (54) ANTIBODY-DRUG CONJUGATES INCLUDING ANTIBODY AGAINST HUMAN DLK1, AND USE THEREOF

(57) The present invention relates to new antibody-drug conjugates (ADCs) targeting DLK1, active metabolites of such ADCs, methods for preparation of such ADCs, uses for such ADCs in treatment and/or prevention of illnesses, and uses for such ADCs in production of drugs for treatment and/or prevention of diseases, more specifically, uses for such ADCs in producing drugs for treatment and/or prevention of proliferative and/or angiogenetic diseases, for example, cancer. More particularly, the present invention relates to an antibody-drug conjugate comprising an antibody binding with DLK1 or an antigen-binding fragment thereof, and a pharmaceutical composition comprising the same.



(Cont. next page)

FIG. 1

**CTG-0199, SCLC *PDX* model (IV administration)**

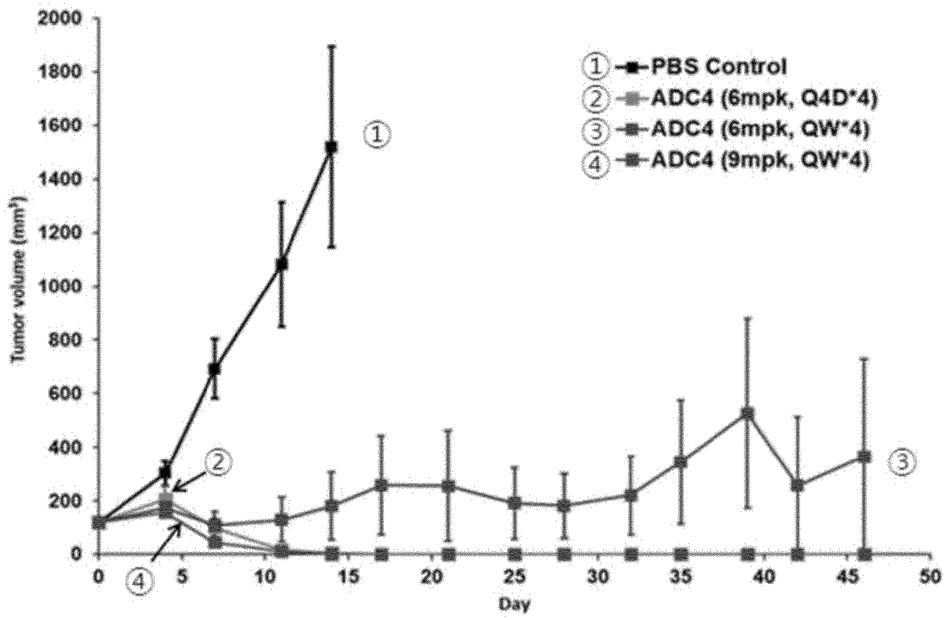


| Feature | Linker-toxin (DAR) | DAR |
|---|---|---|
| ADC4 | LBG*-MMAE | DAR 4 |

- Athymic Nude-Foxn1nu mice, subcu, n=8/group
- IV injection, repeat dose administration
- The graph shows the mean tumor size and SEM.

* LBG: LCB's proprietary BG linker

| Group | PR | CR |
|---|---|---|
| ADC4, 6mpk/Q4D*4 | 0/8 | 8/8 |
| ADC4, 6mpk/QW*4 | 0/8 | 6/8 |
| ADC4, 9mpk/QW*4 | 0/8 | 8/8 |

- PR : partial remission defined as 50~100% tumor regression
- CR : complete remission

- **14 days after IV administration, all treatment groups showed significant tumor growth inhibition or complete remission (CR)**

<110> legochembioscience
ybiologics

<120> Antibody-drug conjugate comprising antibody binding to antibody against human DLK1 and its use

<130> PCT2020-005

<150> KR 10-2019-0025987
<151> 2019-03-06

<150> KR 10-2020-0027373
<151> 2020-03-04

<160> 128

<170> KoPatentIn 3.0

<210> 1
<211> 25
<212> PRT
<213> Artificial Sequence

<220>
<223> Artificial Sequence

<400> 1
Gln Val Gln Leu Val Glu Ser Gly Gly Thr Leu Val Gln Pro Gly Gly
1 5 10 15

Ser Leu Arg Leu Ser Cys Ala Ala Ser
20 25

<210> 2
<211> 8
<212> PRT
<213> Artificial Sequence

<220>
<223> Artificial Sequence

<400> 2
Gly Phe Thr Phe Ser Ser His Ala
1 5

<210> 3
<211> 17
<212> PRT
<213> Artificial Sequence

<220>
<223> Artificial Sequence

<400> 3
Met Ser Trp Val Arg Gln Thr Pro Gly Lys Gly Leu Glu Trp Val Ser
1 5 10 15

Ser

<210> 4
<211> 8
<212> PRT
<213> Artificial Sequence

<220>
<223> Artificial Sequence

<400> 4
Ile Thr Lys Ser Gly Ser Gly Thr
1 5

<210> 5
<211> 38
<212> PRT
<213> Artificial Sequence

<220>
<223> Artificial Sequence

<400> 5
Tyr Ser Ala Asp Ser Val Lys Gly Arg Phe Thr Ile Ser Arg Asp Asn
1 5 10 15

Ser Lys Asn Thr Leu Tyr Leu Gln Met Asn Ser Leu Arg Ala Glu Asp
20 25 30

Thr Ala Val Tyr Tyr Cys
35

<210> 6
<211> 13
<212> PRT
<213> Artificial Sequence

<220>
<223> Artificial Sequence

<400> 6
Thr Arg Glu Gly Leu Gly Tyr Tyr Tyr Gly Met Asp Val
1 5 10

<210> 7
<211> 11
<212> PRT
<213> Artificial Sequence

<220>
<223> Artificial Sequence

<400> 7
Trp Gly Gln Gly Thr Thr Val Thr Val Ser Ser
1 5 10

<210> 8
<211> 25
<212> PRT
<213> Artificial Sequence

<220>
<223> Artificial Sequence

<400> 8
Gln Leu Val Leu Thr Gln Pro Pro Ser Val Ser Gly Ala Pro Gly Gln
1 5 10 15

Arg Val Ile Ile Ser Cys Thr Gly Ser
20 25

<210> 9
<211> 9
<212> PRT
<213> Artificial Sequence

<220>
<223> Artificial Sequence

<400> 9
Ser Ser Asn Ile Gly Ala Gly Tyr Asp
1 5

<210> 10
<211> 17
<212> PRT
<213> Artificial Sequence

<220>
<223> Artificial Sequence

<400> 10
Val His Trp Tyr Gln Gln Leu Pro Gly Thr Ala Pro Arg Leu Leu Ile
1 5 10 15

Tyr

<210> 11
<211> 3
<212> PRT
<213> Artificial Sequence

<220>
<223> Artificial Sequence

<400> 11
Gly Ser Thr
1

<210> 12
<211> 36
<212> PRT
<213> Artificial Sequence

<220>
<223> Artificial Sequence

<400> 12
Asn Arg Pro Ser Gly Val Pro Asp Arg Phe Ser Gly Ser Lys Ser Gly
1 5 10 15

Thr Ser Ala Ser Leu Ala Ile Thr Gly Leu Gln Ala Glu Asp Glu Ala
20 25 30

Asp Tyr Tyr Cys
35

<210> 13
<211> 12
<212> PRT
<213> Artificial Sequence

<220>
<223> Artificial Sequence

<400> 13
Gln Ser Tyr Asp Asn Ser Leu Ser Ala His Tyr Val
1 5 10

<210> 14
<211> 10
<212> PRT
<213> Artificial Sequence

<220>
<223> Artificial Sequence

<400> 14
Phe Gly Thr Gly Thr Lys Val Thr Val Leu
1 5 10

<210> 15
<211> 25
<212> PRT
<213> Artificial Sequence

<220>
<223> Artificial Sequence

<400> 15
Gln Val Gln Leu Val Gln Ser Gly Gly Ala Val Val Gln Pro Gly His
1 5 10 15

Ser Leu Arg Leu Ser Cys Glu Ala Ser

20 25

<210> 16
<211> 8
<212> PRT
<213> Artificial Sequence

<220>
<223> Artificial Sequence

<400> 16
Gly Phe Lys Phe Lys Asp Tyr Gly
1 5

<210> 17
<211> 17
<212> PRT
<213> Artificial Sequence

<220>
<223> Artificial Sequence

<400> 17
Met His Trp Val Arg Gln Ala Pro Gly Lys Gly Leu Glu Trp Leu Ala
1 5 10 15

Val

<210> 18
<211> 8
<212> PRT
<213> Artificial Sequence

<220>
<223> Artificial Sequence

<400> 18
Ile Ser His Asp Gly Arg Asn Lys
1 5

<210> 19
<211> 38
<212> PRT
<213> Artificial Sequence

<220>
<223> Artificial Sequence

<400> 19
Asn Tyr Ala Asp Ser Val Lys Gly Arg Leu Thr Ile Ser Arg Asp Asn
1 5 10 15

Ser Lys Asn Thr Leu Ser Phe Gln Met Asn Ser Leu Arg Ala Glu Asp
20 25 30

Thr Ala Val Tyr Tyr Cys
35

<210> 20
<211> 10
<212> PRT
<213> Artificial Sequence

<220>
<223> Artificial Sequence

<400> 20
Val Arg Asp Trp Ser Tyr Ala Phe Asp Ile
1 5 10

<210> 21
<211> 11
<212> PRT
<213> Artificial Sequence

<220>
<223> Artificial Sequence

<400> 21
Trp Gly Gln Gly Thr Leu Val Thr Val Ser Ser
1 5 10

<210> 22
<211> 26
<212> PRT
<213> Artificial Sequence

<220>
<223> Artificial Sequence

<400> 22
Asp Ile Gln Met Thr Gln Ser Pro Ser Phe Leu Ser Ala Ser Val Gly
1 5 10 15

Asp Arg Val Asn Ile Thr Cys Arg Ala Ser
20 25

<210> 23
<211> 6
<212> PRT
<213> Artificial Sequence

<220>
<223> Artificial Sequence

<400> 23
Gln Asp Ile Ser Arg Arg
1 5

<210> 24
<211> 17
<212> PRT
<213> Artificial Sequence

<220>
<223> Artificial Sequence

<400> 24
Leu Ala Trp Tyr Gln Gln Lys Pro Gly Lys Ala Pro Lys Leu Leu Ile
1 5 10 15

Tyr

<210> 25
<211> 3
<212> PRT
<213> Artificial Sequence

<220>
<223> Artificial Sequence

<400> 25
Gly Ala Ala
1

<210> 26
<211> 36
<212> PRT
<213> Artificial Sequence

<220>
<223> Artificial Sequence

<400> 26
Ser Leu Gln Ser Ala Val Ala Ser Arg Phe Ser Gly Ser Gly Ser Gly
1 5 10 15

Thr Glu Phe Thr Leu Thr Ile Ser Ser Leu Gln Pro Glu Asp Phe Ala
20 25 30

Asn Tyr Tyr Cys
35

<210> 27
<211> 9
<212> PRT
<213> Artificial Sequence

<220>
<223> Artificial Sequence

<400> 27
Gln Gln Ile Tyr Thr Thr Pro Leu Thr

1 5

<210> 28
<211> 10
<212> PRT
<213> Artificial Sequence

<220>
<223> Artificial Sequence

<400> 28
Phe Gly Gly Gly Thr Lys Val Glu Ile Lys
1 5 10

<210> 29
<211> 25
<212> PRT
<213> Artificial Sequence

<220>
<223> Artificial Sequence

<400> 29
Gln Met Gln Leu Val Gln Ser Gly Gly Gly Leu Val Gln Pro Gly Arg
1 5 10 15

Ser Leu Arg Leu Ser Cys Ala Ala Ser
20 25

<210> 30
<211> 8
<212> PRT
<213> Artificial Sequence

<220>
<223> Artificial Sequence

<400> 30
Gly Phe Thr Phe Asp Asp Tyr Ala
1 5

<210> 31
<211> 17
<212> PRT
<213> Artificial Sequence

<220>
<223> Artificial Sequence

<400> 31
Met His Trp Val Arg Gln Ala Pro Gly Lys Gly Leu Glu Trp Val Ser
1 5 10 15

Gly

<210> 32
<211> 8
<212> PRT
<213> Artificial Sequence

<220>
<223> Artificial Sequence

<400> 32
Ile Ser Trp Asn Ser Gly Ser Ile
1 5

<210> 33
<211> 38
<212> PRT
<213> Artificial Sequence

<220>
<223> Artificial Sequence

<400> 33
Gly Tyr Ala Asp Ser Val Lys Gly Arg Phe Thr Ile Ser Arg Asp Asn
1 5 10 15

Ala Lys Asn Ser Leu Tyr Leu Gln Met Asn Ser Leu Arg Ala Glu Asp
20 25 30

Thr Ala Val Tyr Tyr Cys
35

<210> 34
<211> 15
<212> PRT
<213> Artificial Sequence

<220>
<223> Artificial Sequence

<400> 34
Thr Lys Gly Pro Gly Leu Ala Thr Gly Lys Val Tyr Phe Asn Ser
1 5 10 15

<210> 35
<211> 11
<212> PRT
<213> Artificial Sequence

<220>
<223> Artificial Sequence

<400> 35
Trp Gly Gln Gly Thr Gln Val Thr Val Ser Ser
1 5 10

<210> 36
<211> 26
<212> PRT
<213> Artificial Sequence

<220>
<223> Artificial Sequence

<400> 36
Asp Ile Gln Met Thr Gln Ser Pro Ser Ser Val Ser Ala Ser Val Gly
1 5 10 15

Asp Arg Val Thr Ile Thr Cys Arg Ala Ser
20 25

<210> 37
<211> 6
<212> PRT
<213> Artificial Sequence

<220>
<223> Artificial Sequence

<400> 37
Gln Arg Ile Ser Ser Trp
1 5

<210> 38
<211> 17
<212> PRT
<213> Artificial Sequence

<220>
<223> Artificial Sequence

<400> 38
Leu Ala Trp Tyr Gln Gln Lys Pro Gly Arg Ala Pro Lys Leu Leu Ile
1 5 10 15

His

<210> 39
<211> 3
<212> PRT
<213> Artificial Sequence

<220>
<223> Artificial Sequence

<400> 39
Ser Ala Ser
1

<210> 40
<211> 36
<212> PRT
<213> Artificial Sequence

<220>
<223> Artificial Sequence

<400> 40
Thr Leu His Asn Gly Val Pro Ser Arg Phe Ser Gly Ser Ala Ser Gly
1 5 10 15

Thr Asp Phe Thr Leu Thr Ile Ser Ser Leu Gln Pro Glu Asp Phe Ala
20 25 30

Ile Tyr Tyr Cys
35

<210> 41
<211> 9
<212> PRT
<213> Artificial Sequence

<220>
<223> Artificial Sequence

<400> 41
Gln Gln Gly His Ser Phe Pro Tyr Thr
1 5

<210> 42
<211> 10
<212> PRT
<213> Artificial Sequence

<220>
<223> Artificial Sequence

<400> 42
Phe Gly Gln Gly Thr Lys Leu Asp Ile Lys
1 5 10

<210> 43
<211> 25
<212> PRT
<213> Artificial Sequence

<220>
<223> Artificial Sequence

<400> 43
Gln Val Gln Leu Val Glu Ser Gly Gly Gly Leu Val Gln Pro Gly Gly
1 5 10 15

Ser Leu Arg Leu Ser Cys Ala Ala Ser
20 25

<210> 44
<211> 8
<212> PRT
<213> Artificial Sequence

<220>
<223> Artificial Sequence

<400> 44
Gly Phe Thr Phe Ser Ser Tyr Trp
1 5

<210> 45
<211> 17
<212> PRT
<213> Artificial Sequence

<220>
<223> Artificial Sequence

<400> 45
Met Asn Trp Val Arg Gln Ala Pro Gly Lys Gly Leu Val Trp Val Ser
1 5 10 15

Arg

<210> 46
<211> 8
<212> PRT
<213> Artificial Sequence

<220>
<223> Artificial Sequence

<400> 46
Ile Ser Pro Asp Gly Ser Ser Thr
1 5

<210> 47
<211> 38
<212> PRT
<213> Artificial Sequence

<220>
<223> Artificial Sequence

<400> 47
Thr Tyr Ala Asp Ser Val Lys Gly Arg Phe Thr Ile Ser Arg Asp Asn
1 5 10 15

Ala Lys Asn Thr Leu Tyr Leu Gln Met Asn Ser Leu Arg Ala Glu Asp
20 25 30

Thr Ala Val Tyr Tyr Cys
35

<210> 48
<211> 15
<212> PRT
<213> Artificial Sequence

<220>
<223> Artificial Sequence

<400> 48
Ala Arg Gly Tyr Ser Pro Lys Tyr Pro Thr Val Gly Leu Asp Val
1 5 10 15

<210> 49
<211> 11
<212> PRT
<213> Artificial Sequence

<220>
<223> Artificial Sequence

<400> 49
Trp Gly Gln Gly Thr Thr Ile Thr Val Ser Ser
1 5 10

<210> 50
<211> 26
<212> PRT
<213> Artificial Sequence

<220>
<223> Artificial Sequence

<400> 50
Asp Ile Val Met Thr Gln Ser Pro Leu Ser Ser Pro Val Thr Leu Gly
1 5 10 15

Gln Pro Ala Ser Ile Ser Cys Arg Ser Ser
20 25

<210> 51
<211> 11
<212> PRT
<213> Artificial Sequence

<220>
<223> Artificial Sequence

<400> 51
Glu Ser Leu Leu His Ser Asn Gly Asn Thr Tyr
1 5 10

<210> 52
<211> 17
<212> PRT
<213> Artificial Sequence

<220>
<223> Artificial Sequence

<400> 52
Leu Thr Trp Leu Gln Gln Arg Pro Gly Gln Pro Pro Arg Leu Leu Ile
1 5 10 15

His

<210> 53
<211> 3
<212> PRT
<213> Artificial Sequence

<220>
<223> Artificial Sequence

<400> 53
Lys Ile Ser
1

<210> 54
<211> 36
<212> PRT
<213> Artificial Sequence

<220>
<223> Artificial Sequence

<400> 54
Asn Arg Phe Ser Gly Val Pro Asp Arg Phe Ser Gly Ser Gly Ala Gly
1 5 10 15

Thr Asp Phe Thr Leu Gln Ile Thr Arg Val Glu Thr Glu Asp Val Gly
20 25 30

Val Tyr Tyr Cys
35

<210> 55
<211> 9
<212> PRT
<213> Artificial Sequence

<220>
<223> Artificial Sequence

<400> 55
Val Gln Thr Thr Gln Trp Pro Trp Thr
1 5

<210> 56
<211> 10
<212> PRT
<213> Artificial Sequence

<220>
<223> Artificial Sequence

<400> 56
Phe Gly Gln Gly Thr Lys Val Glu Ile Lys
1 5 10

<210> 57
<211> 25
<212> PRT
<213> Artificial Sequence

<220>
<223> Artificial Sequence

<400> 57
Gln Val Gln Leu Val Gln Ser Gly Ala Glu Val Lys Lys Pro Gly Ala
1 5 10 15

Ser Val Arg Val Ser Cys Lys Val Ser
20 25

<210> 58
<211> 8
<212> PRT
<213> Artificial Sequence

<220>
<223> Artificial Sequence

<400> 58
Gly Tyr Ser Leu Ser Glu Phe Pro
1 5

<210> 59
<211> 17
<212> PRT
<213> Artificial Sequence

<220>
<223> Artificial Sequence

<400> 59
Ile His Trp Val Arg Gln Ala Pro Arg Met Gly Leu Glu Trp Met Gly
1 5 10 15

Gly

<210> 60
<211> 8
<212> PRT
<213> Artificial Sequence

<220>
<223> Artificial Sequence

<400> 60
Ser Tyr Pro Glu Asp Gly Glu Thr
1 5

<210> 61
<211> 38
<212> PRT
<213> Artificial Sequence

<220>
<223> Artificial Sequence

<400> 61
Leu Tyr Ala Gln Lys Phe Gln Gly Arg Val Thr Met Thr Glu Asp Thr
1 5 10 15

Ser Thr Asp Thr Ala Tyr Met Glu Leu Ser Ser Leu Arg Ser Glu Asp
20 25 30

Thr Ala Val Tyr Tyr Cys
35

<210> 62
<211> 18
<212> PRT
<213> Artificial Sequence

<220>
<223> Artificial Sequence

<400> 62
Ala Arg Leu Asn Tyr Phe Glu Ser Thr Asp Tyr Trp Val Asp Ala Phe
1 5 10 15

Asp Ile

<210> 63
<211> 11
<212> PRT
<213> Artificial Sequence

<220>
<223> Artificial Sequence

<400> 63
Trp Gly Gln Gly Thr Met Val Thr Val Ser Ser

1 5 10

<210> 64
<211> 25
<212> PRT
<213> Artificial Sequence

<220>
<223> Artificial Sequence

<400> 64
Gln Leu Val Leu Thr Gln Pro Tyr Ser Val Ser Glu Ser Pro Gly Lys
1 5 10 15

Thr Ile Thr Ile Ser Cys Thr Arg Ser
20 25

<210> 65
<211> 8
<212> PRT
<213> Artificial Sequence

<220>
<223> Artificial Sequence

<400> 65
Ser Gly Ser Ile Ala Ser Asn Phe
1 5

<210> 66
<211> 17
<212> PRT
<213> Artificial Sequence

<220>
<223> Artificial Sequence

<400> 66
Val Gln Trp Tyr Gln Gln Arg Pro Gly Ser Ala Pro Thr Pro Val Ile
1 5 10 15

Tyr

<210> 67
<211> 3
<212> PRT
<213> Artificial Sequence

<220>
<223> Artificial Sequence

<400> 67
Glu Asp Asn
1

<210> 68
<211> 38
<212> PRT
<213> Artificial Sequence

<220>
<223> Artificial Sequence

<400> 68
Gln Arg Pro Ser Gly Val Pro Asp Arg Phe Ser Gly Ser Ile Asp Ser
1 5 10 15

Ser Ser Asn Ser Ala Ser Leu Thr Ile Ser Gly Val Met Thr Glu Asp
20 25 30

Glu Ala Asp Tyr Tyr Cys
35

<210> 69
<211> 10
<212> PRT
<213> Artificial Sequence

<220>
<223> Artificial Sequence

<400> 69
Gln Ser Tyr Asp Ser Gly Ser Ser Trp Val
1 5 10

<210> 70
<211> 10
<212> PRT
<213> Artificial Sequence

<220>
<223> Artificial Sequence

<400> 70
Phe Gly Gly Gly Thr Lys Leu Thr Val Leu
1 5 10

<210> 71
<211> 25
<212> PRT
<213> Artificial Sequence

<220>
<223> Artificial Sequence

<400> 71
Gln Met Gln Leu Val Glu Ser Gly Gly Gly Leu Val Lys Pro Gly Gly
1 5 10 15

Ser Leu Thr Leu Ser Cys Asp Ala Thr
20 25

<210> 72
<211> 8
<212> PRT
<213> Artificial Sequence

<220>
<223> Artificial Sequence

<400> 72
Gly Phe Asn Phe Gly Ser Tyr Tyr
1 5

<210> 73
<211> 17
<212> PRT
<213> Artificial Sequence

<220>
<223> Artificial Sequence

<400> 73
Met Asn Trp Val Arg Gln Ala Pro Gly Lys Gly Leu Glu Trp Leu Ala
1 5 10 15

His

<210> 74
<211> 8
<212> PRT
<213> Artificial Sequence

<220>
<223> Artificial Sequence

<400> 74
Ile Ser Ser Thr Gly Arg Thr Ile
1 5

<210> 75
<211> 38
<212> PRT
<213> Artificial Sequence

<220>
<223> Artificial Sequence

<400> 75
Tyr Tyr Ala Asp Ser Val Lys Gly Arg Phe Thr Ile Ser Arg Asp Asn
1 5 10 15

Ala Lys Ser Ser Leu Asp Leu Gln Met Asn Ser Leu Arg Ala Glu Asp

20 25 30

Thr Ala Val Tyr Tyr Cys
35

<210> 76
<211> 12
<212> PRT
<213> Artificial Sequence

<220>
<223> Artificial Sequence

<400> 76
Ala Arg Asp Gln Gly Tyr Pro Phe Gly Met Asp Val
1 5 10

<210> 77
<211> 11
<212> PRT
<213> Artificial Sequence

<220>
<223> Artificial Sequence

<400> 77
Trp Gly His Gly Thr Thr Val Thr Val Ser Ser
1 5 10

<210> 78
<211> 25
<212> PRT
<213> Artificial Sequence

<220>
<223> Artificial Sequence

<400> 78
Gln Leu Val Leu Thr Gln Pro Ser Ser Val Ser Gly Ala Pro Gly Gln
1 5 10 15

Arg Val Thr Ile Ser Cys Thr Gly Ser
20 25

<210> 79
<211> 9
<212> PRT
<213> Artificial Sequence

<220>
<223> Artificial Sequence

<400> 79
Ser Ser Asn Ile Gly Ala Gly Tyr Asp
1 5

<210> 80
<211> 17
<212> PRT
<213> Artificial Sequence

<220>
<223> Artificial Sequence

<400> 80
Val Asp Trp Tyr Gln Gln Leu Pro Gly Thr Ala Pro Lys Leu Leu Ile
1 5 10 15

Tyr

<210> 81
<211> 3
<212> PRT
<213> Artificial Sequence

<220>
<223> Artificial Sequence

<400> 81
Gly Asn Thr
1

<210> 82
<211> 36
<212> PRT
<213> Artificial Sequence

<220>
<223> Artificial Sequence

<400> 82
Asn Arg Pro Ser Gly Val Pro Asp Arg Phe Ser Gly Ser Lys Ser Gly
1 5 10 15

Thr Ser Ala Ser Leu Ala Ile Thr Gly Leu Gln Ala Glu Asp Asp Ser
20 25 30

Asp Tyr Tyr Cys
35

<210> 83
<211> 11
<212> PRT
<213> Artificial Sequence

<220>
<223> Artificial Sequence

<400> 83

Gln Ser Tyr Asp Ser Ser Leu Ser Ala Trp Val
1 5 10

<210> 84
<211> 10
<212> PRT
<213> Artificial Sequence

<220>
<223> Artificial Sequence

<400> 84
Phe Gly Gly Gly Thr Lys Leu Thr Val Leu
1 5 10

<210> 85
<211> 25
<212> PRT
<213> Artificial Sequence

<220>
<223> Artificial Sequence

<400> 85
Gln Val Gln Leu Val Glu Ser Gly Gly Gly Val Val Gln Pro Gly Arg
1 5 10 15

Ser Leu Arg Leu Ser Cys Ala Ala Ser
20 25

<210> 86
<211> 8
<212> PRT
<213> Artificial Sequence

<220>
<223> Artificial Sequence

<400> 86
Gly Phe Thr Phe Ser Ser Tyr Ala
1 5

<210> 87
<211> 17
<212> PRT
<213> Artificial Sequence

<220>
<223> Artificial Sequence

<400> 87
Met His Trp Val Arg Gln Ala Pro Gly Lys Gly Leu Glu Trp Val Ala
1 5 10 15

Val

<210> 88
<211> 7
<212> PRT
<213> Artificial Sequence

<220>
<223> Artificial Sequence

<400> 88
Ile Tyr Ser Gly Gly Ser Thr
1 5

<210> 89
<211> 38
<212> PRT
<213> Artificial Sequence

<220>
<223> Artificial Sequence

<400> 89
Tyr Tyr Ala Asp Ser Val Lys Gly Arg Phe Thr Ile Ser Arg Asp Asn
1 5 10 15

Ser Lys Asn Thr Leu Tyr Leu Gln Met Asn Ser Leu Arg Ala Glu Asp
20 25 30

Thr Ala Val Tyr Tyr Cys
35

<210> 90
<211> 20
<212> PRT
<213> Artificial Sequence

<220>
<223> Artificial Sequence

<400> 90
Ala Arg Glu Gly Ser Tyr Asp Val Met Thr Tyr Thr Arg Ile Gly Gly
1 5 10 15

Tyr Phe Asp Tyr
20

<210> 91
<211> 11
<212> PRT
<213> Artificial Sequence

<220>
<223> Artificial Sequence

<400> 91
Trp Gly Gln Gly Ala Leu Val Thr Val Ser Ser
1 5 10

<210> 92
<211> 26
<212> PRT
<213> Artificial Sequence

<220>
<223> Artificial Sequence

<400> 92
Asp Ile Gln Met Thr Gln Ser Pro Ser Ser Val Ser Ala Ser Val Gly
1 5 10 15

Asp Arg Val Thr Ile Thr Cys Arg Ala Ser
20 25

<210> 93
<211> 6
<212> PRT
<213> Artificial Sequence

<220>
<223> Artificial Sequence

<400> 93
Gln Gly Ile Ser Asp Trp
1 5

<210> 94
<211> 17
<212> PRT
<213> Artificial Sequence

<220>
<223> Artificial Sequence

<400> 94
Val Ala Trp Tyr Gln Gln Lys Pro Gly Lys Ala Pro Lys Leu Leu Ile
1 5 10 15

Tyr

<210> 95
<211> 3
<212> PRT
<213> Artificial Sequence

<220>
<223> Artificial Sequence

<400> 95

```
Ala Ala Ser
  1


<210>    96
<211>    36
<212>    PRT
<213>    Artificial Sequence

<220>
<223>    Artificial Sequence


<400>    96
Ser Leu Gln Ser Gly Val Pro Ser Arg Phe Ser Gly Ser Gly Ser Gly
  1               5                  10                  15

Thr Glu Phe Ser Leu Thr Ile Ser Asn Leu Gln Pro Glu Asp Phe Ala
             20                  25                  30

Thr Tyr Tyr Cys
         35


<210>    97
<211>    9
<212>    PRT
<213>    Artificial Sequence

<220>
<223>    Artificial Sequence


<400>    97
Gln Gln Ala Asn Ser Phe Pro Leu Thr
  1               5


<210>    98
<211>    10
<212>    PRT
<213>    Artificial Sequence

<220>
<223>    Artificial Sequence


<400>    98
Phe Gly Pro Gly Thr Lys Val Glu Ile Lys
  1               5                  10


<210>    99
<211>    120
<212>    PRT
<213>    Artificial Sequence

<220>
<223>    Artificial Sequence


<400>    99
Gln Val Gln Leu Val Glu Ser Gly Gly Thr Leu Val Gln Pro Gly Gly
```

1 5 10 15

Ser Leu Arg Leu Ser Cys Ala Ala Ser Gly Phe Thr Phe Ser Ser His
20 25 30

Ala Met Ser Trp Val Arg Gln Thr Pro Gly Lys Gly Leu Glu Trp Val
35 40 45

Ser Ser Ile Thr Lys Ser Gly Ser Gly Thr Tyr Ser Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Thr Arg Glu Gly Leu Gly Tyr Tyr Tyr Gly Met Asp Val Trp Gly Gln
100 105 110

Gly Thr Thr Val Thr Val Ser Ser
115 120

<210> 100
<211> 112
<212> PRT
<213> Artificial Sequence

<220>
<223> Artificial Sequence

<400> 100
Gln Leu Val Leu Thr Gln Pro Pro Ser Val Ser Gly Ala Pro Gly Gln
1 5 10 15

Arg Val Ile Ile Ser Cys Thr Gly Ser Ser Ser Asn Ile Gly Ala Gly
20 25 30

Tyr Asp Val His Trp Tyr Gln Gln Leu Pro Gly Thr Ala Pro Arg Leu
35 40 45

Leu Ile Tyr Gly Ser Thr Asn Arg Pro Ser Gly Val Pro Asp Arg Phe
50 55 60

Ser Gly Ser Lys Ser Gly Thr Ser Ala Ser Leu Ala Ile Thr Gly Leu
65 70 75 80

Gln Ala Glu Asp Glu Ala Asp Tyr Tyr Cys Gln Ser Tyr Asp Asn Ser
85 90 95

Leu Ser Ala His Tyr Val Phe Gly Thr Gly Thr Lys Val Thr Val Leu
100 105 110

<210> 101
<211> 117
<212> PRT
<213> Artificial Sequence

<220>
<223> Artificial Sequence

<400> 101
Gln Val Gln Leu Val Gln Ser Gly Gly Ala Val Val Gln Pro Gly His
1 5 10 15

Ser Leu Arg Leu Ser Cys Glu Ala Ser Gly Phe Lys Phe Lys Asp Tyr
20 25 30

Gly Met His Trp Val Arg Gln Ala Pro Gly Lys Gly Leu Glu Trp Leu
35 40 45

Ala Val Ile Ser His Asp Gly Arg Asn Lys Asn Tyr Ala Asp Ser Val
50 55 60

Lys Gly Arg Leu Thr Ile Ser Arg Asp Asn Ser Lys Asn Thr Leu Ser
65 70 75 80

Phe Gln Met Asn Ser Leu Arg Ala Glu Asp Thr Ala Val Tyr Tyr Cys
85 90 95

Val Arg Asp Trp Ser Tyr Ala Phe Asp Ile Trp Gly Gln Gly Thr Leu
100 105 110

Val Thr Val Ser Ser
115

<210> 102
<211> 107
<212> PRT
<213> Artificial Sequence

<220>
<223> Artificial Sequence

<400> 102
Asp Ile Gln Met Thr Gln Ser Pro Ser Phe Leu Ser Ala Ser Val Gly
1 5 10 15

Asp Arg Val Asn Ile Thr Cys Arg Ala Ser Gln Asp Ile Ser Arg Arg
20 25 30

Leu Ala Trp Tyr Gln Gln Lys Pro Gly Lys Ala Pro Lys Leu Leu Ile
35 40 45

Tyr Gly Ala Ala Ser Leu Gln Ser Ala Val Ala Ser Arg Phe Ser Gly
50 55 60

Ser Gly Ser Gly Thr Glu Phe Thr Leu Thr Ile Ser Ser Leu Gln Pro
65 70 75 80

Glu Asp Phe Ala Asn Tyr Tyr Cys Gln Gln Ile Tyr Thr Thr Pro Leu
85 90 95

Thr Phe Gly Gly Gly Thr Lys Val Glu Ile Lys
100 105

<210> 103

<211> 122
<212> PRT
<213> Artificial Sequence

<220>
<223> Artificial Sequence

<400> 103
Gln Met Gln Leu Val Gln Ser Gly Gly Gly Leu Val Gln Pro Gly Arg
1 5 10 15

Ser Leu Arg Leu Ser Cys Ala Ala Ser Gly Phe Thr Phe Asp Asp Tyr
20 25 30

Ala Met His Trp Val Arg Gln Ala Pro Gly Lys Gly Leu Glu Trp Val
35 40 45

Ser Gly Ile Ser Trp Asn Ser Gly Ser Ile Gly Tyr Ala Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

Thr Lys Gly Pro Gly Leu Ala Thr Gly Lys Val Tyr Phe Asn Ser Trp
100 105 110

Gly Gln Gly Thr Gln Val Thr Val Ser Ser
115 120

<210> 104
<211> 107
<212> PRT
<213> Artificial Sequence

<220>
<223> Artificial Sequence

<400> 104
Asp Ile Gln Met Thr Gln Ser Pro Ser Ser Val Ser Ala Ser Val Gly
1 5 10 15

Asp Arg Val Thr Ile Thr Cys Arg Ala Ser Gln Arg Ile Ser Ser Trp
20 25 30

Leu Ala Trp Tyr Gln Gln Lys Pro Gly Arg Ala Pro Lys Leu Leu Ile
35 40 45

His Ser Ala Ser Thr Leu His Asn Gly Val Pro Ser Arg Phe Ser Gly
50 55 60

Ser Ala Ser Gly Thr Asp Phe Thr Leu Thr Ile Ser Ser Leu Gln Pro
65 70 75 80

Glu Asp Phe Ala Ile Tyr Tyr Cys Gln Gln Gly His Ser Phe Pro Tyr
85 90 95

Thr Phe Gly Gln Gly Thr Lys Leu Asp Ile Lys

100 105

<210> 105
<211> 122
<212> PRT
<213> Artificial Sequence

<220>
<223> Artificial Sequence

<400> 105

```
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Asn Trp Val Arg Gln Ala Pro Gly Lys Gly Leu Val Trp Val
        35                  40                  45

Ser Arg Ile Ser Pro Asp Gly Ser Ser Thr Thr Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Tyr Ser Pro Lys Tyr Pro Thr Val Gly Leu Asp Val Trp
            100                 105                 110

Gly Gln Gly Thr Thr Ile Thr Val Ser Ser
        115                 120
```

<210> 106
<211> 112
<212> PRT
<213> Artificial Sequence

<220>
<223> Artificial Sequence

<400> 106

```
Asp Ile Val Met Thr Gln Ser Pro Leu Ser Ser Pro Val Thr Leu Gly
1               5                   10                  15

Gln Pro Ala Ser Ile Ser Cys Arg Ser Ser Glu Ser Leu Leu His Ser
            20                  25                  30

Asn Gly Asn Thr Tyr Leu Thr Trp Leu Gln Gln Arg Pro Gly Gln Pro
        35                  40                  45

Pro Arg Leu Leu Ile His Lys Ile Ser Asn Arg Phe Ser Gly Val Pro
    50                  55                  60

Asp Arg Phe Ser Gly Ser Gly Ala Gly Thr Asp Phe Thr Leu Gln Ile
65                  70                  75                  80
```

```
Thr Arg Val Glu Thr Glu Asp Val Gly Val Tyr Tyr Cys Val Gln Thr
                85                  90                  95

Thr Gln Trp Pro Trp Thr Phe Gly Gln Gly Thr Lys Val Glu Ile Lys
            100                 105                 110




<210>   107
<211>   125
<212>   PRT
<213>   Artificial Sequence

<220>
<223>   Artificial Sequence


<400>   107
Gln Val Gln Leu Val Gln Ser Gly Ala Glu Val Lys Lys Pro Gly Ala
1               5                   10                  15

Ser Val Arg Val Ser Cys Lys Val Ser Gly Tyr Ser Leu Ser Glu Phe
            20                  25                  30

Pro Ile His Trp Val Arg Gln Ala Pro Arg Met Gly Leu Glu Trp Met
        35                  40                  45

Gly Gly Ser Tyr Pro Glu Asp Gly Glu Thr Leu Tyr Ala Gln Lys Phe
    50                  55                  60

Gln Gly Arg Val Thr Met Thr Glu Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Arg Leu Asn Tyr Phe Glu Ser Thr Asp Tyr Trp Val Asp Ala Phe
            100                 105                 110

Asp Ile Trp Gly Gln Gly Thr Met Val Thr Val Ser Ser
        115                 120                 125


<210>   108
<211>   111
<212>   PRT
<213>   Artificial Sequence

<220>
<223>   Artificial Sequence


<400>   108
Gln Leu Val Leu Thr Gln Pro Tyr Ser Val Ser Glu Ser Pro Gly Lys
1               5                   10                  15

Thr Ile Thr Ile Ser Cys Thr Arg Ser Ser Gly Ser Ile Ala Ser Asn
            20                  25                  30

Phe Val Gln Trp Tyr Gln Gln Arg Pro Gly Ser Ala Pro Thr Pro Val
        35                  40                  45
```

Ile Tyr Glu Asp Asn Gln Arg Pro Ser Gly Val Pro Asp Arg Phe Ser
50 55 60

Gly Ser Ile Asp Ser Ser Ser Asn Ser Ala Ser Leu Thr Ile Ser Gly
65 70 75 80

Val Met Thr Glu Asp Glu Ala Asp Tyr Tyr Cys Gln Ser Tyr Asp Ser
85 90 95

Ser Ser Ser Trp Val Phe Gly Gly Gly Thr Lys Leu Thr Val Leu
100 105 110

<210> 109
<211> 119
<212> PRT
<213> Artificial Sequence

<220>
<223> Artificial Sequence

<400> 109
Gln Met Gln Leu Val Glu Ser Gly Gly Gly Leu Val Lys Pro Gly Gly
1 5 10 15

Ser Leu Thr Leu Ser Cys Asp Ala Thr Gly Phe Asn Phe Gly Ser Tyr
20 25 30

Tyr Met Asn Trp Val Arg Gln Ala Pro Gly Lys Gly Leu Glu Trp Leu
35 40 45

Ala His Ile Ser Ser Thr Gly Arg Thr Ile Tyr Tyr Ala Asp Ser Val
50 55 60

Lys Gly Arg Phe Thr Ile Ser Arg Asp Asn Ala Lys Ser Ser Leu Asp
65 70 75 80

Leu Gln Met Asn Ser Leu Arg Ala Glu Asp Thr Ala Val Tyr Tyr Cys
85 90 95

Ala Arg Asp Gln Gly Tyr Pro Phe Gly Met Asp Val Trp Gly His Gly
100 105 110

Thr Thr Val Thr Val Ser Ser
115

<210> 110
<211> 111
<212> PRT
<213> Artificial Sequence

<220>
<223> Artificial Sequence

<400> 110
Gln Leu Val Leu Thr Gln Pro Ser Ser Val Ser Gly Ala Pro Gly Gln
1 5 10 15

Arg Val Thr Ile Ser Cys Thr Gly Ser Ser Ser Asn Ile Gly Ala Gly

```
            20                  25                  30

Tyr Asp Val Asp Trp Tyr Gln Gln Leu Pro Gly Thr Ala Pro Lys Leu
        35                  40                  45

Leu Ile Tyr Gly Asn Thr Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Asp Ser Asp Tyr Tyr Cys Gln Ser Tyr Asp Ser Ser
                85                  90                  95

Leu Ser Ala Trp Val Phe Gly Gly Gly Thr Lys Leu Thr Val Leu
            100                 105                 110



<210>    111
<211>    126
<212>    PRT
<213>    Artificial Sequence

<220>
<223>    Artificial Sequence


<400>    111
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Tyr Ser Gly Gly Ser Thr Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Arg Glu Gly Ser Tyr Asp Val Met Thr Tyr Thr Arg Ile Gly Gly Tyr
            100                 105                 110

Phe Asp Tyr Trp Gly Gln Gly Ala Leu Val Thr Val Ser Ser
        115                 120                 125



<210>    112
<211>    107
<212>    PRT
<213>    Artificial Sequence

<220>
<223>    Artificial Sequence
```

<400> 112
Asp Ile Gln Met Thr Gln Ser Pro Ser Ser Val Ser Ala Ser Val Gly
1 5 10 15

Asp Arg Val Thr Ile Thr Cys Arg Ala Ser Gln Gly Ile Ser Asp Trp
20 25 30

Val Ala Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Glu Phe Ser Leu Thr Ile Ser Asn Leu Gln Pro
65 70 75 80

Glu Asp Phe Ala Thr Tyr Tyr Cys Gln Gln Ala Asn Ser Phe Pro Leu
85 90 95

Thr Phe Gly Pro Gly Thr Lys Val Glu Ile Lys
100 105

<210> 113
<211> 38
<212> DNA
<213> Artificial Sequence

<220>
<223> Artificial Sequence

<400> 113
aggggggccgt gggggccgct gaatgcttcc cggcctgc 38

<210> 114
<211> 40
<212> DNA
<213> Artificial Sequence

<220>
<223> Artificial Sequence

<400> 114
tagcggccga cgcggccaac tggccctcgg tgaggagagg 40

<210> 115
<211> 6
<212> PRT
<213> Artificial Sequence

<220>
<223> Artificial Sequence

<400> 115
Gln Gly Ile Ser Ser Ala
1 5

<210> 116
<211> 9
<212> PRT
<213> Artificial Sequence

<220>
<223> Artificial Sequence

<400> 116

```
Gln Gln Ser Tyr Thr Thr Pro Leu Thr
1               5
```

<210> 117
<211> 26
<212> PRT
<213> Artificial Sequence

<220>
<223> Artificial Sequence

<400> 117

```
Asp Ile Gln Met Thr Gln Ser Pro Ser Ser Leu Ser Ala Ser Leu Gly
1               5                   10                  15

Asp Arg Val Thr Ile Thr Cys Arg Ala Ser
            20                  25
```

<210> 118
<211> 36
<212> PRT
<213> Artificial Sequence

<220>
<223> Artificial Sequence

<400> 118

```
Ser Leu Gln Ser Gly Val Pro Ser Arg Phe Ser Gly Ser Gly Ser Gly
1               5                   10                  15

Thr Asp Phe Thr Leu Thr Ile Asn Ser Leu Gln Pro Glu Asp Phe Ala
            20                  25                  30

Thr Tyr Tyr Cys
        35
```

<210> 119
<211> 25
<212> PRT
<213> Artificial Sequence

<220>
<223> Artificial Sequence

<400> 119

```
Gln Val Gln Leu Val Gln Ser Gly Gly Gly Val Val Gln Pro Gly Gly
```

```
  1               5                   10                  15

Ser Leu Arg Leu Ser Cys Ala Ala Ser
             20                  25


<210>    120
<211>    26
<212>    PRT
<213>    Artificial Sequence

<220>
<223>    Artificial Sequence


<400>    120
Asp Ile Gln Met Thr Gln Ser Pro Ser Phe Leu Ser Ala Ser Val Gly
  1               5                   10                  15

Asp Arg Val Thr Ile Thr Cys Arg Ala Ser
             20                  25


<210>    121
<211>    6
<212>    PRT
<213>    Artificial Sequence

<220>
<223>    Artificial Sequence


<400>    121
His Asp Ile Ser Ser Ser
  1               5


<210>    122
<211>    17
<212>    PRT
<213>    Artificial Sequence

<220>
<223>    Artificial Sequence


<400>    122
Leu Ala Trp Tyr Gln Gln Lys Ser Gly Lys Ala Pro Lys Leu Leu Ile
  1               5                   10                  15

Tyr


<210>    123
<211>    36
<212>    PRT
<213>    Artificial Sequence

<220>
<223>    Artificial Sequence
```

<400> 123
Asn Leu Lys Ser Gly Val Pro Ser Arg Phe Ser Gly Ser Gly Ser Gly
1 5 10 15

Thr Asp Phe Ser Leu Thr Ile Ser Ser Leu Gln Pro Glu Asp Phe Ala
20 25 30

Thr Tyr Tyr Cys
35

<210> 124
<211> 9
<212> PRT
<213> Artificial Sequence

<220>
<223> Artificial Sequence

<400> 124
Gln Gln Ser Tyr Thr Thr Val Leu Thr
1 5

<210> 125
<211> 10
<212> PRT
<213> Artificial Sequence

<220>
<223> Artificial Sequence

<400> 125
Phe Gly Gly Gly Thr Lys Leu Glu Ile Lys
1 5 10

<210> 126
<211> 107
<212> PRT
<213> Artificial Sequence

<220>
<223> Artificial Sequence

<400> 126
Asp Ile Gln Met Thr Gln Ser Pro Ser Ser Leu Ser Ala Ser Leu Gly
1 5 10 15

Asp Arg Val Thr Ile Thr Cys Arg Ala Ser Gln Gly Ile Ser Ser Ala
20 25 30

Leu Ala Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Asn Ser Leu Gln Pro
65 70 75 80

Glu Asp Phe Ala Thr Tyr Tyr Cys Gln Gln Ser Tyr Thr Thr Pro Leu
85 90 95

Thr Phe Gly Gly Gly Thr Lys Val Glu Ile Lys
100 105

<210> 127
<211> 117
<212> PRT
<213> Artificial Sequence

<220>
<223> Artificial Sequence

<400> 127
Gln Val Gln Leu Val Gln Ser Gly Gly Gly Val Val Gln Pro Gly Gly
1 5 10 15

Ser Leu Arg Leu Ser Cys Ala Ala Ser Gly Phe Lys Phe Lys Asp Tyr
20 25 30

Gly Met His Trp Val Arg Gln Ala Pro Gly Lys Gly Leu Glu Trp Leu
35 40 45

Ala Val Ile Ser His Asp Gly Arg Asn Lys Asn Tyr Ala Asp Ser Val
50 55 60

Lys Gly Arg Leu Thr Ile Ser Arg Asp Asn Ser Lys Asn Thr Leu Ser
65 70 75 80

Phe Gln Met Asn Ser Leu Arg Ala Glu Asp Thr Ala Val Tyr Tyr Cys
85 90 95

Val Arg Asp Trp Ser Tyr Ala Phe Asp Ile Trp Gly Gln Gly Thr Leu
100 105 110

Val Thr Val Ser Ser
115

<210> 128
<211> 107
<212> PRT
<213> Artificial Sequence

<220>
<223> Artificial Sequence

<400> 128
Asp Ile Gln Met Thr Gln Ser Pro Ser Phe Leu Ser Ala Ser Val Gly
1 5 10 15

Asp Arg Val Thr Ile Thr Cys Arg Ala Ser His Asp Ile Ser Ser Ser
20 25 30

Leu Ala Trp Tyr Gln Gln Lys Ser Gly Lys Ala Pro Lys Leu Leu Ile
35 40 45

Tyr Ser Ala Ser Asn Leu Lys Ser Gly Val Pro Ser Arg Phe Ser Gly

```
        50                  55                  60

Ser Gly Ser Gly Thr Asp Phe Ser Leu Thr Ile Ser Ser Leu Gln Pro
65                  70                  75                  80

Glu Asp Phe Ala Thr Tyr Tyr Cys Gln Gln Ser Tyr Thr Thr Val Leu
                85                  90                  95

Thr Phe Gly Gly Gly Thr Lys Leu Glu Ile Lys
            100                 105
```

## Description

### TECHNICAL FIELD

The present invention relates to new antibody-drug conjugates (ADCs) targeting DLK1, active metabolites of such ADCs, methods for preparation of such ADCs, uses for such ADCs in treatment and/or prevention of illnesses, and uses for such ADCs in production of drugs for treatment and/or prevention of diseases, more specifically, uses for such ADCs in producing drugs for treatment and/or prevention of proliferative and/or angiogenetic diseases, for example, cancer. More particularly, the present invention relates to an antibody-drug conjugate comprising an antibody binding with DLK1 or an antigen-binding fragment thereof, and a pharmaceutical composition comprising the same.

### BACKGROUND

Cancer is a disease caused by abnormal lumps of uncontrolled cell growth in the tissues of the body, and is the result of uncontrolled cell growth in a variety of tissues. Tumors in early stage cancers may be removed through surgical and radiotherapeutic measures, and metastasized tumors are generally treated palliatively using chemotherapy.

Most chemotherapy agents administered non-orally may induce unwanted adverse effects or serious toxicity as a result of systemic administration. Accordingly, the focus of development has been on developing novel chemotherapy agents to achieve increased efficacy and minimal toxicity / adverse effects through improved and selective action of chemotherapy agents on tumor cells or immediately adjacent tissues.

An antibody-drug conjugate (ADC) is a new targeted technology where a toxin or drug is bonded to an antibody which in turn binds to an antigen, the toxin or drug released into a tumor cell, etc., to cause cell death. The technology has superior efficacy over antibody drugs, and is able to substantially reduce the risk of adverse effects compared to conventional anti-cancer agents, as it specifically delivers drugs to the target cancer cells with minimum impact to healthy cells, and only releases drugs under specific conditions.

The basic structure of an antibody-drug conjugate is "antibody - linker - small molecule drug (toxin)". Here, the linker needs to play not only the functional role of linking the antibody and drug, but also ensure that the drug is released properly through antibody-drug dissociation (e.g. as a result of hydrolysis by enzyme) after being circulated through the body and reaching the target cells, and exhibits efficacy against the target cancer cells. That is, the stability of the linker plays a very important role in the efficacy and systemic toxicity of an antibody-drug conjugate (Discovery Medicine 2010, 10(53): 329-39).

The inventors of the present invention have developed and secured a patent for a linker including an effective self-immolative group, which is more stable in the plasma and in circulation, and allows a drug to be easily released and exhibit efficacy in a cancer cell (Korean Registered Patent No. 1,628,872, etc.). Meanwhile, use of monoclonal antibodies for cancer treatment is having substantial success. Monoclonal antibodies are suitable for target-oriented addressing of tumor tissue and tumor cells. Antibody-drug conjugates have become a novel and powerful option for therapy of lymphomas and solid tumors, and recently, immunoregulatory antibodies are seeing significant success in clinical trials. Development of therapeutic antibodies is based on a profound understanding of cancer serology, protein engineering technology, mechanisms of action and resistance, and interactions between immune systems and cancer cells.

Antigens which are expressed on the surface of human cancer cells are defined as a broad range of targets which are over-expressed compared to normal tissues, mutated or selectively expressed. The key problem is identifying the appropriate antigens for antibody-based therapies. These therapeutic agents mediate changes in antigen or receptor function (i.e., as a stimulant or antagonist), regulate the immune system through Fc and T cell activation, and exhibit efficacy through the delivery of specific drugs that bind to antibodies targeting specific antigens. Molecular techniques that can alter antibody pharmacokinetics, functional function, size and immune stimulation are emerging as key elements in the development of novel antibody-based therapies. Evidence from clinical trials of therapeutic antibodies in cancer patients highlights the importance of approaches for selecting optimized antibodies, including affinity and binding of the target antigen and antibodies, selection of antibody structure, and therapeutic approaches (blocking signaling or immune function).

Related research is in progress on antibodies whose antigen is DLK1. Human-derived DLK1 (delta-like 1 homolog (Drosophila)) is a single-pass transmembrane protein whose whole chain comprises 383 amino acids. The protein has 6 EGF (epidermal growth factor-like repeat) domains in the extracellular domain.

DLK1 is generally referred to by the gene DLK1 due to its homology in amino acid sequence with Delta, a ligand of the Notch receptor which is a cell differentiation control factor, and other names by which it is referred to are Pref-1, pG2, SCP-1 and ZOG. While DLK1 is a transmembrane protein, it is well reported as a protein where the extracellular domain is shed from the cell membrane by TACE (Tumor necrosis factor alpha converting enzyme) and functions separately.

DLK1 exhibits high expression in undifferentiated fetal cells with a high proliferation index. In particular, whereas

high expression is exhibited in the liver, kidneys, skeletal muscles and brain, etc., of the fetus, expression is not seen in most tissues after birth, with expression limited to only certain cells such as preadipocytes, pancreatic islet cells, thymic stromal cells and adrenal gland cells.

As for research into the functions of DLK1, DLK1 is most researched as Pref-1 (preadipocyte factor-1) which is a factor that inhibits adipose cell differentiation. Aside from its ability to suppress adipose cell differentiation, DLK1 is reported to be able to suppress differentiation of hematopoietic stem cells, to play a role in regulating differentiation of lymphoid progenitor cells, and to be associated with wound healing.

Further, DLK1 is reported as being expressed with high frequency in various cancers or tumors. Cancers in which the expression of DLK1 has been confirmed to date include the solid cancers of neuroendocrine tumors, neuroblastoma, glioma, neurofibromatosis type 1, small cell lung cancer, liver cancer, renal cancer, ovarian cancer, colorectal cancer, breast cancer and pancreatic cancer, and the blood cancers of myelodysplastic syndrome and acute myeloid leukemia. As for research on the association between nDLK1 and cancer, there have been reports that DLK1 is overexpressed in brain cancer cells (gliomas), and that overexpressing the cDNA of DLK1 in brain cancer cells increases proliferation and migration of brain cancer cells. It has also been reported that DLK1 expression in liver cancer is elevated compared to normal liver cells, and that when DLK1 expression is reduced through siRNA tests, tumor size decreases.

To this technical background, the inventors of the present invention have worked to develop antibodies which bind specifically to DLK1, as a result developing anti-DLKl antibody exhibiting superior binding to DLK1. The inventors have confirmed that, by applying a linker including an effective self-immolative group, which is more stable in the plasma and in circulation, and allows a drug to be easily released and exhibit efficacy in a cancer cell, to the anti-DLKl antibody to further reinforce the effect of the antibody, it is possible to provide a novel antibody-drug conjugate (ADC) which targets DLK1 and is effective at treating and/or preventing cancer illnesses, and thereby completed the present invention.

## DETAILED DESCRIPTION OF THE INVENTION

## TECHNICAL PROBLEM

A purpose of the present invention is to provide a novel antibody-drug conjugate targeting DLK1, or a salt or solvate thereof.

Another purpose of the present invention is to provide a drug-antibody conjugate including an antibody which binds specifically to DLK1 and a drug binding thereto, and a pharmaceutical composition including the same.

Another purpose of the present invention is to provide a method for preventing or treating a proliferative, cancer or angiogenetic disease, the method comprising a step of administering an individual with an antibody-drug conjugate comprising a pharmaceutically efficacious amount of antibody which specifically binds to DLK1 and a drug which binds to the same.

Another purpose of the present invention is to provide a use for an antibody-drug conjugate comprising a pharmaceutically efficacious amount of antibody which specifically binds to DLK1 and a drug which binds to the same as a pharmaceutical composition for preventing or treating a proliferative, cancer or angiogenetic disease.

Yet another purpose of the present invention is to provide an antibody-linker-drug (toxin) system which allows a drug and/or toxin to safely reach a target cell and effectively exhibit efficacy while substantially reducing toxicity, by grafting technology for a linker including a self-immolative group which is more stable in the plasma and in circulation, and allows a drug to be easily released in a cancer cell to maximize efficacy.

## TECHNICAL SOLUTION

One aspect of the present invention provides an antibody conjugate of the General Formula I, or a pharmaceutically acceptable salt or solvate thereof.

$$[\text{General Formula I}] \quad Ab - (X)_y$$

Where:

Ab is an anti-DLKl antibody or an antigen-binding fragment thereof,
X is independently a chemical residue comprising at least one active agent and a linker, and;
the linker links an antibody and the active agent, and;
y is an integer from 1 through 20.

The anti-DLKl antibody or antigen-binding fragment thereof, that is the antibody which binds to DLK1 or antigen-binding fragment of the same, comprises: a heavy chain variable region comprising at least one heavy chain CDR1 selected from the group comprising SEQ ID NO. 2, 16, 30, 44, 58, 72 and 86, at least one heavy chain CDR2 selected from the group comprising SEQ ID NO. 4, 18, 32, 46, 60, 74 and 88, and at least one heavy chain CDR3 selected from the group comprising SEQ ID NO. 6, 20, 34, 48, 62, 76 and 90, and;
a light chain variable region comprising at least one light chain CDR1 selected from the group comprising SEQ ID No. 9, 23, 37, 51, 65, 79, 93, 115 and 121, at least one light chain CDR2 selected from the group comprising SEQ ID No. 11, 25, 39, 53, 67, 81 and 95, and at least one light chain CDR3 selected from the group comprising SEQ ID No. 13, 27, 41, 55, 69, 83, 97, 116 and 125.

The antibody according to the present invention may, for example, bind specifically to the extracellular domain of human DLK1.

The term "antibody" as used in the present specification refers to DLK1, in particular anti-DLK1 antibody which binds specifically to the extracellular domain of human DLK1 protein. Included in the scope of the present invention is not only the complete form of the antibody which binds specifically to DLK1, but also antigen-binding fragments of the antibody molecule.

The complete antibody has a structure having 2 full length heavy chains and 2 full length light chains, the respective light chains linked to a heavy chain by a disulfide bond. The heavy chain constant regions have types gamma ($\gamma$), mu ($\mu$), alpha ($\alpha$), delta ($\delta$) and epsilon ($\varepsilon$), with subclasses gammal ($\gamma$1), gamma2 (y2), gamma3 ($\gamma$3), gamma4 ($\gamma$1), alpha1 ($\alpha$1) and alpha2 ($\alpha$2). The light chain constant region has types kappa ($\kappa$) and lambda ($\lambda$).

An antigen-binding fragment of an antibody or antibody fragment refers to a fragment with the functionality to bind to an antigen, and includes Fab, F(ab'), F(ab')2 and Fv, etc. Among antibody fragments, Fab has a structure of light chain and heavy chain variable regions, a light chain constant region and a first constant region of a heavy chain (CH1), and 1 antigen-binding site. Fab' differs from Fab in that it has a hinge region comprising at least one cysteine residue at the C-terminal of the heavy chain CH1 domain. The F(ab)'2 antibody is formed when the cysteine residue of the hinge region of Fab' forms a disulfide bond. Fv is a minimum antibody fragment which only has a heavy chain variable region and light chain variable region, and recombinant technologies for preparing Fv fragments are disclosed in PCT International Published Patent Applications WO88/10649, WO88/106630, WO88/07085, WO88/07086 and WO88/09344. A two-chain Fv has a heavy chain variable region and light chain variable region linked by a noncovalent bond, and a single-chain Fv (scFv) generally has a heavy chain variable region and light chain variable region linked by a covalent bond through a peptide linker, or linked directly at the C-terminal, allowing it to form structures such as a dimer, as can a two-chain Fv. Such antibody fragments may be obtained by using protein hydrolyzing enzyme (for example, restriction cutting the entire antibody with papain yields Fab, and cutting with pepsin can yield F(ab)'2 fragment), and may also be prepared using gene recombinant technology.

In embodiments, the antibody according to the present invention is of the Fv form (for example, scFv), or in its complete antibody form. Further, the heavy chain constant region may be any one isotype selected from among gamma ($\gamma$), mu ($\mu$), alpha ($\alpha$), delta ($\delta$) and epsilon ($\varepsilon$). For example, the constant region is Gamma1 (IgGl), Gamma3 (IgG3) or Gamma4 (IgG4). The light chain constant region may be the kappa or lambda type.

The term "heavy chain" as used in the present specification refers to the full length heavy chain comprising the variable region domain VH which comprises amino acid sequences having sufficient variable region sequences to give antigen specificity and the three constant region domains CH1, CH2 and CH3, and all fragments of the same. Further the term "light chain" as used in the present specification refers to the full length light chain comprising the variable region domain VL which comprises amino acid sequences having sufficient variable region sequences to give antigen specificity and the constant region domain CL, and all fragments of the same.

The antibody of the invention includes, but is not limited to, monoclonal antibody, multispecific antibody, human antibody, humanized antibody, chimera antibody, single chain Fvs (scFV), single chain antibody, Fab fragment, F(ab') fragment, disulfide-bond Fvs (sdFV) and anti-idiotype (anti-Id) antibody, or epitope-binding fragments of the above antibodies, etc.

Monoclonal antibodies refer to antibodies obtained from a substantially homogeneous antibody group, that is, except for possible naturally occurring mutations in which individual antibodies that occupy the population may be present in minor amounts. Monoclonal antibodies are highly specific and are directed against a single antigenic site. In contrast to a conventional (polyclonal) antibody formulation which comprises different antibodies directed to different determining factors, the respective monoclonal antibodies are directed against a single determining factor on the antigen. For example, the useful monoclonal antibodies of the present invention may be prepared using the hybridoma method, or by using recombinant DNA methods on bacteria or eukaryotic animal cells or plant cells (see US Patent 4,816,567). Further, the monoclonal antibody may be isolated from a phage antibody library.

In one embodiment of the present invention, the phage display method was used to pan a natural human single chain Fv library (native human single chain Fv library) to prepare seven types of monoclonal human antibody which bind specifically to DLK1.

"Phage display" is a technique for displaying a mutant polypeptide as a fusion protein with the phage, for

example, at least part of the coat protein on the surface of the phage particle. The usefulness of phage display is that large libraries of randomized protein variants may be targeted to quickly and efficiently classify sequences that bind to target antigens in high affinity. Displaying peptides and protein libraries on phages has been used to screen millions of polypeptides to identify polypeptides with specific binding properties.

Phage display technology has provided a powerful tool for generating and screening new proteins that bind to specific ligands (e.g. antigens). Using phage display technology, large libraries of protein variants may be generated and sequences that bind with high affinity to target antigens may be quickly classified. The nucleic acid encoding the mutant polypeptide is fused with a nucleic acid sequence encoding a viral coat protein, e.g. a gene III protein or a gene VIII protein. A monophasic phage display system has been developed in which a nucleic acid sequence encoding a protein or polypeptide is fused with a nucleic acid sequence encoding a part of the gene III protein. In the 1-phage display system, the gene fusion is expressed at a low level and the wild-type gene III protein is also expressed, thereby maintaining the infectivity of the particles.

Demonstrating the expression of peptides on the fibrous phage surface and the expression of functional antibody fragments in the peripheral cytoplasm of E. coli is important in developing antibody phage display libraries. Libraries of antibodies or antigen-binding polypeptides have been prepared in a number of ways, for example by altering a single gene by inserting a random DNA sequence, or by cloning a related gene sequence. The library may be screened for the expression of antibodies or antigen binding proteins with the desired characteristics.

Phage display technology has several advantages over conventional hybridomas and recombinant methods for producing antibodies with the desired characteristics. This technique allows the generation of large antibody libraries with a variety of sequences in a short time without the use of animals. The production of hybridomas and the production of humanized antibodies may require several months of manufacturing time. In addition, since no immunity is required, the phage antibody library can generate antibodies against antigens that are toxic or have low antigenicity. Phage antibody libraries can also be used to generate and identify novel therapeutic antibodies.

Techniques for generating human antibodies from non-immunized humans, germline sequences, or sub-sensitized B cell Ig repertoires may be used, which are immunized using a phage display library. Various lymphatic tissues may be used to prepare an undetected or non-immunogenic antigen-binding library.

Techniques for identifying and separating high affinity antibodies from phage display libraries are important for new antibody separation for therapy. The separation of high affinity antibodies from the library can depend on the size of the library, the production efficiency in bacterial cells and the variety of libraries. The size of the library is reduced by inefficient folding of the antibody or antigen binding protein and inefficient production due to the presence of the stop codon. Expression in bacterial cells may be inhibited when the antibody or antigen binding domain is not properly folded. Expression may be improved by alternately mutating the residues at the surface of the variable / constant interface or the selected CDR residues. The sequence of the framework region is one element to provide appropriate folding in the case of generating antibody phage libraries in bacterial cells.

It is important to generate various libraries of antibody or antigen binding proteins in high affinity antibody separation. CDR3 regions have been found to often participate in antigen binding. The CDR3 region on the heavy chain varies considerably in terms of size, sequence and structural conformation, and thus various libraries may be prepared using the CDR3 region.

Also, diversity may be generated by randomizing the CDR regions of the variable heavy and light chains using all 20 amino acids at each position. The use of all 20 amino acids results in an increased variability in antibody sequences and an increased chance of identifying new antibodies.

"Epitope" refers to a protein determinant to which an antibody can specifically bind. Epitopes usually consist of a group of chemically active surface molecules, such as amino acids or sugar side chains, and generally have specific three-dimensional structural characteristics as well as specific charge characteristics. Three-dimensional epitopes and non-stereo epitopes are distinguished in that the binding to the former is lost but not to the latter in the presence of a denatured solvent.

The non-human (e.g., murine) antibody of the "humanized" form is a chimeric antibody containing minimal sequence derived from non-human immunoglobulin. In most cases, the humanized antibody is a non-human species (donor antibody), such as a mouse, a rat, a rabbit or a non-human, having a desired specificity, affinity and ability to retain a residue from the hypervariable region of the recipient and is a human immunoglobulin (acceptor antibody) that has been replaced with a residue from the hypervariable region of the primate.

The above-mentioned "human antibody" means a molecule derived from human immunoglobulin, wherein all the amino acid sequences constituting the antibody including the complementarity determining region and the structural region are composed of human immunoglobulin.

The other chain(s) may be derived from another species or may be derived from another antibody class or subgroup, such as a portion of the heavy chain and / or light chain derived from a particular species or identical or homologous to a corresponding sequence in an antibody belonging to a particular antibody class or subclass, "chimeric" antibodies (immunoglobulins) identical or homologous to the corresponding sequences in antibodies belonging to the

subclass as well as fragments of said antibodies exhibiting the desired biological activity.

As used herein, an "antibody variable domain" refers to the light and heavy chain portions of an antibody molecule comprising complementarity determining regions (CDRs; i.e., CDR1, CDR2, and CDR3), and the amino acid sequence of the framework region (FR). VH refers to the variable domain of the heavy chain. VL refers to the variable domain of the light chain.

"Complementarity determining regions" (CDRs; i.e., CDR1, CDR2, and CDR3) refer to the amino acid residues of an antibody variable domain that are necessary for antigen binding. Each variable domain typically has three CDR regions identified as CDR1, CDR2 and CDR3.

In the present invention, the antibody binding to DLK1 or an antigen-binding fragment thereof may specifically include the CDR sequences stated in Table 1 below. Of these, Korean Patent Application No. 10-2018-0107639 has confirmed that the anti-DLKl antibodies of two types (18A5 and 27F7) and two other 18A5 mutant antibodies (18A5_LS_1A10 and 18A5 AM 1A12) are capable of binding to cells where DLK1 is overexpressed, and may be developed into anti-DLKl antibody-drug conjugates which can target DLK1 expressed on the surface of cancer cells to cause cancer cell apoptosis.

[Table 1]

| Clone | FR1 | CDR1 | FR2 | CDR2 | FR3 | CDR3 | FR4 | SEQ ID NO. |
|---|---|---|---|---|---|---|---|---|
| 17B11-HC | QVQLVESGGTLVQPGGSLRLSCAAS | GFTFSSHA | MSWVRQTPGKGLEWVSS | ITKSGSGT | YSADSVKGRFTISRDNSKNTLYLQMNSLRAEDTAVYYC | TREGLGYYGMDV | WGQGTTVTVSS | 99 |
| SEQ ID NO | 1 | 2 | 3 | 4 | 5 | 6 | 7 | |
| 17B11-LC | QLVLTQPPSVSGAPGQRVIISCTGS | SSNIGAGYD | VHWYQQLPGTAPRLLIY | GST | NRPSGVPDRFSGSKSGTSASLAITGLQAEDEADYYC | QSYDNSLSAHYV | FGTGTKVTVL | 100 |
| SEQ ID NO. | 8 | 9 | 10 | 11 | 12 | 13 | 14 | |
| 18A5-HC | QVQLVQSGGAVVQPGHSLRLSCEAS | GFKFKDYG | MHWVRQAPGKGLEWLAV | ISHDGRNK | NYADSVKGRLTISRDNSKNTLSFQMNSLRAEDTAVYYC | VRDWSYAFDI | WGQGTLVTVSS | 101 |
| SEQ ID NO. | 15 | 16 | 17 | 18 | 19 | 20 | 21 | |
| 18A5-LC | DIQMTQSPSFLSASVGDRVNITCRAS | QDISRR | LAWYQQKPGKAPKLLIY | GAA | SLQSAVASRFSGSGSGTEFTLTISSLQPEDFANYYC | QQIYTTPLT | FGGGTKVEIK | 102 |
| SEQ ID NO | 22 | 23 | 24 | 25 | 26 | 27 | 28 | |

(continued)

| Clone | FR1 | CDR1 | FR2 | CDR2 | FR3 | CDR3 | FR4 | SEQ ID NO. |
|---|---|---|---|---|---|---|---|---|
| 20D3-HC | QMQL<br>VQSG<br>GGLV<br>QPGRS<br>LRLSC<br>AAS | GFTF<br>DDY<br>A | MHWV<br>RQAPG<br>KGLE<br>WVSG | ISWN<br>SGSI | GYADSVK<br>GRFTISR<br>DNAKNSL<br>YLQMNSL<br>RAEDTAV<br>YYC | TKGP<br>GLAT<br>GKVY<br>FNS | WGQ<br>GTQV<br>TVSS | 103 |
| SEQ ID NO. | 29 | 30 | 31 | 32 | 33 | 34 | 35 | |
| 20D3-LC | DIQM<br>TQSPS<br>SVSAS<br>VGDR<br>VTITC<br>RAS | QRIS<br>SW | LAWY<br>QQKP<br>GRAPK<br>LLIH | SAS | TLHNGVP<br>SRFSGSAS<br>GTDFTLTI<br>SSLQPEDF<br>AIYYC | QQGH<br>SFPYT | FGQG<br>TKLD<br>IK | 104 |
| SEQ ID NO. | 36 | 37 | 38 | 39 | 40 | 41 | 42 | |
| 21D8-HC | QVQL<br>VESG<br>GGLV<br>QPGG<br>SLRLS<br>CAAS | GFTF<br>SSY<br>W | MNWV<br>RQAPG<br>KGLV<br>WVSR | ISPD<br>GSST | TYADSVK<br>GRFTISR<br>DNAKNTL<br>YLQMNSL<br>RAEDTAV<br>YYC | ARGY<br>SPKYP<br>TVGL<br>DV | WGQ<br>GTTI<br>TVSS | 105 |
| SEQ ID NO. | 43 | 44 | 45 | 46 | 47 | 48 | 49 | |
| 21D8-LC | DIVMT<br>QSPLS<br>SPVTL<br>GQPAS<br>ISCRS<br>S | ESLL<br>HSN<br>GNT<br>Y | LTWL<br>QQRP<br>GQPPR<br>LLIH | KIS | NRFSGVP<br>DRFSGSG<br>AGTDFTL<br>QITRVET<br>EDVGVYY<br>C | VQTT<br>QWP<br>WT | FGQG<br>TKVE<br>IK | 106 |

(continued)

| Clone | FR1 | CDR1 | FR2 | CDR2 | FR3 | CDR3 | FR4 | SEQ ID NO. |
|---|---|---|---|---|---|---|---|---|
| SEQ ID NO. | 50 | 51 | 52 | 53 | 54 | 55 | 56 | |
| 21F9-HC | QVQLVQSGAEVKKPGASVRVSCKVS | GYSLSEFP | IHWVRQAPRMGLEWMGG | SYPEDGET | LYAQKFQGRVTMTEDTSTDTAYMELSSLRSEDTAVYYC | ARLNYFESTDYWVDAFDI | WGQGTMVTVSS | 107 |
| SEQ ID NO. | 57 | 58 | 59 | 60 | 61 | 62 | 63 | |
| 21F9-LC | QLVLTQPYSVSESPGKTITISCTRS | SGSIASNF | VQWYQQRPGSAPTPVIY | EDN | QRPSGVPDRFSGSIDSSSNSASLTISGVMTEDEADYYC | QSYDSGSSWV | FGGGTKLTVL | 108 |
| SEQ ID NO. | 64 | 65 | 66 | 67 | 68 | 69 | 70 | |
| 27F7-HC | QMQLVESGGGLVKPGGSLTLSCDAT | GFNFGSYY | MNWVRQAPGKGLEWLAH | ISSTGRTI | YYADSVKGRFTISRDNAKSSLDLQMNSLRAEDTAVYYC | ARDQGYPFGMDV | WGHGTTVTVSS | 109 |
| SEQ ID NO. | 71 | 72 | 73 | 74 | 75 | 76 | 77 | |

(continued)

| Clone | FR1 | CDR1 | FR2 | CDR2 | FR3 | CDR3 | FR4 | SEQ ID NO. |
|---|---|---|---|---|---|---|---|---|
| 27F7-LC | QLVLTQPSSVSGAPGQRVTISCTGS | SSNIGAGYD | VDWYQQLPGTAPKLLIY | GNT | NRPSGVPDRFSGSKSGTSASLAITGLQAEDDSDYYC | QSYDSSLSAWV | FGGGTKLTVL | 110 |
| SEQ ID NO. | 78 | 79 | 80 | 81 | 82 | 83 | 84 | |
| 35E2-HC | QVQLVESGGGVVQPGRSLRLSCAAS | GFTFSSYA | MHWVRQAPGKGLEWVAV | IYSGGST | YYADSVKGRFTISRDNSKNTLYLQMNSLRAEDTAVYYC | AREGSYDYMTYTRIGGYFDY | WGQGALVTVSS | 111 |
| SEQ ID NO. | 85 | 86 | 87 | 88 | 89 | 90 | 91 | |
| 35E2-LC | DIQMTQSPSSVSASVGDRVTITCRAS | QGISDW | VAWYQQKPGKAPKLLIY | AAS | SLQSGVPSRFSGSGSGTEFSLTISNLQPEDFATYYC | QQANSFPLT | FGPGTKVEIK | 112 |
| SEQ ID NO. | 92 | 93 | 94 | 95 | 96 | 97 | 98 | |
| 18A5 LS_1A 10 -HC | QVQLVQSGGAVVQPGHSLRLSCEAS | GFKFKDYG | MHWVRQAPGKGLEWLAV | ISHDGRNK | NYADSVKGRLTISRDNSKNTLSFQMNSLRAEDTAVYYC | VRDWSYAFDI | WGQGTLVTVSS | 101 |

(continued)

| Clone | FR1 | CDR1 | FR2 | CDR2 | FR3 | CDR3 | FR4 | SEQ ID NO. |
|---|---|---|---|---|---|---|---|---|
| SEQ ID NO. | 15 | 16 | 17 | 18 | 19 | 20 | 21 | |
| 18A5 LS_1A 10 LC | DIQM TQSPS SLSAS LGDR VTITC RAS | QGIS SA | LAWY QQKP GKAP KLLIY | AAS | SLQSGVP SRFSGSG SGTDFTL TINSLQPE DFATYYC | QQSY TTPLT | FGGG TKVE IK | 126 |
| SEQ ID NO. | 117 | 115 | 24 | 95 | 118 | 116 | 28 | |
| 18A5 AM 1 A12 -HC | QVQL VQSG GGVV QPGG SLRLS CAAS | GFK FKD YG | MHWV RQAPG KGLE WLAV | ISHD GRN K | NYADSVK GRLTISR DNSKNTL SFQMNSL RAEDTAV YYC | VRDW SYAF DI | WGQ GTLV TVSS | 127 |
| SEQ ID NO. | 119 | 16 | 17 | 18 | 19 | 20 | 21 | |
| 18A5 AM 1 A12 -LC | DIQM TQSPS FLSAS VGDR VTITC RAS | HDIS SS | LAWY QQKS GKAP KLLIY | SAS | NLKSGVP SRFSGSG SGTDFSL TISSLQPE DFATYYC | QQSY TTVL T | FGGG TKLE IK | 128 |
| SEQ ID NO. | 120 | 121 | 122 | 39 | 123 | 124 | 125 | |

A "framework region" (FR) is a variable domain residue other than a CDR residue. Each variable domain typically has four FRs identified as FR1, FR2, FR3, and FR4.

Embodiments of the present invention may comprise: at least one heavy chain FR1 selected from the group comprising SEQ ID No. 1, 15, 29, 43, 57, 71, 85 and 119; at least one heavy chain FR2 selected from the group comprising SEQ ID No. 3, 17, 31, 45, 59, 73 and 87; at least one heavy chain FR3 selected from the group comprising SEQ ID No. 5, 19, 33, 47, 61, 75 and 89; at least one heavy chain FR4 selected from the group comprising SEQ ID No. 7, 21, 35, 49, 63, 77 and 91; at least one light chain FR1 selected from the group comprising SEQ ID No. 8, 22, 36, 50, 64, 78, 92, 117 and 120; at least one light chain FR2 selected from the group comprising SEQ ID No. 10, 24, 38, 52, 66, 80, 94 and 122; at least one light chain FR3 selected from the group comprising SEQ ID No. 12, 26, 40, 54, 68, 82, 96, 118 and 123, and; at least one light chain FR4 selected from the group comprising SEQ ID No. 14, 28, 42, 56, 70, 84, 98 and 125.

An "Fv" fragment is an antibody fragment that contains complete antibody recognition and binding sites. This region consists of one heavy chain variable domain and one light chain variable domain, for example, dimers substantially tightly covalently associated with scFv.

A "Fab" fragment contains the variable and constant domains of the light chain and the variable and first constant domain (CH1) of the heavy chain. F(ab')2 antibody fragments generally comprise a pair of Fab fragments covalently linked by their hinge cysteine near their carboxy ends.

A "single chain Fv" or "scFv" antibody fragment comprises the VH and VL domains of an antibody, which domains are within a single polypeptide chain. The Fv polypeptide may further comprise a polypeptide linker between the VH domain and the VL domain such that the scFv can form the desired structure for antigen binding.

The antibody according to the present invention may be monovalent or divalent, and comprises a single chain or a double chain.

Functionally, the binding affinity of the antibody to the extracellular domain of DLK1 is in a range of $10^{-5}$M to $10^{-12}$M. For example, the binding affinity is $10^{-6}$M to $10^{-12}$M, $10^{-7}$M to $10^{-12}$M, $10^{-8}$M to $10^{-12}$M, $10^{-9}$M to $10^{-12}$M, $10^{-5}$M to $10^{-11}$M, $10^{-6}$M to $10^{-11}$M, $10^{-7}$M to $10^{-11}$M, $10^{-8}$M to $10^{-11}$M, $10^{-9}$M to $10^{-11}$M, $10^{-10}$M to $10^{-11}$M, $10^{-5}$M to $10^{-10}$M, $10^{-6}$M to $10^{-10}$M, $10^{-7}$M to $10^{-10}$M, $10^{-8}$M to $10^{-10}$M, $10^{-9}$M to $10^{-10}$M, $10^{-5}$M to $10^{-9}$M, $10^{-6}$M to $10^{-9}$M, $10^{-7}$M to $10^{-9}$M, $10^{-8}$M to $10^{-9}$M, $10^{-5}$M to $10^{-8}$M, $10^{-6}$M to $10^{-8}$M, $10^{-7}$M to $10^{-8}$M, $10^{-5}$M to $10^{-7}$M, $10^{-6}$M to $10^{-7}$M or $10^{-5}$M to $10^{-6}$M.

Further, the antibodies of the present invention are antibodies with increased affinity for the antigen. The term "affinity" refers to the ability to specifically recognize and bind to specific sites of an antigen. High specificity, together with the specificity of these antibodies, is an important factor in the immune response. Any of various analyses known to the art, for example radioimmunoassays (RIA) and ELISA may be used to determine affinity, which may be expressed as various quantitative values. The affinity of an antibody to an antigen may generally be represented by the dissociation constant (Kd) of a specific antibody-antigen interaction. A lower Kd value indicates higher affinity of an antibody to an antigen. For example, the Kd value of the 18A5 antibody of the present invention is 0.52, and that of the 27F7 antibody is 0.22. This indicates these are high-affinity antibodies which bind with specificity to human DLK1.

The antibody binding to the extracellular domain of DLK1, or antigen-binding fragment thereof, may comprise a heavy chain variable region including a sequence that has at least 90% sequence homology with a sequence selected from the group comprising SEQ ID No. 99, 101, 103, 105, 107, 109, 111 and 127. The antibody binding to the extracellular domain of DLK1, or antigen-binding fragment, thereof, may comprise a heavy chain variable region selected from the group comprising SEQ ID No. 99, 101, 103, 105, 107, 109, 111 and 127.

Further, the antibody binding to the extracellular domain of DLK1, or antigen-binding fragment thereof, may comprise a light chain variable region including a sequence that has at least 90% sequence homology with a sequence selected from the group comprising SEQ ID No. 100, 102, 104, 106, 108, 110, 112, 126 and 128. The antibody binding to the extracellular domain of DLK1, or antigen-binding fragment, thereof, may comprise a light chain variable region selected from the group comprising SEQ ID No. 100, 102, 104, 106, 108, 110, 112, 126 and 128.

The antibody or antibody fragment of the present invention may contain, within the scope of specifically recognizing DLK1, the sequence of the anti-DLKl antibody of the present invention described herein as well as biological equivalents thereof. For example, additional changes may be made to the amino acid sequence of the antibody to further improve the binding affinity and / or other biological properties of the antibody. Such modifications include, for example, deletion, insertion and / or substitution of the amino acid sequence residues of the antibody. Such amino acid variations are made based on the relative similarity of the amino acid side chain substituents, such as hydrophobicity, hydrophilicity, charge, size, and the like. By analysis of the size, shape and type of amino acid side chain substituents, arginine, lysine and histidine are both positively charged residues; Alanine, glycine and serine have similar sizes; Phenylalanine, tryptophan and tyrosine have similar shapes. Thus, based on these considerations, arginine, lysine and histidine; alanine, glycine and serine; and phenylalanine, tryptophan and tyrosine are biologically functional equivalents.

Considering the mutation having the above-mentioned biological equivalent activity, the antibody of the present invention or the nucleic acid molecule encoding the same is interpreted to include a sequence showing substantial identity with the sequences under SEQ ID NO. The above-mentioned substantial identity is determined by aligning the above-described sequence of the present invention with any other sequence as much as possible and analyzing the

aligned sequence using an algorithm commonly used in the art, with a homology of at least 90%, and most preferably at least 95% homology, at least 96%, at least 97%, at least 98%, or at least 99%.

Alignment methods for sequence comparison are well known in the art. The NCBI Basic Local Alignment Search Tool (BLAST) is accessible from NBCI and may be used in conjunction with sequence analysis programs such as blastp, blasm, blastx, tblastn and tblastx on the Internet. BLSAT is available at www.ncbi.nlm.nih.gov/BLAST/. A comparison of sequence homology using this program may be found at www.ncbi.nlm.nih.gov/BLAST/blast help.html.

Based on this, the antibody or antigen-binding fragment thereof of the present invention is 90%, 91%, 92%, 93%, 94%, 95%, 96%, 97%, 98%, 99% compared to the specified sequence or all described in the specification. Such homology may be determined by sequence comparison and / or alignment by methods known in the art. For example, sequence comparison algorithms (i.e., BLAST or BLAST 2.0), manual alignment, visual inspection may be used to determine the percent sequence homology of nucleic acids or proteins of the invention.

In another aspect, the present invention relates to a nucleic acid encoding the antibody or antigen-binding fragment thereof.

The nucleic acid encoding the antibody or antigen-binding fragment thereof of the present invention may be isolated to recombinantly produce the antibody or antigen-binding fragment thereof. The nucleic acid is isolated and inserted into a replicable vector for further cloning (amplification of DNA) or for further expression. Based on this, the present invention relates to a vector comprising the nucleic acid in another aspect.

"Nucleic acid" is meant to encompass DNA (gDNA and cDNA) and RNA molecules inclusively, and the nucleotides that are the basic building blocks of nucleic acids include natural nucleotides as well as analogs with modified sugar or base sites. The sequences of nucleic acids encoding heavy and light chain variable regions of the invention may be modified. Such modifications include addition, deletion, or non-conservative or conservative substitutions of nucleotides.

The DNA encoding the antibody is readily isolated or synthesized using conventional procedures (e.g., by using oligonucleotide probes capable of specifically binding to the DNA encoding the heavy and light chains of the antibody). Many vectors are available. Vector components generally include, but are not limited to, one or more of the following: signal sequence, origin of replication, one or more marker genes, enhancer elements, promoters, and transcription termination sequences.

As used in the present specification, the term "vector" refers to a plasmid vector as a means for expressing a gene of interest in a host cell; cosmid vector; viral vectors such as bacteriophage vectors, adenovirus vectors, retrovirus vectors, and adeno-associated virus vectors, and the like. The nucleic acid encoding the antibody in the vector is operably linked with a promoter.

"Operatively linked" means a functional binding between a nucleic acid expression control sequence (e.g., an array of promoters, signal sequences, or transcriptional regulator binding sites) and another nucleic acid sequence, whereby the regulatory sequence is the other nucleic acid. To control transcription and / or translation of the sequence.

In the case of a prokaryotic cell as a host, powerful promoters capable of promoting transcription (e.g., tac promoter, lac promoter, lacUV5 promoter, lpp promoter, pL$\lambda$ promoter, pR$\lambda$ promoter, rac5 promoter, amp promoter, recA promoter, SP6 promoter, trp promoter and T7 promoter, etc.), ribosome binding sites for initiation of translation, and transcription / translation termination sequences. Also, for example, when the eukaryotic cell is a host, a promoter derived from the genome of the mammalian cell (e.g., a metallothionine promoter, a $\beta$-actin promoter, a human hemoglobin promoter and a human muscle creatine promoter) or a mammal Promoters derived from animal viruses (e.g., adenovirus late promoter, vaccinia virus 7.5K promoter, SV40 promoter, cytomegalovirus (CMV) promoter, tk promoter of HSV, mouse breast tumor virus (MMTV) promoter, LTR promoter of HIV) , promoter of the Moroni virus, promoter of the Epsteinbar virus (EBV) and Loose Sacoma virus (RSV) promoter) may be used, and generally has a polyadenylation sequence as a transcription termination sequence.

In some cases, the vector may be fused with other sequences to facilitate purification of the antibody expressed therefrom. Sequences to be fused include, for example, glutathione S-transferase (Pharmacia, USA), maltose binding protein (NEB, USA), FLAG (IBI, USA) and 6x His (hexahistidine; Quiagen, USA).

Such vectors include antibiotic resistance genes commonly used in the art as selection markers and include, for example, resistance genes against ampicillin, gentamicin, carbenicillin, chloramphenicol, streptomycin, kanamycin, geneticin, neomycin and tetracycline.

In another aspect, the present invention relates to a cell transformed with the above-mentioned vector. The cells used to produce the antibodies of the invention may be prokaryote, yeast or higher eukaryote cells, but are not limited thereto.

Bacterial strains such as Escherichia coli, Bacillus subtilis and Bacillus thuringiensis, Streptomyces and Pseudomonas (e.g. Pseudomonas putida), and prokaryotic host cells such as Proteus mirabilis and Staphylococcus (e.g., Staphylococcus carnosus) may be used.

However, animal cells are of the greatest interest, and examples of useful host cell lines are, but are not limited to, COS-7, BHK, CHO, CHOK1, DXB-11, DG-44, CHO / -DHFR, CV1, COS-7, HEK293, BHK, TM4, VERO, HELA,

MDCK, BRL 3A, W138, Hep G2, SK-Hep, MMT, TRI, MRC 5, FS4, 3T3, RIN, A549, PC12, K562, PER.C6, SP2/0, NS-0, U20S, or HT1080.

In another aspect, the present invention relates to a method for preparing the above-stated antibodies or antigen-binding fragments thereof, the method comprising: (a) a step of culturing the cells; and (b) a step of recovering the antibody or antigen-binding fragment thereof from the cultured cells.

The cells may be cultured in various media. Commercially available media may be used without limitation as the culture medium. All other necessary supplements known to those skilled in the art may be included at appropriate concentrations. Culture conditions, such as temperature, pH, and the like, are already in use with host cells selected for expression, which will be apparent to those skilled in the art.

In the recovery of the antibody or antigen-binding fragment thereof, impurities may be removed by, for example, centrifugation or ultrafiltration, and the result may be purified using, for example, affinity chromatography or the like. Other purification techniques such as anion or cation exchange chromatography, hydrophobic interaction chromatography, hydroxylapatite chromatography and the like may be used.

In one aspect of the present invention, the linker between the antibody and the active agent may be cleavable.

In one aspect of the present invention, the linker has the structure of Chemical Formula IIa below:

[Chemical Formula IIa]

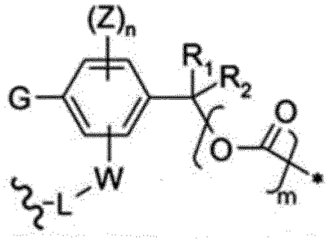

Where:

G is a sugar, a sugar acid or a sugar derivative;
W is -C(O)-, -C(O)NR'-, -C(O)O-, $-S(O)_2NR'-$, -P(O)R"NR'-, -S(O)NR'-, or $-PO_2NR'-$; In a case where C(O), S or P is linked directly with the phenyl ring, R' and R" are, respectively and independently, hydrogen, $(C_1-C_8)$ alkyl, $(C_3-C_8)$ cycloalkyl, $(C_1-C_8)$ alkoxy, $(C_1-C_8)$ alkylthio, mono- or di-$(C_1-C_8)$ alkylamino, $(C_3-C_{20})$ heteroaryl, or $(C_6-C_{20})$ aryl;
Each Z is, respectively and independently, $(C_1-C_8)$ alkyl, halogen, cyano or nitro;
n is an integer of 0 through 3;
m is 0 or 1;
L is absent, or;
Contains at least one branching unit (BR) and at least one connection unit;
$R_1$ and $R_2$ are, respectively and independently, hydrogen, $(C_1-C_8)$ alkyl or $(C_3-C_8)$ cycloalkyl, or the $R_1$ and $R_2$, together with the carbon atoms to which they are bound, form a $(C_3-C_8)$ cycloalkyl ring, and;
In the above formula, '~' denotes a region which binds to an antibody, and * denotes a region which binds to a drug or a toxin.

In one aspect of the present invention, the sugar or sugar acid is a monosaccharide.

In one aspect of the present invention, G is a glucuronic acid moiety or a compound of the structure of Chemical Formula (IIIa) below:

[Chemical Formula (IIIa)]

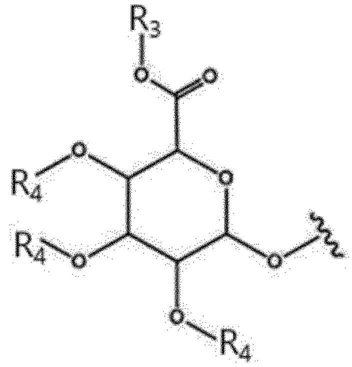

Where:

$R_3$ is a hydrogen or carboxyl protective group, and;
Each $R_4$ is, respectively and independently, a hydrogen or hydroxyl protective group.

In one aspect of the present invention, $R_3$ is hydrogen, and each $R_4$ is hydrogen.
Further $R_1$ and $R_2$ are respectively hydrogen.
Further, each Z is, respectively and independently, ($C_1$-$C_8$) alkyl, halogen, cyano or nitro.
Further, W is -C(O)-, -C(O)NR'- or -C(O)O-. More specifically, W is -C(O)NR'-, where C(O) is linked to a phenyl ring, and NR' is linked to L.
Further, n is 0, 1, 2 or 3, specifically 0, 1 or 2, and more specifically 0.
More specifically, G is a compound of the structure of chemical formula (IIIa) below:

[Chemical Formula (IIIa)]

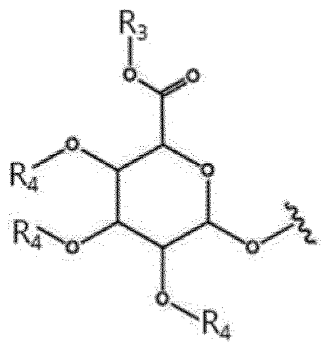

Where:

$R_3$ is a hydrogen or carboxyl protective group;
Each $R_4$ is, respectively and independently, a hydrogen or hydroxyl protective group, and;
W is -C(O)NR'-, where C(O) is linked to a phenyl ring, and NR' is linked to L, each Z is ($C_1$-$C_8$) alkyl, halogen, cyano or nitro, n is 0, m is 1, and $R_1$ and $R_2$ are respectively hydrogen.

In one aspect of the present invention, at least one branching unit is an alkylene having 1 to 100 carbon atoms, where the carbon atoms of the alkylene may be substituted by one or more heteroatoms selected from a group comprised of N, O and S, and the alkylene may further be substituted with one or more alkyls having 1 to 20 carbon atoms.
Specifically, at least one branching unit is $C_1$-$C_{50}$ alkylene or a 1 to 50 atom heteroalkylene, and may satisfy at least one of the following:

(i) the branching unit comprises at least one unsaturated bond;
(ii) 2 atoms in the branching unit are the same as in a substituent, this completing a heteroarylene;
(iii) the branching unit is a 1 to 50 atom heteroalkylene, and;

(iv) the alkylene is substituted by at least one $C_{1}$-$_{20}$ alkyl.

Further, the at least one branching unit is a nitrogen-comprising 1-50 atom heteroalkylene, the linker comprises at least 2 atoms of a hydrophilic amino acid, and the nitrogen may form a peptide bond with the carbonyl of a hydrophilic amino acid.

In one aspect of the present invention, the at least one branching unit is a hydrophilic amino acid.

In one aspect of the present invention, the hydrophilic amino acid may be arginine, aspartate, asparagine, glutamate, glutamine, histidine, lysine, ornithine, proline, serine or threonine.

Further, the hydrophilic amino acid may covalently bond an oxime of a linker to a polyethylene glycol unit of the linker.

In one aspect of the present invention, the hydrophilic amino acid may be an amino acid including a side chain having a moiety which has electric charge in aqueous solution at a neutral pH.

In one aspect of the present invention, the hydrophilic amino acid is aspartate or glutamate. In one aspect of the present invention, the hydrophilic amino acid is ornithine or lysine.

In one aspect of the present invention, the at least one branching unit is -C(O)-, -C(O)NR'-, -C(O)O-, $-S(O)_2NR'$-, -P(O)R"NR'-, -S(O)NR'-, or $-PO_2NR'$-, and R' and R" are, respectively and independently, hydrogen, $(C_1$-$C_8)$ alkyl, $(C_3$-$C_8)$ cycloalkyl, $(C_1$-$C_8)$ alkoxy, $(C_1$-$C_8)$ alkylthio, mono- or di-$(C_1$-$C_8)$ alkylamino, $(C_3$-$C_{20})$ heteroaryl, or $(C_6$-$C_{20})$ aryl.

In one aspect of the present invention, the at least one branching unit is -C(O)NR'-, and R' is hydrogen.

In one aspect of the present invention, at least one connecting unit is represented by General Formula VIII or General Formula IX:

[General Formula VIII] $-(CH_2)_r(V(CH_2)_p)_q-$

[General Formula IX] $-(CH_2CH_2X)_w-$

Where: V is a single bond, -O-, -S-, $-NR^{21}$-, $-C(O)NR^{22}$-, $NR^{23}C(O)$- , $NR^{24}SO_2$-, or $-SO_2NR^{25}$-; X is -O-, $C_1$-$C_8$ alkylene or $-NR^{21}$-;
$R^{21}$ to $R^{25}$ are, independently and respectively hydrogen, $(C_1$-$C_6)$ alkyl, $(C_1$-$C_6)$ alkyl $(C_6$-$C_{20})$ aryl, or $(C_1$-$C_6)$ alkyl $(C_3$-$C_{20})$ heteroaryl;
r is an integer of 0 to 10;
p is an integer of 0 to 10;
q is an integer of 1 to 20, and;
w is an integer of 1 to 20.

In one aspect of the present invention, q may be 4 to 20, more specifically 6 to 20. Further, q may be 2 to 12, more specifically 2, 5 or 11. Further, may be 2. Further, p may be 2. Further, V may be -O-.

More specifically, r may be 2, p may be 2, q may be 2, 5 or 11, and V may be -O-.

Further, in one aspect of the present invention, X may be -O-. Further, w may be an integer of 6 to 20.

More specifically, X may be -O-, and w may be 6 to 20.

In one aspect of the present invention, the at least one connecting unit comprises at least one polyethylene glycol unit represented by

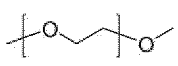 or 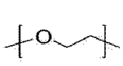,

where n is 1 to 12.

In one aspect of the present invention, the at least one connecting unit may be 1 to 12 - $OCH_2CH_2$- units, 3 to 12 $-OCH_2CH_2$- units, 5 to 12 $-OCH_2CH_2$- units, 6 to 12 $-OCH_2CH_2$- units, or 3 $-OCH_2CH_2$- units.

In one aspect of the present invention, at least one connection unit is $-(CH_2CH_2X)_w-$,

Where X is a single bond, -O-, $(C_1$-$C_8)$ alkylene, or $-NR_{21}$-;
$R_{21}$ is hydrogen, $(C_1$-$C_6)$ alkyl, $(C_1$-$C_6)$ alkyl $(C_6$-$C_{20})$ aryl, or $(C_1$-$C_6)$ alkyl $(C_3$-$C_{20})$ heteroaryl, and;
w is an integer of 1 to 20, specifically 1, 3, 6 or 12.
In one aspect of the present invention, X is -O-, and w is an integer of 6 to 20.

In one aspect of the present invention, the linker further comprises binding units formed by 1,3-dipolar cycloaddition reactions, hetero-diels reactions, nucleophilic substitution reactions, non-aldol type carbonyl reactions, additions to carbon-carbon multiple bonds, oxidation reactions or click reactions.

In one aspect of the present invention, the binding unit is formed by a reaction between acetylene and azide, or a reaction between aldehyde or a ketone group and hydrazine or alkoxyamine.

In one aspect of the present invention, the binding unit is:
or

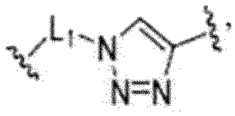,
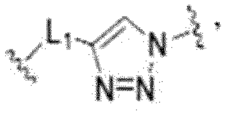,
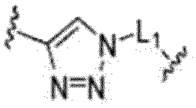
또는
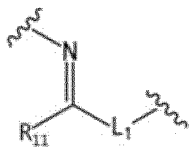
,

Where,

$L_1$ is a single bond or an alkylene having 1 to 30 carbon atoms;
$R_{11}$ is hydrogen or an alkyl having 1 to 10 carbon atoms, specifically methyl.

In one aspect of the present invention, $L_1$ is a single bond, an alkylene having 11 carbon atoms, or an alkylene having 12 carbon atoms.

Further, in one aspect of the invention, the binding unit comprises

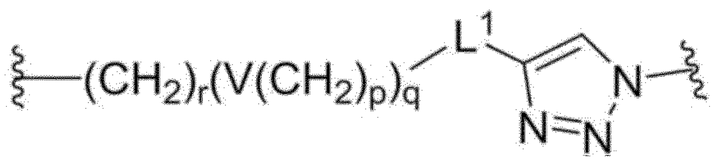

or

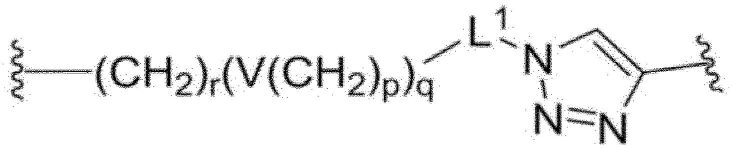
,

V is a single bond, -O-, -S-, $-NR^{21}-$, $-C(O)NR^{22}-$, $NR^{23}C(O)-$, $NR^{24}SO_2-$, or $-SO_2NR^{25}-$;
$R^{21}$ to $R^{25}$ are, independently and respectively hydrogen, $C_1$-$C_6$ alkyl, $C_1$-$C_6$ alkyl $C_6$-$C_{20}$ aryl, or $C_1$-$C_6$ alkyl $C_3$-$C_{20}$ heteroaryl;
r is an integer of 1 to 10;
p is an integer of 0 to 10;
q is an integer of 1 to 20, and;
$L^1$ is a single bond.

In one aspect of the present invention, r may be 2 or 3. Further, p may be 1 or 2. Further, q may be 1 through 6.

More specifically, in the binding unit, r may be 2 or 3; p may be 1 or 2, and; q may be 1 through 6.

Further, in one aspect of the present invention, the binding unit may be

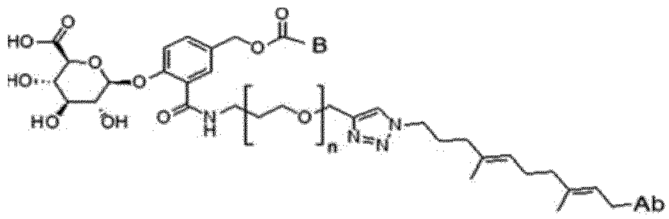
,

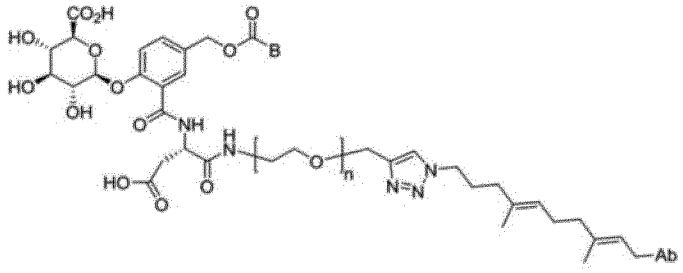

or;

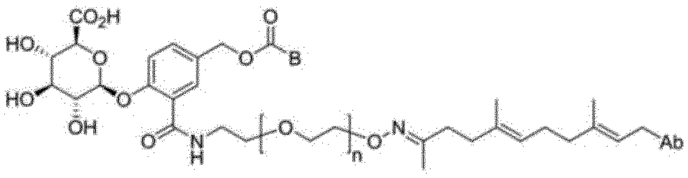

where:

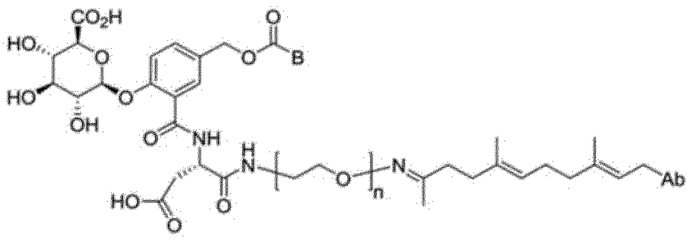

where Ab is an anti-DLKI antibody; B is an active agent, and; n is an integer of 0 to 20.

In one aspect of the present invention, the branching unit is

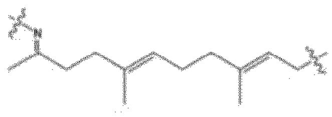

or

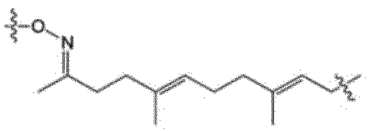

.

In one aspect of the present invention, the linker may comprise 3 to 50 heteroalkylenes including oxime,

the oxygen atom of the oxime is on the side of L linked to W and the carbon atom of the oxime is on the side of L linked to Ab, or;

the carbon atom of the oxime is on the side of L linked to W and the oxygen atom of the oxime is on the side of L linked to Ab.

In one aspect of the present invention, the linker may additionally contain at least one isoprenyl unit having the structure

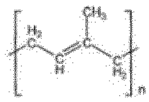

, where n is at least 2.

In one aspect of the preset invention, at least one isoprenyl unit is an isoprenoid transferase substrate or a product of isoprenoid transferase.

In one aspect of the present invention, the isoprenyl unit of the linker binds covalently with an antibody through a thioether bond, and the thioether bond includes a sulfur atom of cysteine.

Further, the isoprenyl unit may covalently bind the oxime included in the linker to an antibody.

In one aspect of the present invention, the antibody includes an amino acid motif recognized by isoprenoid transferase, and the thioether bond includes a sulfur atom of the cysteine of the amino acid motif.

In one aspect of the present invention, the antibody binding to DLK1 or antigen-binding fragment thereof includes an amino acid motif recognized by isoprenoid transferase, and the thioether bond includes a sulfur atom of the cysteine of the amino acid motif.

In one aspect of the present invention, the amino acid motif is a sequence selected from a group comprised of CXX, CXC, XCXC, XXCC and CYYX, where C denotes cysteine; Y in each case independently denotes an aliphatic amino acid; X in each case independently denotes glutamine, glutamate, serine, cysteine, methionine, alanine or leucine, and; the thioether bond includes a sulfur atom of the cysteine of the amino acid motif.

In one aspect of the present invention, the amino acid motif is a CYYX sequence, and Y in each case is independently alanine, isoleucine, leucine, methionine or valine.

In one aspect of the present invention, the amino acid motif is a CVIM or CVLL sequence.

In one aspect of the present invention, at least one of the 1 to 10 amino acids preceding the amino acid motif may, respectively and independently, be selected from among glycine, arginine, aspartic acid and serine. For example, in one aspect of the present invention, at least one of the 7 amino acids preceding the amino acid motif are glycine. Alternatively, at least 3 of the 7 amino acids preceding the amino acid motif are, respectively and independently, selected from among glycine, arginine, aspartic acid and serine. Alternatively, 1 to 10 amino acids preceding the amino acid motif are glycine. Specifically, at least 1, 2, 3, 4, 5, 6, 7, 8, 9 or 10 amino acids preceding the amino acid motif are glycine.

In one aspect of the present invention, the antibody may include the amino acid sequence GGGGGGGCVIM.

In one aspect of the present invention, L comprises at least one branch linker covalently bonded to Ab,

i) the respective branch linkers comprise a branched unit covalently bonded to Ab by a primary linker (PL);
ii) the respective branch linkers bind a first active agent to a branched unit, and comprise a first branch (B1) comprising a second linker (SL) and a cleaving group (CG), and;
iii) the respective branch linkers comprise a) a second branch (B2) wherein a second active agent is covalently bonded to a branched unit by a second linker (SL) and a cleaving group (CG), or b) a second branch wherein a polyethylene glycol moiety is covalently bonded to a branched unit,

and the respective cleaving groups are hydrolyzed to release an active agent from an antibody conjugate.

In one aspect of the present invention, the branch linker is

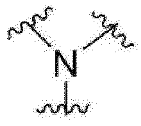 , 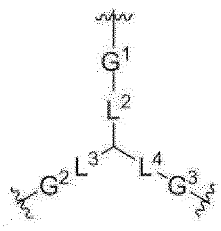 ,

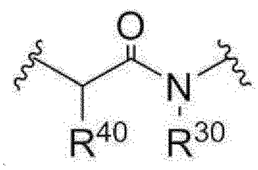 or 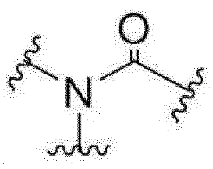 ;

$L_2$, $L_3$ and $L_4$ are, respectively and independently, direct bonds or $-C_nH_{2n}-$, n is an integer of 1 to 30,

$G^1$, $G^2$ and $G^3$ are, respectively and independently, a direct bond,

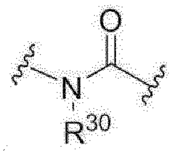

,

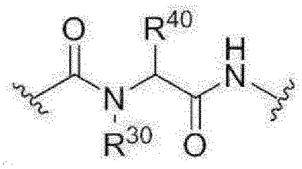

,

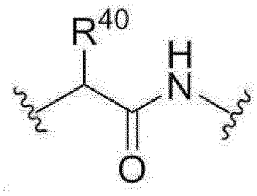

or

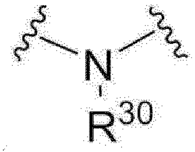

,

and;

$R^{30}$ is hydrogen or $C_{1-30}$ alky.

More specifically, the branch linker comprises

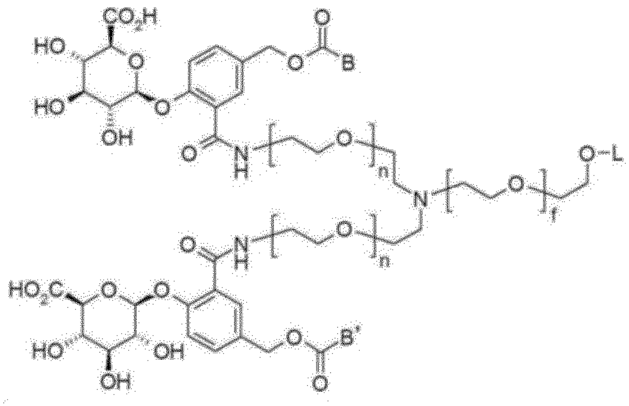

,

B and B' indicate active agents which may be different or identical;
n indicates, respectively and independently, an integer of 0 to 30;
f indicates, respectively and independently, an integer of 0 to 30, and;
L indicates a bond to Ab.

In one aspect of the present invention, n is an integer of 1 to 20, more specifically an integer of 1 to 10, or 4 to 20.

In one aspect of the present invention, L comprises oxime, and at least one polyethylene glycol unit covalently binds the oxime to an active agent.

In one aspect of the present invention, the cleaving group may be cleaved within a target cell, and the cleaving group may release one or more active agents.

In one aspect of the present invention, the linker comprises at least one branch linker covalently bonded to Ab, and comprises at least two active agents covalently linked to the branch linker.

Specifically, 1 branch linker may bind with Ab.

Further, two or more branch linkers may bind to Ab, and the respective branch linkers may bind to at least two active agents. More specifically, 3 branch linkers may bind to Ab. Alternatively, 4 branch linker may bind to Ab.

In one aspect of the present invention, the respective branch linkers bind to at least two identical or different active agents. In one aspect of the present invention, the respective active agents are bonded to the branch linker by a cleavable bond.

In one aspect of the present invention, the respective branch linkers comprise a branched unit, the respective active agents are bonded to the branched unit through a second linker, and the branched units are bonded to an antibody by a first linker.

In one aspect of the present invention, the branched unit may be a nitrogen atom. Further, the branched unit may be an amide, and the first linker or the second linker may comprise a carbonyl of the amide. Alternatively, the branched unit may be a lysine unit.

In yet another aspect of the present invention, the linker may comprise:

(a) at least one branching unit; (b) at least one connection unit; (c) at least one binding unit (BU), and; at least one trigger unit (TU).

Here, the connection unit connects the trigger unit and binding unit, the trigger unit and the branching unit, or the branching unit and the binding unit;

the at least one trigger unit may release at least one drug or toxin, and the branching unit links a connection unit and trigger unit, or a connection unit and another connection unit.

In one aspect of the present invention, the trigger unit has the structure of Chemical Formula (IIb) below:

[Chemical Formula (IIb)]

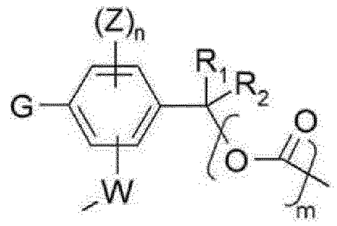

,

Where:

G is a sugar, a sugar acid or a sugar derivative;

W is -C(O)-, -C(O)NR'-, -C(O)O-, $-S(O)_2NR'-$, -P(O)R"NR'-, -S(O)NR'-, or $-PO_2NR'-$; In a case where C(O), S or P is linked directly with the phenyl ring, R' and R" are, respectively and independently, hydrogen, $(C_1-C_8)$ alkyl, $(C_3-C_8)$ cycloalkyl, $(C_1-C_8)$ alkoxy, $(C_1-C_8)$ alkylthio, mono- or di-$(C_1-C_8)$ alkylamino, $(C_3-C_{20})$ heteroaryl, or $(C_6-C_{20})$ aryl, and W is linked to a connection unit or a branching unit;

Each Z is, respectively and independently, $(C_1-C_8)$ alkyl, halogen, cyano or nitro;

n is an integer of 1 through 3;

m is 0 or 1;

$R_1$ and $R_2$ are, respectively and independently, hydrogen, $(C_1-C_8)$ alkyl or $(C_3-C_8)$ cycloalkyl, or the $R_1$ and $R_2$, together with the carbon atoms to which they are bound, form a $(C_3-C_8)$ cycloalkyl ring.

In one aspect of the present invention, the sugar or sugar acid is a monosaccharide.

In one aspect of the present invention, G is a compound having the structure of Chemical Formula (IIIa) below:

[Chemical Formula (IIIa)]

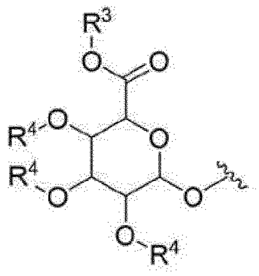

Where:

$R_3$ is a hydrogen or carboxyl protective group, and;
Each $R_4$ is, respectively and independently, a hydrogen or hydroxyl protective group.

In one aspect of the present invention, $R_3$ is hydrogen, and each $R_4$ is hydrogen.

In one aspect of the present invention, W is -C(O)NR'-, where C(O) is linked to a phenyl ring, and NR' is linked to L.

In one aspect of the present invention, Z is hydrogen.

In one aspect of the present invention, $R_1$ and $R_2$ are respectively hydrogen.

In one aspect of the present invention, the connection unit is represented as $-(CH_2)_r(V(CH_2)_p)_q-$, $-((CH_2)_pV)_q-$, $-(CH_2)_r(V(CH_2)_p)_qY-$, $-((CH_2)_pV)_q(CH_2)_r-$, $-Y((CH_2)_pV)_q-$ or $-(CH_2)_r(V(CH_2)_p)_qYCH_2-$,
Where:

r is an integer of 0 to 10;
p is an integer of 1 to 10;
q is an integer of 1 to 20;
V and Y are, independently and respectively a single bond, -O-, -S-, $-NR^{21}-$, $-C(O)NR_{22}-$, $NR_{23}C(O)-$, $NR_{24}SO_2-$, or $-SO_2NR_{25}-$ and;
$R_{21}$ to $R_{25}$ are, independently and respectively hydrogen, $(C_1-C_6)$ alkyl, $(C_1-C_6)$ alkyl $(C_6-C_{20})$ aryl, or $(C_1-C_6)$ alkyl $(C_3-C_{20})$ heteroaryl.

In one aspect of the present invention, r is 2.

In one aspect of the present invention, p is 2.

In one aspect of the present invention, q is an integer of 6 to 20.

In one aspect of the present invention, q is 2, 5 or 11.

In one aspect of the present invention, V and Y are, respectively and independently, -O-.

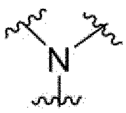 , 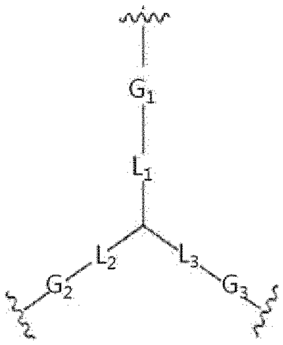 , 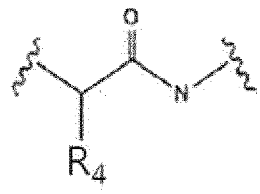 , 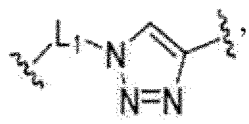 ,

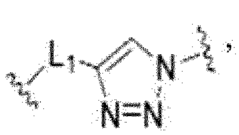 , 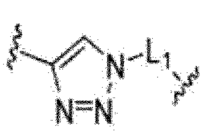 or 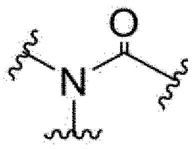 ;

,

Where:

$L_1$, $L_2$ and $L_3$ are, respectively and independently, a direct bond or $-C_nH_{2n}-$;
n is an integer of 1 through 30;

Where:

$G_1$, $G_2$ and $G_3$ are, respectively and independently, a direct bond,

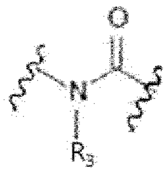,

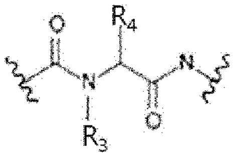, 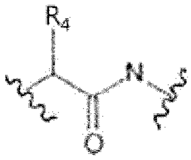 or 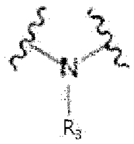,

$R_3$ is hydrogen or $C_1$-$C_{30}$ alkyl;
$R_4$ is hydrogen or $L_4$-$COOR_5$; $L_4$ is a direct bond or $-C_nH_{2n}-$; n is an integer of 1 to 10, and $R_5$ is hydrogen or $C_1$-$C_{30}$ alkyl.

In one aspect of the present invention, the branching unit is

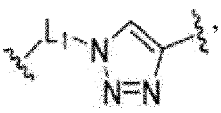, 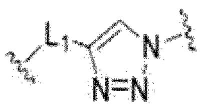, 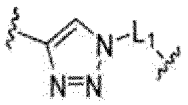 or 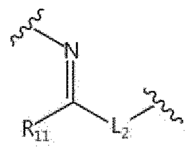,

Where $L_1$ is a direct bond or an alkylene having 1 to 30 carbon atoms;
$R_{11}$ is hydrogen or an alkyl having 1 to 10 carbon atoms, specifically methyl;
$L_2$ is an alkylene having 1 to 30 carbon atoms, and;
the branching unit links a connection unit and an antibody.

In one aspect of the present invention, $L_1$ is an alkylene having 12 carbon atoms.
In one aspect of the present invention, $R_{11}$ is methyl.
In one aspect of the present invention, $L_2$ is an alkylene having 11 carbon atoms.
In one aspect of the present invention, the branched unit is

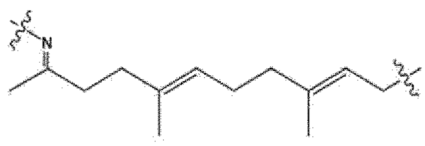 or 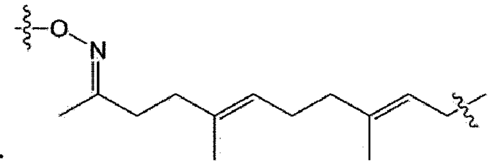.

In one aspect of the present invention, the isoprenyl unit of the linker binds covalently with an antibody through a thioether bond, and the thioether bond includes a sulfur atom of cysteine.
In one aspect of the present invention, the antibody includes an amino acid motif recognized by isoprenoid transferase, and the thioether bond includes a sulfur atom of the cysteine of the amino acid motif.
In one aspect of the present invention, the amino acid motif is a sequence selected from a group comprised of CXX, CXC, XCXC, XXCC and CYYX, where C denotes cysteine; Y in each case independently denotes an aliphatic amino acid; X in each case independently denotes glutamine, glutamate, serine, cysteine, methionine, alanine or leucine, and; the thioether bond includes a sulfur atom of the cysteine of the amino acid motif.
In one aspect of the present invention, the amino acid motif is a CYYX sequence, and Y in each case is independently alanine, isoleucine, leucine, methionine or valine.
In one aspect of the present invention, the amino acid motif is a CVIM or CVLL sequence.
In one aspect of the present invention, at least one of the 7 amino acids preceding the amino acid motif may, respectively and independently, be selected from among glycine, arginine, aspartic acid and serine.
In one aspect of the present invention, at least 3 of the 7 amino acids preceding the amino acid motif are, respectively and independently, selected from among glycine, arginine, aspartic acid and serine.

Alternatively, 1 to 10 amino acids preceding the amino acid motif are glycine. Specifically, at least 1, 2, 3, 4, 5, 6, 7, 8, 9 or 10 amino acids preceding the amino acid motif are glycine.
In one aspect of the present invention, the antibody may include the amino acid sequence GGGGGGGCVIM.
Further,, in one aspect of the present invention, the active agent may be a chemotherapeutic agent or a toxin.
Further, the active agent may be an immunoregulatory compound, anti-cancer agent, antiviral, antibacterial, antifungal, antiparasitic or a combination of these, and the active agents listed below may be selectively used:

(a) erlotinib, bortezomib, fulvestrant, sutent, letrozole, imatinib mesylate, PTK787/ZK 222584, oxaliplatin, 5-fluorouracil, leucovorin, rapamycin, lapatinib, lonafarnib, sorafenib, gefitinib, AG1478, AG1571, thiotepa, cyclophosphamide, busulfan, improsulfan, piposulfan, benzodopa, carboquone, meturedopa, uredopa, ethylenimine, altretamine, triethylenemelamine, triethylenephosphoramide, triethylenethiophosphoramide, teimethylolomelamine, bullatacin, bullataciionone, camptothecin, topotecan, bryostatin, callystatin, CC-1065, adozelesin, carzelesin, bizelesin, cryptophycin 1, cryptophycin 8, dolastatin, duocarmycin, KW-2189, CB1-TM1, eleutherobin, pancratistatin, sarcodictyin, spongistatin, chlorambucil, chlornaphazine, cholophosphamide, estramustine, ifosfamide, mechlorethamine, melphalan, novembichin, phenesterine, prednimustine, trofosfamide, uracil mustard, carmustine, chlorozotoxin, fotemustine, lomustine, nimustine, ranimustine, calicheamicin, calicheamicin gamma 1, calicheamicin omega 1, dynemicin, dynemicin A, clodronate, esperamicin, neocarzinostatin chromophore, aclacinomysins, actinomycin, antrymycin, azaserine, bleomycins, catcinomycin, carabicin, carninomycin, carzinophilin, chromomycins, dactinomycin, daunorubicin, detorubucin, 6-diazo-5-oxo-L-norleucine, doxorubicin, morpholino-doxorubicin, cyanomorpholino-doxorubicin, 2-pyrrolino-doxorubucin, liposomal doxorubicin, deoxydoxorubicin, epirubicin, esorubicin, marcellomycin, mitomycin C, mycophenolic acid, nogalamycin, olivomycins, peplomycin, potfiromycin, puromycin, quelamycin, rodorubicin, streptomigrin, streptozocin, tudercidin, ubenimex, zinostatin, zorubicin, 5-fluoDLKacil, denopterin, methotrexate, pteropterin, trimetrexate, fludarabine, 6-mercaptopurine, thiamiprine, thiguianine, ancitabine, azacytidine, 6-azauridine, carmofur, cytarabine, dideoxyuridine, doxifluridine, enocitabine, floxuridine, calusterone, dromostanolone, propionate, epitiostanol, mepitiostane, testolactone, aminoglutethimide,mitotane, trilostane, folinic acid, aceglatone, aldophosphamide glycoside, aminolevulinic acid, eniluracil, amsacrine, bestrabucil, bisantrene, edatrexate, defofamine, democolcine, diaziquone, elfornithine, elliptinium acetate, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, polysaccharide-k, razoxane, rhizoxin, sizofiran, spirogermanium, tenuazonic acid, triaqizuone, 2,2', 2''-trichlorotriethylamine, T-2 toxin, verracurin A, roridin A, anguidine, urethane, vindesine, dacarbazine, mannomustine, mitobronitol, mitolactol, pipobroman, gacytosine, arabinoside, cyclophosphamide, thiotepa, paclitaxel, albumin-engineered nanoparticle formulation of paclitaxel, docetaxel, chlorambucil, gemcitabine, 6-thioguanine, mercaptopurine, cisplatin, carboplatin, vinblastine, platinum, etoposide, ifosfamide, mitoxantrone, vincristine, vinorelbine, novantrone, teniposide, edatrexate, daunomycin, aminopterin, xeloda, ibandronate, CPT-11, topoisomerase inhibitor RFS 2000, difluoromethylornithine, retinoic acid, capecitabine, or pharmaceutically acceptable salts, solvates or acids thereof;
(b) monokine, lympokine, traditional polypeptide hormone, parathyroid hormone, thyroxine, relaxin, pDLKelaxin, glycoprotein hormone, follicle stimulating hormone, thyroid stimulating hormone, luteinizing hormone, hepatic growth factor fibroblast growth factor, prolactin, placental lactogen, tumor necrosis factor, tumor necrosis factor-a, tumor necrosis factor-β, Mullerian inhibiting substance, mouse gonadotropin associated peptide, inhibin, activin, vascular endothelial growth factor, thrombopoietin, erythropoietin, osteoinductive factor, interferon, interferon-α, interferon-β, interferon-γ, colony stimulating factor (CSF), macrophage-CSF, granulocyte-macrophage-CSF), granulocyte-macrophage-CSF, granulocyte-CSF, interleukin (IL), IL-1, IL-1α, IL-2, IL-3, IL-4, IL-5, IL-6, IL-7, IL-8, IL-9, IL-10, IL-11, IL-12, tumor necrosis factor, TNF-α, TNF-β, polypeptide factor, LIF, kit ligand, or mixtures thereof;
(c) diphtheria toxin, botulinum toxin, tetanus toxin, dysentery toxin, cholera toxin, amanitin, α-amatinin, pyrrolobenzodiazepine, pyrrolobenzodiazepine derivative, indolinobenzodiazepine, pyridobenzodiazepine, tetrodotoxin, brevetoxin, ciguatoxin, ricin, AM toxin, auristatin, tubulysin, geldanamycin, maytansinoid, calicheamicin, daunomycin, doxorubicin, methotrexate, vindesine, SG2285, dolastatin, dolastatin analog, auristatin, cryptophycin, camptothecin, rhizoxin, rhizoxin derivatives, CC-1065, CC-1065 analogs or derivatives, duocarmycin, enediyne antibiotic, esperamicin, epothilone, toxoid, or mixtures thereof;
(d) affinity ligand, where the affinity ligand is a substrate, inhibitor, active agent, neurotransmitter, radioactive isotope, or mixtures thereof;
(e) radioactive label, 32P, 35S, fluorescent dye, electron dense reagent, enzyme, biotin, streptavidin, dioxigenin, hapten, immunogenic protein, nucleic acid molecule with a sequence complementary to a target, or mixtures thereof;
(f) immunomodulatory compound, anti-cancer agent, anti-viral agent, anti-bacterial agent, antifungal agent, anti-parasitic agent, or mixtures thereof;
(g) tamoxifen, raloxifene, droloxifene, 4-hydroxytamoxifen, trioxifene, keoxifene, LY117018, onapristone or toremifene;

(h) 4(5)-imidazole, aminoglutethimide, megestrol acetate, exemestane, letrozole or anastrozole
(i) flutamide, nilutamide, bicalutamide, leuprolide, goserelin, or troxacitabine;
(j) aromatase inhibitor;
(k) protein kinase inhibitor;
(l) lipid kinase inhibitor;
(m) antisense oligonucleotide;
(n) ribozyme;
(o) vaccine, and;
(p) anti-angiogenic agent.

Yet another aspect of the present invention provides, in relation to the preparation of a drug for prevention or treatment of a proliferative, cancer or angiogenetic disease, a use of an antibody-drug conjugate or a pharmaceutically acceptable salt or solvate thereof.

Yet another aspect of the present invention provides a method for drug for prevention or treatment of a proliferative, cancer or angiogenetic disease by administering an individual with a pharmaceutical composition for prevention or treatment of a proliferative, cancer or angiogenetic disease, the pharmaceutical composition comprising an antibody-drug conjugate or a pharmaceutically acceptable salt or solvate thereof as an active ingredient, wherein the pharmaceutical composition is mixed and administered with at least one anti-proliferative, cytostatic or cytotoxic substance.

Yet another aspect of the present invention provides an antibody-drug conjugate of the General Formula Ia below:

[General Formula Ia] $\quad$ Ab-(Linker-D)$_n$,

Where:

Ab is an anti-DLKl antibody;
Linker is a linker;
D is a pyrrolobenzodiazepine dimer as an active agent, and;
the linker and the antibody are linked through the N10 or N10' position of the pyrrolobenzodiazepine dimer.

Here, a pyrrolobenzodiazepine dimer prodrug, a pharmaceutically acceptable salt or solvate of the same, where: at the N10 and N10' positions of the pyrrolobenzodiazepine dimer, respectively and independently, are attached any one selected from the group comprised of - C(O)O*, -S(O)O*, -C(O)*, -C(O)NR*, -S(O)$_2$NR*, -(P(O)R')NR*, -S(O)NR* and -PO$_2$NR* groups;

where * is a region where a linker is attached;
where R and R' are respectively and independently H, OH, $N_3$, CN, $NO_2$, SH, $NH_2$, $ONH_2$, $NHNH_2$, halo, substituted or unsubstituted $C_{1-8}$ alkyl, substituted or unsubstituted $C_{3-8}$ cycloalkyl, substituted or unsubstituted $C_{1-8}$ alkoxy, substituted or unsubstituted $C_{1-8}$ alkylthio, substituted or unsubstituted $C_{3-20}$ heteroaryl, substituted or unsubstituted $C_{5-20}$ aryl, or mono- or di-$C_{1-8}$ alkylamino, and;
where, in a case where the $C_{1-8}$ alkyl, $C_{3-8}$ cycloalkyl, $C_{1-8}$ alkoxy, $C_{1-8}$ alkylthio, $C_{3-20}$ heteroaryl or $C_{5-20}$ aryl is substituted, the substitution is by a substitution group selected from a group comprising H, OH, $N_3$, CN, $NO_2$, SH, $NH_2$, $ONH_2$, $NNH_2$, halo, $C_{1-6}$ alkyl, $C_{1-6}$ alkoxy and C6-12 aryl.

A pyrrolobenzodiazepine dimer prodrug, pharmaceutically acceptable salt or solvate thereof, may be used as an active agent.

In one aspect of the present invention, a pyrrolobenzodiazepine dimer precursor is provided. In a case of administering in the form of the precursor according to the present invention, there are advantages over conventional PBD drugs in that: additional reactions are necessary for conversion to an efficacious drug upon exposure to blood, preventing the potential for adverse reactions which may arise when the linker is dissolved unexpectedly; in that toxicity to normal cells is reduced, and in that the drug is more stable.

Further, when preparing antibody-drug conjugates, whereas antibody-drug conjugate prepared using conventional methods has high impurity content, and the exposed imine group is subject to nucleophile attack, posing a risk of a drug of an undesirable structure being generated. On the other hand, an antibody-drug conjugate prepared using the method according to the present invention has an advantage of ease of isolation due to high purity, and further improved physical properties over conventional PBD or PBD dimer.

In one aspect of the present invention, the pyrrolobenzodiazepine dimer precursor is a pyrrolobenzodiazepine dimer precursor characterized in that it has the structure of General Formula X or General Formula XI below, or a

pharmaceutically acceptable salt or solvate thereof:

[General Formula X]

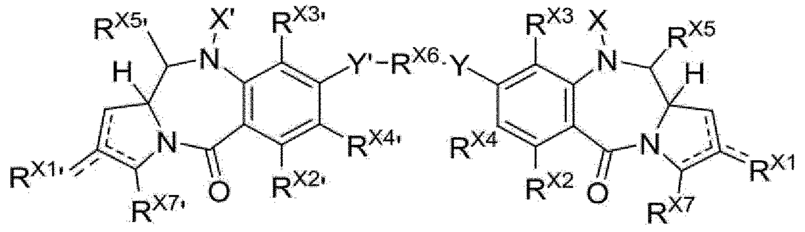

[General Formula XI]

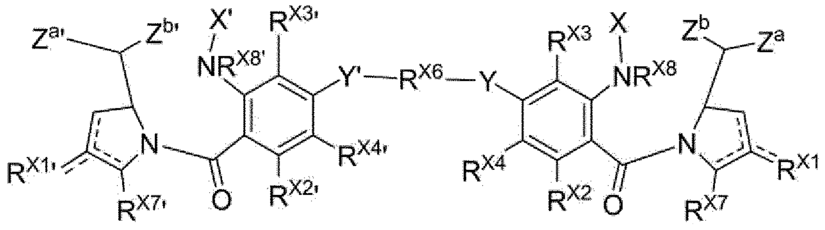

In the above formulae:

The dotted lines denote the possible existence of double bonds between C1 and C2, between C2 and C3, between C'1 and C'2, or between C'2 and C'3;

$R^{X1}$ and $R^{X1'}$ are independently selected from H, OH, =O, $=CH_2$ CN, $R^m$ $OR^m$, $=CH-R^{m'}$, $=C(R^{m'})_2$, $O-SO_2-R^m$, $CO_2R^m$, $COR^m$, halo and dihalo;

$R^{m'}$ is selected from among $R^m$, $CO_2R^m$, $COR^m$, CHO, $CO_2H$ and halo;

each $R^m$ is independently selected from a group comprised of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl and 5 to 7 membered heteroaryl;

$R^{X2}$, $R^{X2'}$, $R^{X3}$, $R^{X3'}$, $R^{X5}$ and $R^{X5'}$ are independently selected from among H, $R^m$, OH, $OR^m$, SH, $SR^m$, $NH_2$, $NHR^m$, $NR^m{}_2$, $NO_2$, $Me_3SN$ and halo;

$R^{X4}$ and $R^{X4'}$ are independently selected from among H, $R^m$, OH, $OR^m$, SH, $SR^m$, $NH_2$, $NHR^m$, $NR^m{}_2$, $NO_2$, $Me_3SN$, halo, $C_{1-6}$ alkyl, $C_{1-6}$ alkoxy, $C_{2-6}$ alkenyl, $C_{2-6}$ alkynyl, $C_{3-6}$ cycloalkyl, 3 to 7 membered heterocycloalkyl, $C_{5-12}$ aryl, 5 to 7 membered heteroaryl, - CN, -NCO, $-OR^n$, $-OC(O)R^n$, $-OC(O)NR^nR^{n'}$, $-OS(O)R^n$, $-OS(O)_2R^n$, $-SR^n$, $-S(O)R^n$, - $S(O)_2R^n$, $-S(O)NR^nR^{n'}$, $-S(O)_2NR^nR^{n'}$, $-OS(O)NR^nR^{n'}$, $-OS(O)_2NR^nR^{n'}$, $-NR^nR^{n'}$, - $NR^nC(O)R^{\circ}$, $-NR^nC(O)OR^{\circ}$, $-NR^nC(O)NR^{\circ}R^{\circ'}$, $-NR^nS(O)R^{\circ}$, $-NR^nS(O)_2R^{\circ}$, - $NR^nS(O)NR^{\circ}R^{\circ'}$, $-NR^nS(O)_2NR^{\circ}R^{\circ'}$, $-C(O)R^n$, $-C(O)OR^n$ and $-C(O)NR^nR^{n'}$;

$R^X$ and $R^{X'}$ are independently selected from among H, OH, $N_3$, CN, $NO_2$, SH, $NH_2$, $ONH_2$, $NHNH_2$, halo, $C_{1-8}$ alkyl, $C_{3-8}$ cycloalkyl, $C_{1-8}$ alkoxy, $C_{1-8}$ alkylthio, $C_{3-20}$ heteroaryl, $C_{5-20}$ aryl or mono- or di-$C_{1-8}$ alkylamino;

Y and Y' are independently selected from O, S and N(H);

$R^{x6}$ is independently selected from $C_{3-12}$ alkylene, $C_{3-12}$ alkenylene, or $C_{3-12}$ heteroalkylene;

$R^{X7}$ and $R^{X7'}$ are independently selected from among H, $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, 5 to 7 membered heteroaryl, - $OR^r$, $-OC(O)R^r$, $-OC(O)NR^rR^{r'}$, $-OS(O)R^r$, $-OS(O)_2R^r$, $-SR^r$, $-S(O)R^r$, $-S(O)_2R^r$, - $S(O)NR^rR^{r'}$, $-S(O)^2NR^rR^{r'}$, $-OS(O)NR^rR^{r'}$, $-OS(O)_2NR^rR^{r'}$, $-NR^rR^{r'}$, $-NR^rC(O)R^s$, - $NR^rC(O)OR^s$, $-NR^rC(O)NR^sR^{s'}$, $-NR_rS(O)R^s$, $-NR_rS(O)_2R^s$, $-NR_rS(O)NR^sR^{s'}$, - $NR_rS(O)_2NR^sR^s$, $-C(O)R^r$, $-C(O)OR^s$ or $-C(O)NR^rR^{r'}$;

each $R^r$, $R^{r'}$, $R^s$ and $R^{s'}$ is independently selected from H, $C_{1-7}$ alkyl, $C_{2-7}$ alkenyl, $C_{2-7}$ alkynyl, $C_{3-13}$ cycloalkyl, 3 to 7 membered heterocycloalkyl, $C_{5-10}$ aryl and 5 to 7 membered heteroaryl;

each $R^{X8}$ and $R^{X8'}$ is independently selected from H, $C_{1-6}$ alkyl, $C_{2-6}$ alkenyl, $C_{2-6}$ alkynyl, $C_{3-6}$ heteroalkyl, 3 to 7 membered heterocycloalkyl, $C_{5-10}$ aryl, 5 to 7 membered heteroaryl, - $S(O)R^m$, $-S(O)_2R^m$, $-S(O)NR^mR^{m'}$, $-S(O)_2NR^mR^{m'}$, $-NR^mR^{m'}$, $-NR^mC(O)R^m$, - $NR^mC(O)OR^n$, $-NR^mC(O)NR^nR^{n'}$, $-NR^mS(O)R^n$, $-NR^mS(O)_2R^n$,

$-NR^mS(O)NR^nR^{n'}$, - $NR^mS(O)_2NR^nR^{n'}$, $-C(O)R^m$, $-C(O)OR^m$ and $-C(O)NR^mR^{m'}$;

$Z^a$ is selected from among $OR^{X12a}$, $NR^{X12a}R^{X12a}$, or $SR^{X12a}$;

$Z^b$ is selected from among $OR^{X13a}$, $NR^{X13a}R^{X13a}$, or $SR^{X13a}$;

$Z^{a'}$ is selected from among $OR^{X12a}$, $NR^{X12a}R^{X12a}$, or $SR^{X12a}$;

$Z^{b'}$ is selected from among $OR^{X13a'}$, $NR^{X13a'}R^{X13a'}$, or $SR^{X13a'}$;

each $R^{X12a}$, $R^{X12a'}$, $R^{X13a'}$ and $R^{x13a'}$ is independently selected from H, $C_{1-6}$ alkyl, $C_{2-6}$ alkenyl, $C_{2-6}$ alkynyl, $C_{3-6}$ cycloalkyl, 3 to 7 membered heterocycloalkyl, $C_{5-10}$ aryl, 5 to 7 membered heteroaryl, $-C(O)R^{X15a}$, $-C(O)OR^{X15a}$ and $-C(O)NR^{X15a}R^{X15a'}$; and;

each $R^{X15a}$ and $R^{x15a'}$ is independently selected from $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl, and 5 to 7 membered heteroaryl, and:

The $R^{X13a}$ and $R^{X14a}$ optionally join the atoms to which they are attached to form 3 to 7 membered heterocyclyl, 3 to 7 membered heterocycloalkyl, or 3 to 7 membered heteroaryl, and the $R^{X13a'}$ and $R^{X14a'}$ optionally join theatoms to which they are attached to form 3 to 7 membered heterocyclyl, 3 to 7 membered heterocycloalkyl, or 3 to 7 membered heteroaryl, and;

each of the $R^n$, $R^{n'}$, $R^{\circ}$, $R^{\circ'}$, $R^p$ and $R^{p'}$ is independently selected from among H, $C_{1-7}$ alkyl, $C_{2-7}$ alkenyl, $C_{2-7}$ alkynyl, $C_{3-13}$ cycloalkyl, 3 to 7 membered heterocycloalkyl, $C_{5-10}$ aryl, and 5 to 7 membered heteroaryl.

Further, the $R^m$ is independently selected from a group comprised of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl and 5 to 7 membered heteroaryl, and;

the $R^m$ is additionally substituted with of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl or 5 to 7 membered heteroaryl.

Further,, $R^{x4}$ and $R^{x4'}$ are independently selected from among H, $R^m$, OH, $OR^m$, SH, $SR^m$, $NH_2$, $NHR^m$, $NR^mR^{m'}$, $NO_2$, Me3Sn, halo, $C_{1-6}$ alkyl $C_{1-6}$ alkoxy, $C_{2-6}$ alkenyl, $C_{2-6}$ alkynyl, $C_{3-6}$ cycloalkyl, 3 to 7 membered heterocycloalkyl, $C_{5-12}$ aryl, 5 to 7 membered heteroaryl, -CN, -NCO, $-OR^n$, $-OC(O)R^n$, $-OC(O)NR^nR^{n'}$, $-OS(O)R^n$, $-OS(O)_2R^n$, $-SR^n$, $-S(O)R^n$, $-S(O)_2R^n$, $-S(O)NR^nR^{n'}$, $-S(O)_2NR^nR^{n'}$, $-OS(O)NR^nR^{n'}$, $-OS(O)_2NR^nR^{n'}$, $-NR^nR^{n'}$, $-NR^nC(O)R^{\circ}$, $-NR^nC(O)OR^{\circ}$, - $NR^nC(O)NR^{\circ}R^{\circ'}$, $-NR^nS(O)R^{\circ}$, $-NR^nS(O)_2R^{\circ}$, $-NR^nS(O)NR^{\circ}R^{\circ'}$,$-NR^nS(O)_2NR^{\circ}R^{\circ'}$, $-C(O)R^n$, - $C(O)OR^n$ and $-C(O)NR^n$-$R^{n'}$, and;

The $R^{X4}$ or $R^{X4'}$ is $C_{1-6}$ alkyl, $C_{1-6}$ alkoxy, $C_{2-6}$ alkenyl, $C_{2-6}$ alkynyl, $C_{3-6}$ cycloalkyl, 3 to 7 membered heterocycloalkyl, $C_{5-12}$ aryl or 5 to 7 membered heteroaryl, and additionally is substituted with at least one $C_{1-6}$ alkyl, $C_{1-6}$ alkoxy, $C_{2-6}$ alkenyl, $C_{2-6}$ alkynyl, $C_{3-6}$ cycloalkyl, 3 to 7 membered heterocycloalkyl, $C_{5-10}$ aryl, 5 to 7 membered heteroaryl, - $OR^p$, $-OC(O)R^p$, $-OC(O)NR^pR^{p'}$, $-OS(O)R^p$, $-OS(O)_2R^p$, $-SR^p$, $-S(O)R^p$, $-S(O)_2R^p$, - $S(O)NR^pR^{p'}$, $-S(O)_2NR^pR^{p'}$, $-OS(O)NR^pR^{p'}$, $-OS(O)_2NR^pR^{p'}$, $-NR^pR^{p'}$, $-NR^pC(O)R^q$, - $NR^pC(O)OR^q$, $-NR^pC(O)NR^qR^{q'}$, $-NR^pS(O)R^q$, $-NR^pS(O)_2R^q$, $-NR^pS(O)NR^qR^{q'}$, - $NR^pS(O)_2NR^qR^{q'}$, $-C(O)R^p$, $-C(O)OR^p$ or $-C(O)NR^pR^p$.

Further, the $R^{X7}$ and $R^{X7'}$ are independently selected from among H, $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, 5 to 7 membered heteroaryl, $-OR^r$, $-OC(O)R^r$, $-OC(O)NR^rR^{r'}$, $-OS(O)R^r$, $-OS(O)_2R^r$, $-SR^r$, $-S(O)R^r$, $-S(O)_2R^r$, - $S(O)NR^rR^{r'}$, $-S(O)_2NR^rR^{r'}$, $-OS(O)NR^rR^{r'}$, $-OS(O)_2NR^rR^{r'}$, $-NR^rR^{r'}$, $-NR^rC(O)R^s$, $-NR^rC(O)OR^s$, - $NR^rC(O)NR^sR^{s'}$, $-NR^rS(O)R^s$, $-NR^rS(O)_2R^s$, $-NR^rS(O)NR^sR^{s'}$, $-NR^rS(O)_2NR^sR^s$, $-C(O)R^r$, - $C(O)OR^s$ or $-C(O)NR^rR^{r'}$;

$R^{X7}$ and $R^{X7'}$ are independently selected from among $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, or 5 to 7 membered heteroaryl, and are additionally substituted by $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, 5 to 7 membered heteroaryl, $-OR^t$, $-OC(O)R^t$, $-OC(O)NR^tR^{t'}$, $-OS(O)R^t$, $-OS(O)_2R^r$, $-SR^t$, $-S(O)R^t$, $-S(O)_2R^t$, $-S(O)NR^tR^{t'}$, $-S(O)_2NR^tR^{t'}$, $-OS(O)NR^tR^{t'}$, $-OS(O)_2NR^tR^{t'}$, $-NR^tR^{t'}$, $-NR^tC(O)R^u$, $-NR^tC(O)OR^u$,$-NR^tC(O)NR^uR^{u'}$, - $NR^tS(O)R^u$, $-NR^tS(O)_2R^u$, $-NR^tS(O)NR^uR^{u'}$, $-NR^tS(O)_2NR^uR^{u'}$, $-C(O)R^t$, $-C(O)OR^t$ or - $C(O)NR^tR^{t'}$, and;

The $R^r$, $R^{r'}$, $R^s$, $R^{s'}$, $R^t$, $R^{t'}$, $R^u$ and $R^{u'}$ are independently selected from among H, $C_{1-7}$ alkyl, $C_{2-7}$ alkenyl, $C_{2-7}$ alkynyl, $C_{3-13}$ cycloalkyl, 3 to 7 membered heterocycloalkyl, $C_{5-10}$ aryl, and 5 to 7 membered heteroaryl.

Further, $R^{X1}$ and $R^{X1'}$ are independently selected from $R^m$, and;

$R^m$ is selected from among $C_{1-6}$ alkyl, $C_{2-6}$ alkenyl, $C_{5-7}$ aryl and $C_{3-6}$ heteroaryl. Further, $R^{X2}$, $R^{X2'}$, $R^{X3}$, $R^{X3'}$, $R^{X5}$ and $R^{X5'}$ are independently selected from H or OH. Further, $R^{X4}$ and $R^{X4'}$ are independently selected from $R^m$, and; $R^m$ is $C_{1-6}$ alkoxy.

Further, the $R^{X4}$ and $R^{X4'}$ are independently any one selected from a group comprised of methoxy, ethoxy and butoxy .

Further, the Y and Y' are O.

Further,, the $R^{x6}$ is $C_{3-12}$ alkylene, $C_{3-12}$ alkenylene or $C_{3-12}$ heteroalkylene, with the $R^{x6}$ substituted by -NH2, $-NHR^m$, $-NHC(O)R^m$, $-NHC(O)R^m$, $-NHC(O)CH_2-[OCH_2CH_2]_n-R^{xx}$, or - $[CH_2CH_2O]_n-R^{XX}$;

The $R^{xx}$ is H, OH, $N_3$, CN, $NO_2$, SH, $NH_2$, $ONH_2$, $NHNH_2$, halo, $C_{1-8}$ alkyl, $C_{3-8}$ cycloalkyl, $C_{1-8}$ alkoxy, $C_{1-8}$ alkylthio, $C_{3-20}$ heteroaryl, $C_{5-20}$ aryl or mono- or di-$C_{1-8}$ alkyl amino, and; n is an integer of 1 through 6.

Further, in one aspect of the present invention, the active agent is a pyrrolobenzodiazepine dimer denoted by General Formula XII or General Formula XIII;

[General Formula XII]

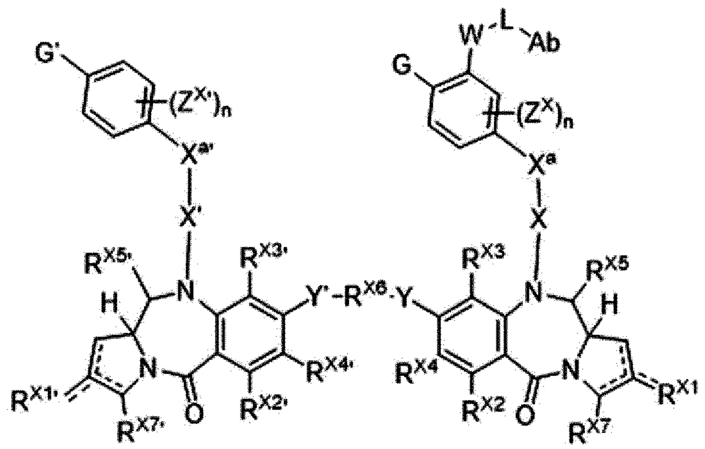

[General Formula XIII]

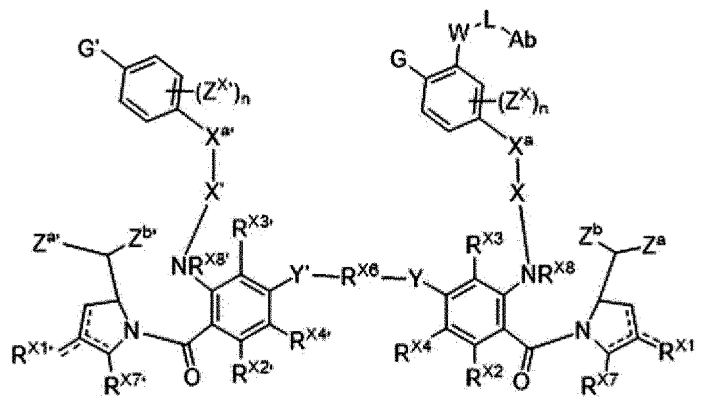

The $X^a$ and $X^{a'}$ are independently selected from between a bond or $C_{1-6}$ alkylene;

$Z^{X'}$ and $Z^x$ are independently selected from among hydrogen, $C^{1-8}$ alkyl, halogen, cyano, nitro,

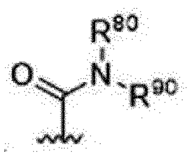
,
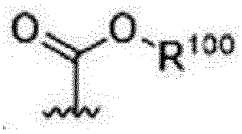

or $-(CH_2)_m-OCH_3$;

The respective $R^{80,}$ $R^{90}$ and $R^{100}$ are selected from among hydrogen, $C_{1-8}$ alkyl, $C_{2-6}$ alkenyl and $C_{1-6}$ alkoxy, and; m is an integer of 0 to 12.

The $Z^{X'}$ and $Z^X$ are independently any one selected from a group comprised of hydrogen,

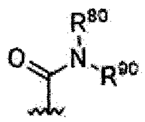 , 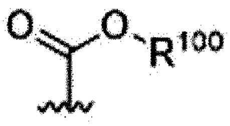

and $-(CH_2)_m-OCH_3$;

The respective $R^{80}$, $R^{90}$ and $R^{100}$ are selected from among hydrogen, $C_{1-3}$ alkyl and $C_{1-3}$ alkoxy; m is an integer of 1 to 6 and; the active agent is any one selected from a group comprised of:

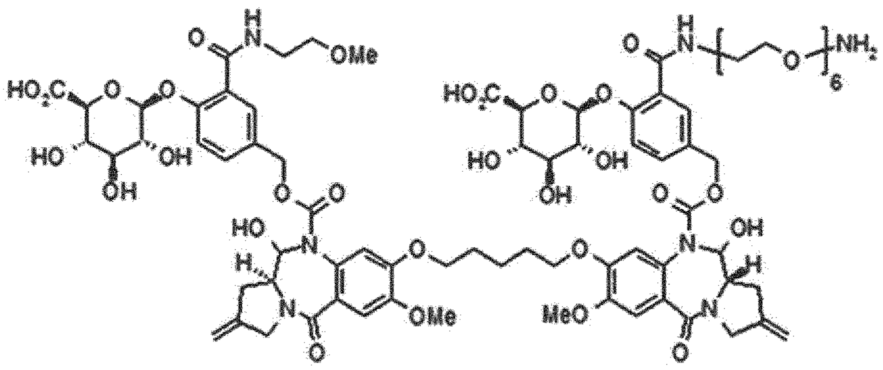

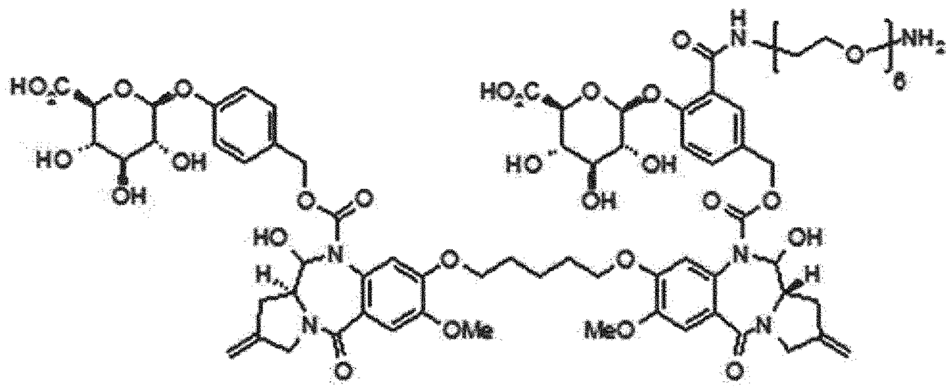

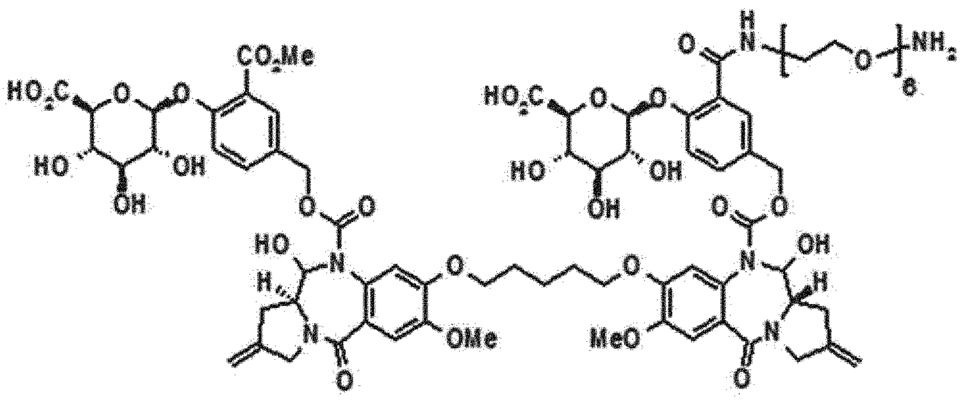

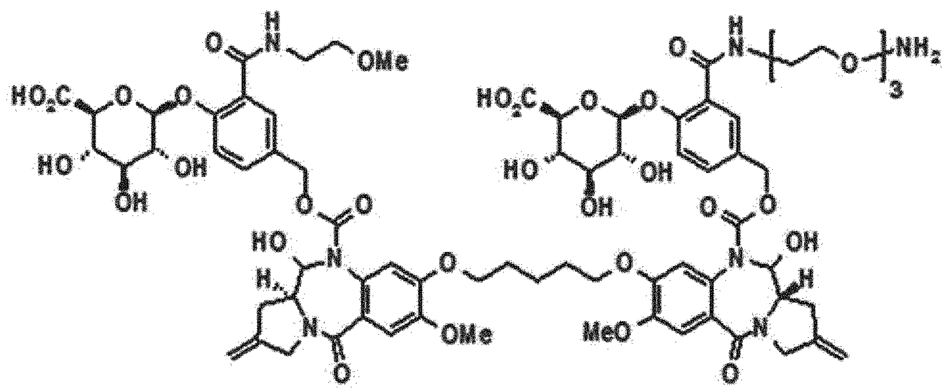

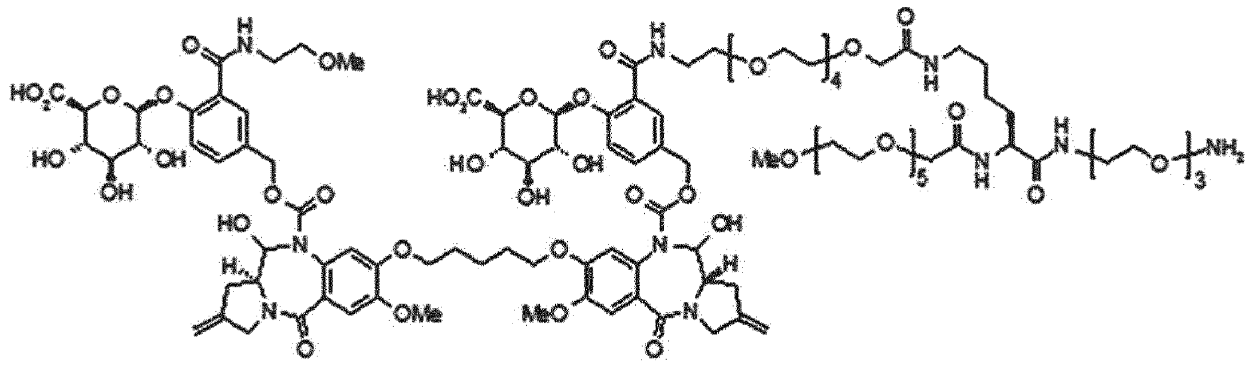

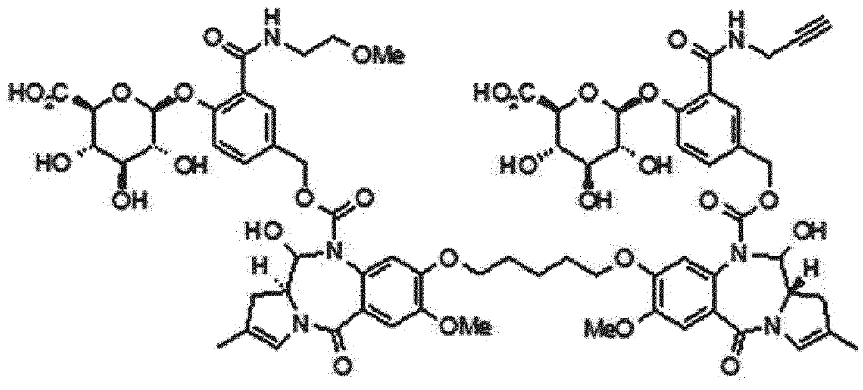

,

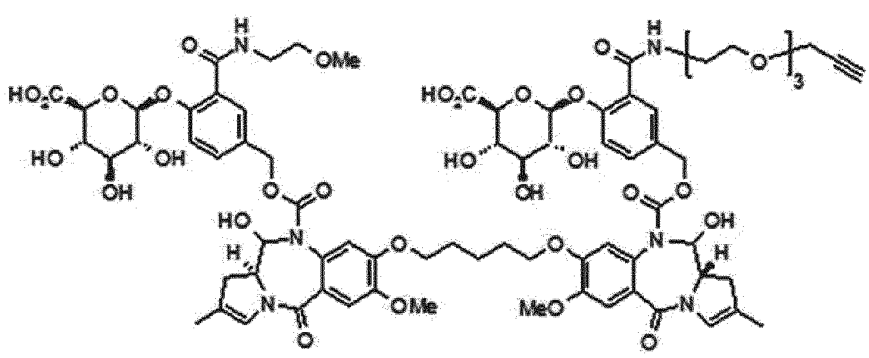

,

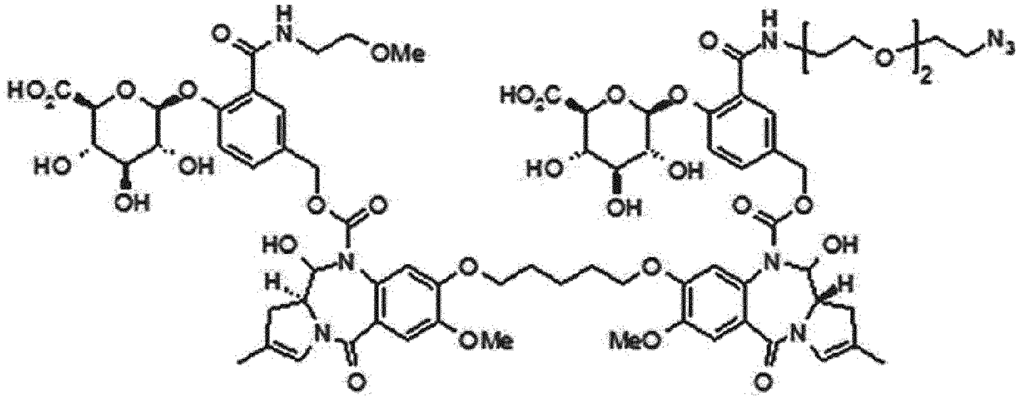

,

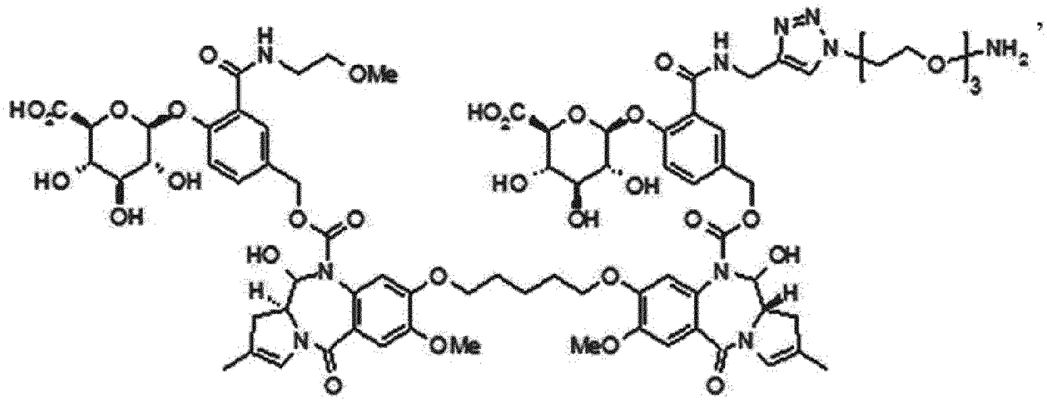

,

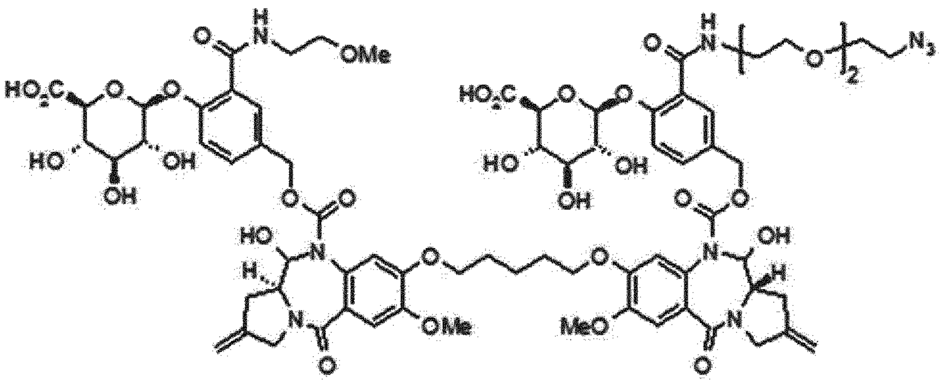

,

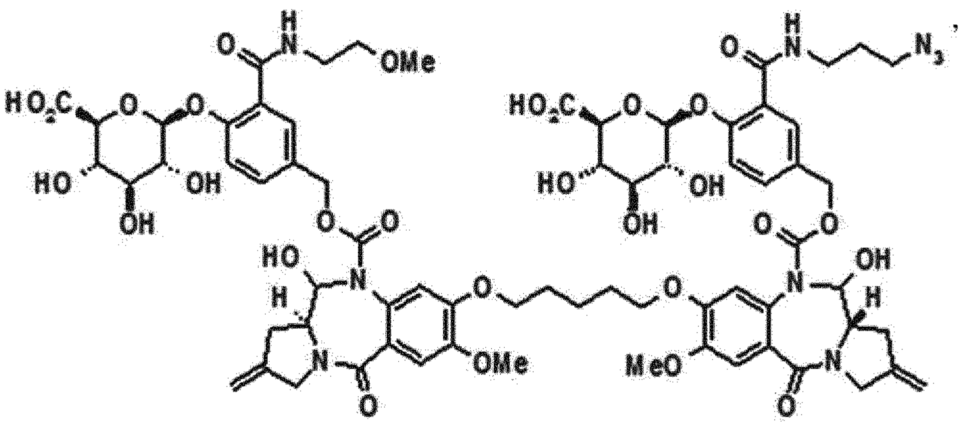

,

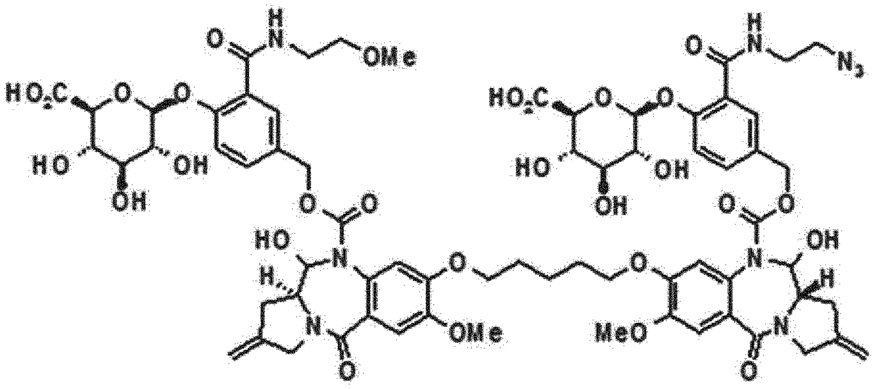
,

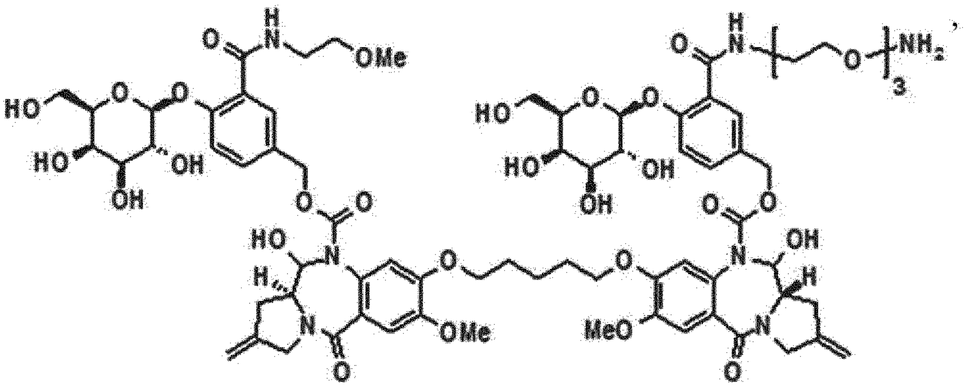
,

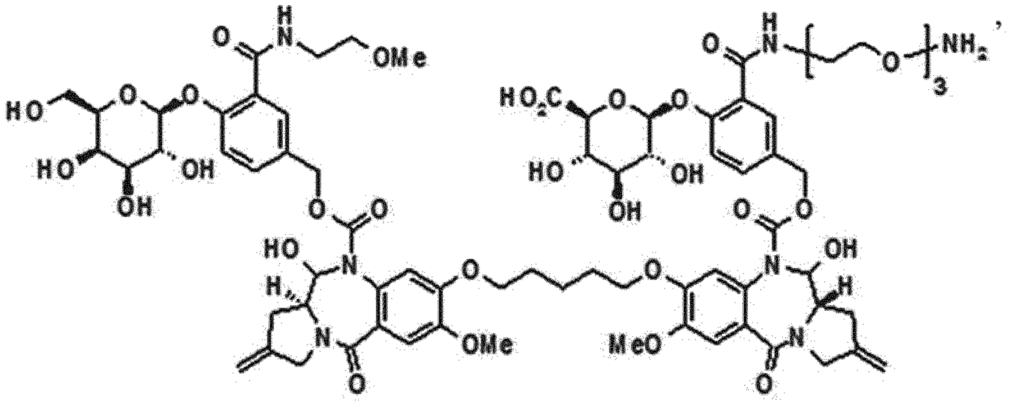
,

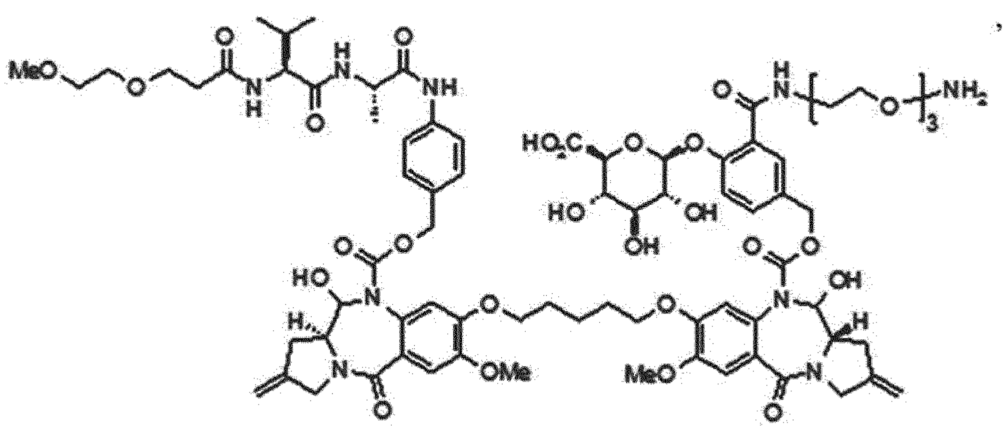
,

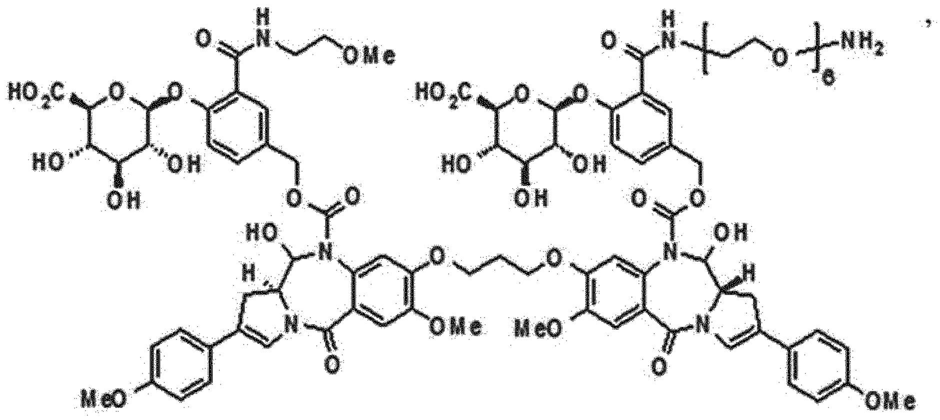
,

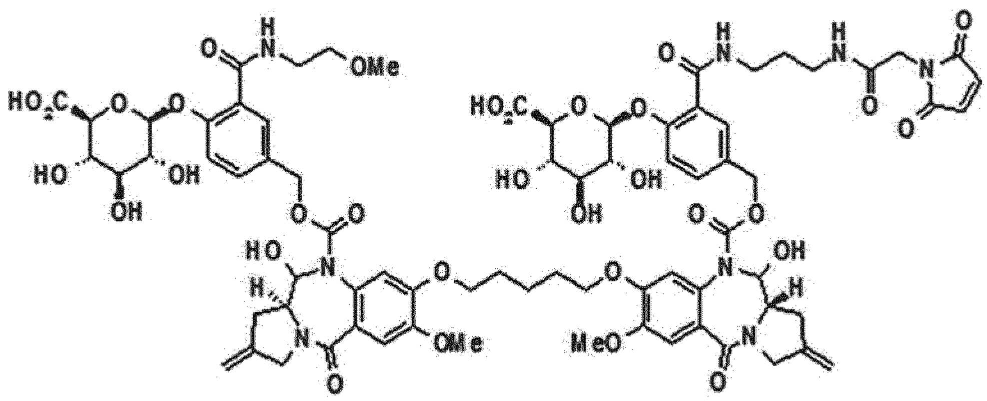
,

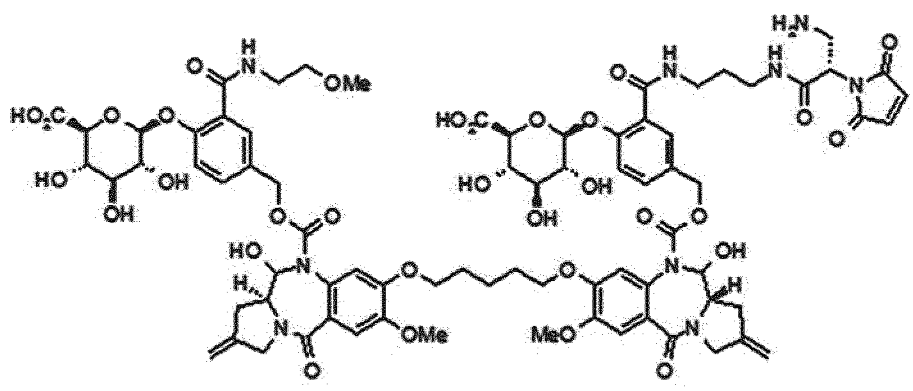
,

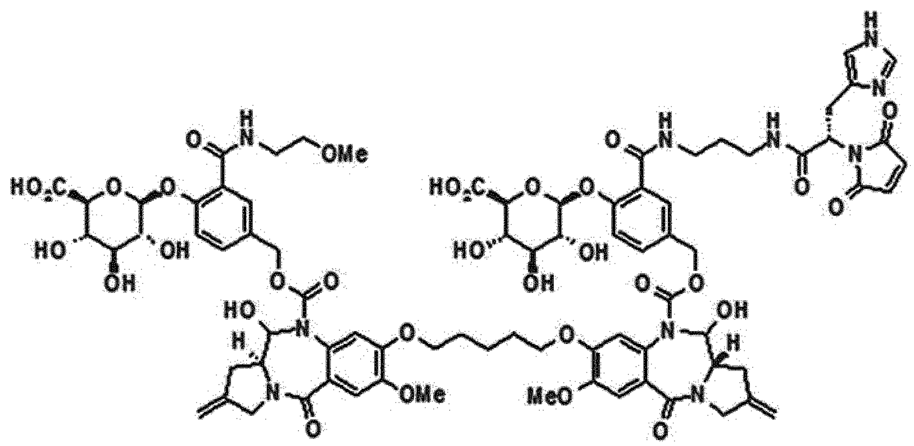
,

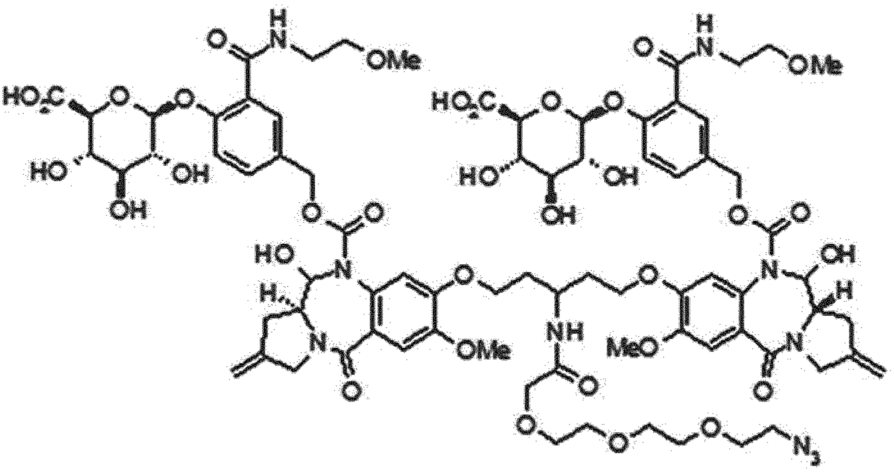

,

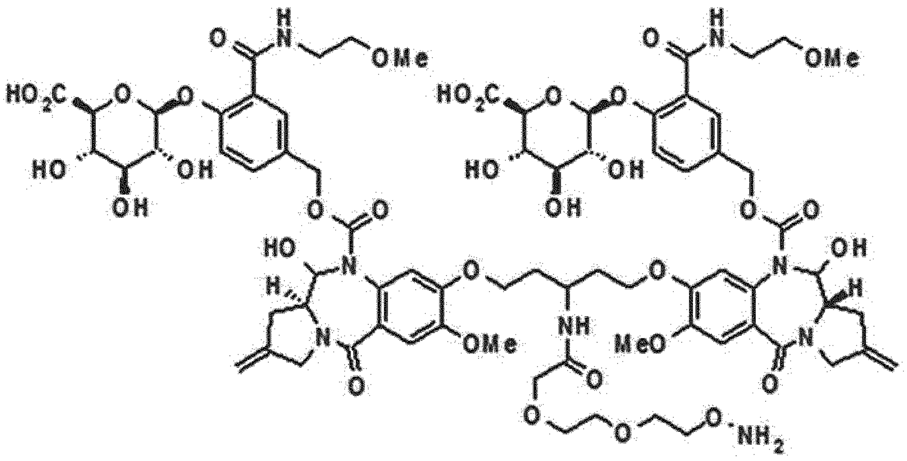

,

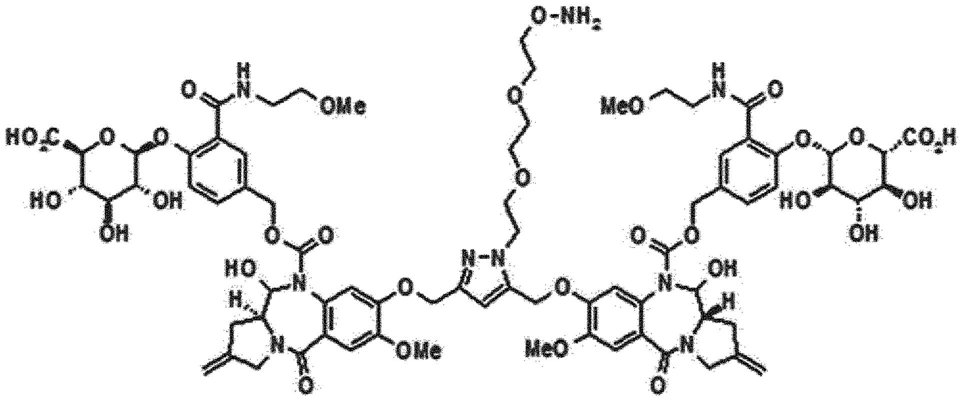

,

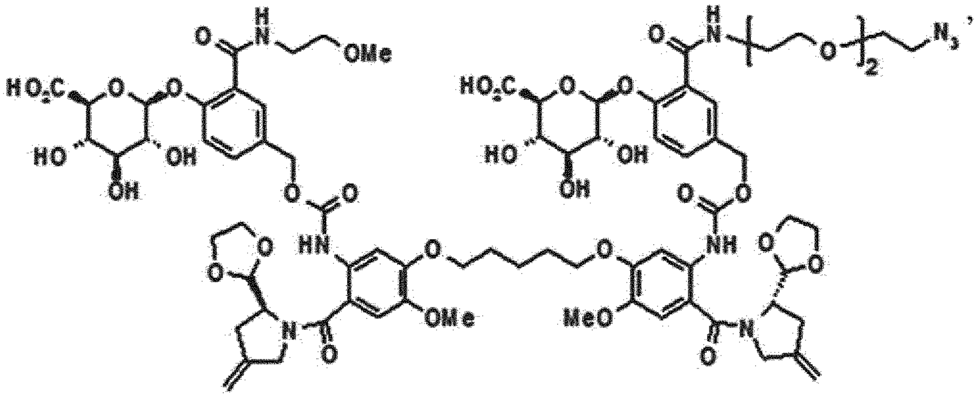

,

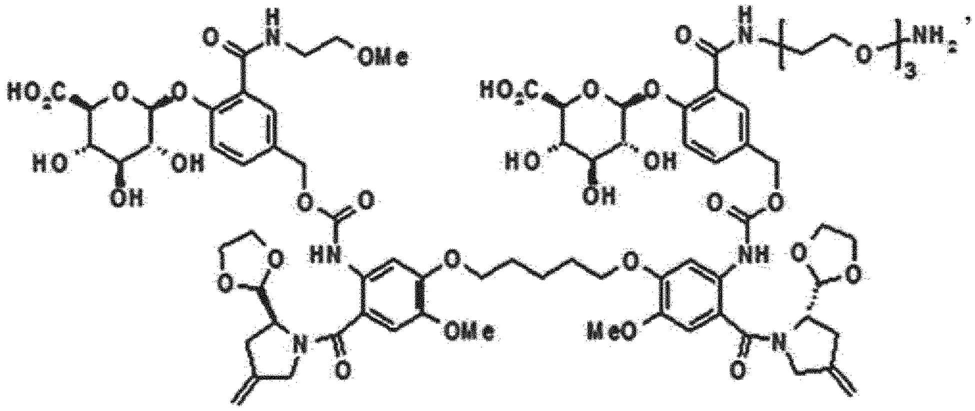

,

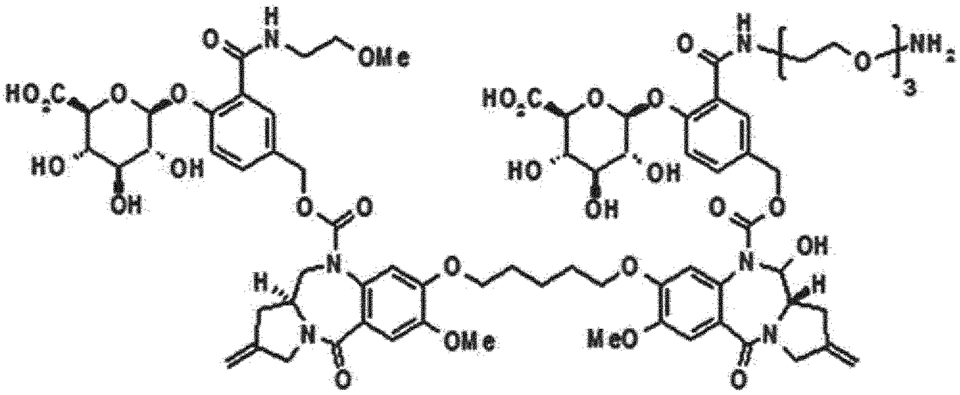

,

and;

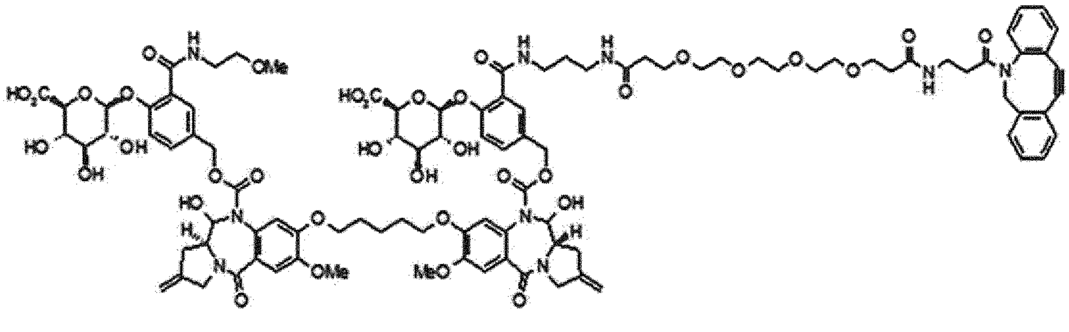

In the present specification, "conjugates" refers to cell binding agents that are covalently linked to one or more molecules of a cytotoxic compound. Here, " cell binding agent" is a molecule having affinity for a biological target, for

example, a ligand, protein, antibody, specifically a monoclonal antibody, protein or antibody fragment, and the binding agent serves to direct the biologically active compound to the biological target. In one aspect of the present invention, the conjugate may be designed to target tumor cells through cell surface antigens. The antigen may be a cell surface antigen that is overexpressed or expressed in an abnormal cell type. Specifically, the target antigen may be expressed only on proliferative cells (e.g., tumor cells). Target antigens may be selected based on different expression, usually between proliferative and normal tissues. In the present invention, a ligand is bonded to a linker.

In the present specification, "antibody" refers to an immunoglobulin molecule which, through at least 1 antigen recognizing site located in the variable region of the immunoglobulin molecule, can bind with specificity to a target, for example a carbohydrate, polynucleotide, lipid or polypeptide, etc. The term "antibody" as used in the present specification encompasses not only complete monoclonal or polyclonal antibody, but also a given antigen-binding part (for example, "antigen-binding fragment") of a complete antibody having the capacity to bind with specificity to a certain antigen) or a single chain thereof, a fused protein comprising antibody, or any other modified arrangement of immunoglobulin molecule comprising an antigen recognizing site, non-limiting examples of which are: Fab; Fab'; $F(ab')_2Fd$ fragment; Fv fragment; single domain antibody (dAb) fragment; isolated complementarity determining region (CDR); single chain (scFv) and single domain antibody (e.g. shark and camel antibody), a maxibody, a minibody, an intrabody, a diabody, a triabody, a tetrabody, a v-NAR, or a bis-scFv (See e.g. the literature [Hollinger and Hudson, 2005, Nature Biotechnology 23(9): 1126-1136]".

Antibodies include any category of antibody, for example IgG, IgA or IgM (or sub-categories thereof), and the antibody does not need to be of a specific type. Depending on the amino acid sequence of the constant domain of the heavy chain of the antibody, an immunoglobulin may be assigned to different categories. There exist five major types of immunoglobulin: IgA, IgD, IgE, IgG and IgM, of which a number may be additionally categorized into sub-categories (isotypes), for example, IgG1, IgG2, IgG3, IgG4, IgAl and IgA2. The heavy chain (HC) constant domains corresponding to different categories are respectively referred to as, respectively, alpha, delta, epsilon, gamma and mu. The subunit structures and three-dimensional configuration of different categories of immunoglobulin are widely known to the art. The antibody of the present invention may be prepared using techniques widely known to related arts, for example, recombinant techniques, phage display techniques, synthesis techniques, combinations of the foregoing techniques, or other techniques readily known to related arts.

In the present specification, "isolated antibody" refers to an antibody which does not substantially comprise a different antibody having specificity to another antigen, which may substantially not comprise other cellular material and/or chemical substances.

In the present specification, "biological target" refers to an antigen located on the surface of a tumor, cancer cell or extracellular matrix.

In the present specification, "linker" refers to a compound which covalently bonds a cytotoxic compound to a ligand. In one aspect of the present invention, the linker disclosed in PCT/US2016/063564 and PCT/US2016/063595 may be used as the linker.

In the present specification, "therapeutic agent" refers to an agent that exerts cytotoxicity, cell proliferation inhibition and / or immunomodulatory effects on cancer cells or activated immune cells. Examples of therapeutic agents include cytotoxic agents, chemotherapeutic agents, cell proliferation inhibitors, and immunomodulators.

In the present specification, "chemotherapeutic agent" is a chemical compound useful in the treatment of cancer.

In the present specification, "subject" is intended to include human and non-human animals, particularly mammals. Examples of subjects include human subjects, e.g., disorders, such as those described herein, e.g., human patients with cancer or normal subjects. "Non-human animal" is used in all vertebrate animals, such as non-mammals (e.g., Chickens, amphibians, reptiles) and mammals such as non-human primates, livestock and / or animals (e.g., sheep, dogs, cats, cows, pigs, etc.) and rodents (e.g., mice, rats, hamsters, guinea pigs, etc.). In certain embodiments, the subject is a human patient. In certain examples, the subject is a human patient.

In the present specification, "treating" or "treating" refers to both therapeutic treatment and prophylactic or preventative measures. A subject in need of treatment includes a subject already having a disorder, and a subject susceptible to a disorder or a subject to be prevented from disorder. Thus, when used in reference to a subject in need of the disease or treatment, the term encompasses an inhibition or slowing of disease progression, remission of disease, prevention of symptoms, reduction of disease and / or symptom severity, but is not limited to these.

In the present specification, "administer" or "administering" refers to providing and / or contacting and / or delivering a compound or compounds by any suitable route to achieve the desired effect. Administration may be by oral, sublingual, parenteral (e.g. intravenous, subcutaneous, intradermal, intramuscular, intraarticular, intraarterial, intrathecal, intrasternal, intraspinal, Rectal, nasal, buccal, rectal, vaginal, nasal, ophthalmic, inhaled and implanted.

In the present specification, "unsubstituted or substituted" refers to a parent group which may be unsubstituted or substituted, "substituted" refers to a parent group having at least 1 substituent group, and substituent group refers to a chemical part which is covalently bonded to a parent group or fused to a parent group.

In the present specification, "halo" refers to fluorine, chlorine, bromine or iodine, etc.

In the present specification, "alkyl" is a monovalent moiety obtained by removing a hydrogen atom from a carbon atom of an aliphatic or alicyclic, saturated or unsaturated (unsaturated, fully unsaturated) hydrocarbon compound. Examples of saturated alkyls include methyl, ethyl, Butyl, n-pentyl (amyl), n-hexyl, n-heptyl and the like, saturated cyclic alkyl groups such as methyl, ethyl, Isopropyl, isobutyl, sec-butyl, tert-butyl, isopentyl and neopentyl, etc.

In the present invention, "alkoxy" refers to -OR where R is an alkyl group, and examples include methoxy, ethoxy, n-propoxy, isopropoxy, n-butoxy, sec-butoxy, isobutoxy and tert-butoxy, etc.

In the present invention, "aryl" refers to a monovalent moiety obtained by removing a hydrogen atom from an aromatic ring atom of an aromatic compound having a ring atom.

In the present invention, "alkenyl" is an alkyl having at least one carbon-carbon double bond. Examples of unsaturated alkenyl groups are ethenyl (vinyl, $-CH=CH_2$), 1-propenyl ($-CH=CH-CH_3$), 2-propenyl, isopropenyl, butenyl, pentenyl and hexenyl, etc.

In the present invention, "alkynyl" is an alkyl group having at least one carbon-carbon triple bond, and examples of unsaturated alkynyl group include ethynyl and 2-propynyl, etc.

In the present invention, "carboxy" refers to -C(=O)OH.

In the present invention, "formyl" refers to -C(=O)H.

In the present invention, "aryl" refers to a monovalent moiety obtained by removing a hydrogen atom from an aromatic ring atom of an aromatic compound. For example, "$C_{5-7}$ aryl" is a moiety having from 5 to 7 ring atoms, which is a monovalent moiety obtained by removing a hydrogen atom from the aromatic ring atoms of an aromatic compound, and "$C_{5-10}$ aryl" is a moiety having from 5 to 10 ring atoms, which is a monovalent moiety obtained by removing a hydrogen atom from the aromatic ring atoms of an aromatic compound. Here, the prefixes ($C_{5-7}$ , $C_{5-10}$, etc.) refer to the number of ring atoms or a range for the number of ring atoms, regardless of whether they are carbon atoms or hetero atoms. For example, "$C_{5-6}$ aryl" relates to an aryl group having 5 or 6 ring atoms. Here, the ring atoms may be all carbon atoms as in a "carboaryl group". Examples of a carboaryl group include, but are not limited to, those derived from benzene, naphthalene, azulene, anthracene, phenanthrene, naphthacene and pyrene. Examples of aryl groups that include a fused ring wherein at least one is an aromatic ring include, but are not limited to, groups derived from indane, indene, isoindene, tetralin, acenaphthene, fluorene, phenalene, acesphenanthrene and aseantrene. Alternatively, the ring atoms may contain one or more heteroatoms as in a "heteroaryl group".

In the present invention, " heteroaryl " refers to aryl containing one or more heteroatoms, such as pyridine, pyrimidine, benzothiophene, furyl, dioxolanyl, pyrrolyl, oxazolyl, pyridyl, pyridazinyl, More specifically benzofuran, isobenzofuran, indole, isoindole, indolizine, indolin, isoindoline, purine (adenine or guanine), benzimidazole, indazole, benzoxazole, benzisoxazole, benzodioxole, benzofuran, benzotriazole, benzothiofuran, benzothiazole, $C_9$ having two fused rings derived from benzothiazole, chromene, iso-chromene, chroman, is-chroman, benzodioxane, quinoline, isoquinoline, quinolizine, benzoxazine, benzodiazine, pyridopyridine, quinoxaline, quinazoline, cinnoline, phthalazine, naphthyridine, $C_{10}$ having two fused rings derived from pteridin, $C_{11}$ having two fused rings derived from benzodiazepine, carbazole, dibenzofuran, dibenzothiophene, carboline, pyrimidine, $C_{13}$ having three fused rings derived from pyridoindole, acridine, xanthene, thioxanthene, phenoxathiine, phenazine, phenoxazine, phenothiazine, thianthrene, phenanthridine, phenanthroline, and $C_{14}$ having three fused rings derived from phenazine.

In the present invention, "cycloalkyl" refers to an alkyl group which is a cycloalkyl group, and relates to a monovalent moiety obtained by removing a hydrogen atom from an alicyclic ring atom of a cyclic hydrocarbon compound. Examples of cycloalkyl groups include, but are not limited to, those derived from:

Saturated single ring hydrocarbon compounds: cyclopropane, cyclobutane, cyclopentane, cyclohexane, cycloheptane, methylcyclopropane, dimethylcyclopropane, methylcyclobutane, dimethylcyclobutane, methylcyclopentane, dimethylcyclopentane and methylcyclohexane;
Unsaturated single ring hydrocarbon compounds: cyclopropene, cyclobutene, cyclopentene, cyclohexene, methylcyclopropene, dimethylcyclopropene, methylcyclobutene, dimethylcyclobutene, methylcyclopentene, dimethylcyclopentene and methylcyclohexene;
and saturated heterocyclic hydrocarbon compounds: norcaran, norphenen, and norbornene.

In the present invention, "heterocyclyl" refers to a monovalent moiety obtained by removing a hydrogen atom from a ring atom of a heterocyclic compound.

In the present invention, the prefixes (e.g., $C_{1-12}$ , $C_{3-8}$, etc.) refer to the number of ring atoms or a range for the number of ring atoms, regardless of whether they are carbon atoms or hetero atoms. For example, the term "$C_{3-6}$ heterocyclyl" used in the present specification relates to a heterocyclyl group having 3 to 6 ring atoms.

Examples of single ring heterocyclyl groups include, but are not limited to, those derived from:

$N_1$: aziridine, azetidine, pyrrolidine, pyrroline, 2H- or 3H-pyrrole, piperidine, dihydropyridine, tetrahydropyridine, azepine;

$N_2$: imidazolidine, pyrazolidine, imidazoline, pyrazoline, piperazine;
$O_1$: oxirane, oxetane, oxolane, oxol, oxane, dihydropyran, pyran, oxepine;
$O_2$: dioxolane, dioxane and dioxepane;
$O_3$: trioxane;
$N_1O_1$: tetrahydrooxazole, dihydrooxazole, tetrahydroisoxazole, dihydroisoxazole, morpholine, tetrahydrooxazine, dihydrooxazine,
$S_1$: thiiran, thiethan, thiolan, thian, tiepan;
$N_1S_1$: thiazoline, thiazolidine, thiomorpholine;
$N_2O_1$: oxadiazine;
$O_1S_1$: oxathiol, oxathian, and;
$N_1O_1S_1$: oxathiazine.

In the present invention, "prodrug" refers to compounds which, under in vivo physiological conditions (e.g. enzymatic oxidation, reduction and/or hydrolysis), may, by action of enzyme or gastric acid, be converted directly or indirectly into a pyrrolobenzodiazepine drug.

In the present invention, "pharmaceutically acceptable salt" may be an acid addition salt formed by pharmaceutically acceptable free acid, where the free acid is an organic acid or an inorganic acid.

The organic acids include, but are not limited to, citric acid, acetic acid, lactic acid, tartaric acid, maleic acid, fumaric acid, formic acid, propionic acid, oxalic acid, trifluoroacetic acid, benzoic acid, gluconic acid, methane sulfonic acid, glycolic acid, succinic acid, 4-toluenesulfonic acid, glutamic acid and aspartic acid. Also, the inorganic acids include, but are not limited to, hydrochloric acid, bromic acid, sulfuric acid and phosphoric acid.

For example, if the compound has a functional group which is an anion or may be an inion (e.g. -COOH may be -COO-), a suitable cation may be used for forming a salt. Examples of suitable inorganic cations include, but are not limited to, $Na^+$ and $K^{+,}$ alkaline earth cations such as $Ca^{2+}$ and $Mg^{2+,}$ and other cations such as $Al^{3+,}$ Examples of suitable organic cations include, but are not limited to, ammonium ion (i.e., $NH_4^{+)}$ and substituted ammonium ions (e.g. $NH_3R^+$, $NH_2R_2^+$, $NHR_3^+$, $NR4^+$).

Some examples of suitable substituted ammonium ions are derived from the following: ethylamine, diethylamine, dicyclohexylamine, triethylamine, butylamine, ethylenediamine, ethanolamine, diethanolamine, piperazine, benzylamine, phenylbenzylamine, choline, meglumine and tromethamine, as well as amino acids such as lysine and arginine. An example of a typical quaternary ammonium ion is $N(CH_3)_4^+$.

If the compound has a functional group which may be a cation or a cation (e.g., $-NH_2$ may be $-NH_3^+$), a salt may be formed with a suitable anion. Examples of suitable inorganic anions include, but are not limited to, those derived from the following inorganic acids: hydrochloric acid, hydrobromic acid, hydroiodic acid, sulfuric acid, sulfuric acid, nitric acid, nitrous acid, phosphoric acid and phosphorous acid.

Examples of suitable organic anions include, but are not limited to, those derived from organic acids such as: 2-acetoxybenzoic acid, acetic acid, ascorbic acid, aspartic acid, benzoic acid, camphorsulfonic acid, cinnamic acid, citric acid, But are not limited to, disulfonic acid, ethanesulfonic acid, fumaric acid, glutaronic acid, gluconic acid, glutamic acid, glycolic acid, hydroxymaleic acid, hydroxynaphthalenecarboxylic acid, isethionic acid, lactic acid, But are not limited to, malic acid, methanesulfonic acid, mucilous acid, oleic acid, oxalic acid, palmitic acid, pamic acid, pantothenic acid, phenylacetic acid, phenylsulfonic acid, propionic acid, pyruvic acid, salicylic acid, stearic acid, succinic acid, sulfanilic acid and tartaric acid, etc. Examples of suitable multimeric organic anions include, but are not limited to, those derived from the following multimeric acids: tannic acid, carboxymethylcellulose, etc.

In the present invention, "solvate" refers to a molecular complex between a compound according to the present invention and solvent molecules, examples of which include but are not limited to water, isopropanol, ethanol, methanol, dimethylsulfoxide, ethyl acetate, acetic acid, ethanolamine or the compound according to the present invention bonded to a mixed solvent thereof.

It may be convenient or desirable to prepare, purify and/or handle solvates corresponding to active compounds. In the present specification, the term "solvate" is used in its conventional sense to refer to solutes (e.g. active compounds and salts of active compounds) and complexes of solvents. When the solvent is water, the solvate may conveniently be referred to as a hydrate, such as monohydrate, dihydrate or trihydrate, etc.

The pharmaceutical composition of the present invention may comprise a pharmaceutically acceptable carrier. Examples of pharmaceutically acceptable carrier include large macromolecules that are slowly metabolized, such as proteins, polysaccharides, polylactic acid, polyglycolic acid, polymeric amino acids, amino acid copolymers, lipid aggregates, and the like. Such pharmaceutically acceptable carriers may be appropriately selected and used by a person skilled in the art.

The composition comprising a pharmaceutically acceptable carrier may be of various oral or parenteral formulations. In the case of formulation, a diluent or excipient such as a filler, an extender, a binder, a wetting agent, a disintegrating agent, or a surfactant is usually used.

Solid formulations for oral administration include tablets, pills, powders, granules, capsules, and the like, which may contain at least one excipient such as starch, calcium carbonate, sucrose or lactose, gelatin, . In addition to simple excipients, lubricants such as magnesium stearate, talc, and the like may also be used.

Liquid preparations for oral administration include suspensions, solutions, emulsions, syrups and the like. Various excipients such as wetting agents, sweeteners, fragrances, preservatives and the like may be included in addition to water and liquid paraffin, which are commonly used simple diluents.

Formulations for parenteral administration include sterilized aqueous solutions, non-aqueous solutions, suspensions, emulsions, freeze-dried preparations, and suppositories. Examples of the non-aqueous solvent and the suspending agent include propylene glycol, polyethylene glycol, vegetable oil such as olive oil, and injectable ester such as ethyl oleate. Examples of the suppository base include witepsol, macrogol, tween 61, cacao paper, laurin, glycerogelatin and the like.

The pharmaceutical composition may be in the form of tablets, pills, powders, granules, capsules, suspensions, solutions, emulsions, syrups, sterilized aqueous solutions, non-aqueous solvents, suspensions, emulsions, freeze- It can have one formulation.

For intravenous, intradermal or hypodermic injection, the active ingredient may be in the form of an acceptable aqueous solution for parenteral administration, be pyrogen-free, and have the appropriate pH, isotonicity and stability. A person skilled in the art may prepare an appropriate solution using an isotonic vehicle such as sodium chloride solution, Ringer's solution or Ringer's lactate solution, and preservatives, stabilizers, buffers, anti-oxidants and other additives may be included as necessary. Solid forms suitable for injection may also be prepared as emulsions or in the form of polypeptide encapsulated in liposome.

As used herein, the phrase "effective amount" or "therapeutically effective amount" refers to the amount required to achieve the desired therapeutic result (for dose and duration and means of administration). An effective amount is at least the minimum amount of active agent required to confer a therapeutic benefit to a subject, and is less than the toxic amount. For example, the amount administered may be in a range of approximately 100ng to approximately 100mg/kg per patient, more typically approximately 1μg/kg to approximately 10mg/kg. In a case where an active compound is a salt, ester, amide or prodrug, the dose is calculated based on the parent compound, and accordingly the actual weight used also increases proportionally. The pyrrolobenzodiazepine compound according to the present invention may be formulated to comprise 0.1mg to 3000mg, 1mg to 2000mg, or 10mg to 1000mg of active ingredient per unit dosage form, but is not limited thereto. The active ingredient may be administered to give an active compound peak plasma concentration of approximately 0.05μM to 100μM, 1μM to 50μM, or 5μM to 30μM. For example, optionally it may be administered by intravenous injection of a solution of 0.1 w/v% to 5 w/v% active ingredient in saline solution.

In a pharmaceutical composition, the concentration of the active compound is determined by the absorption, inactivation and excretion rates of the drug, and other factors known not persons skilled in the art. The dose administered may vary depending on the severity of symptoms or the disease. Further, the dose and administration therapy for a specific patient may be adjusted according to the professional judgment of an administration supervisor, giving general consideration to the degree of the patient's symptoms and illness, need for the drug, age, and reactivity to the drug, etc. The range of concentrations stated in the present invention are only intended as an example, and the manner of carrying out the claimed composition is not limited hereto. Further" the active ingredient may be administered once, or smaller doses may be administered across multiple administrations.

The prodrug compound, or prodrug-linker compound, or prodrug-linker-ligand conjugate compound may be used to treat proliferative diseases, in particular cancer. The term "proliferative disease" refers to excessive, abnormal, undesired or unregulated cell growth either in vitro or in vivo, such as hyperplasia or cytogenesis. Non-limiting examples of proliferative disease include neoplasm, tumor, cancer, leukemia, psoriasis, bone disease, fibrous dysplasia and pharyngeal arteriosclerosis. Non-limiting examples of cancer are lung cancer, small cell lung carcinoma, gastrointestinal cancer, colon cancer, intestinal cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma and melanoma.

**Benefits of the invention**

The antibody-drug conjugate according to the present invention, by comprising an anti-DLK1 antibody which binds with specificity to DLK1-expressing cells and is internalized into a cell exhibits the benefits of being able to deliver a drug effectively, selectively and with specificity, and allowing for a drug to bind stably to an antibody to exhibit the intended cytotoxicity while maintaining in vivo stability. In particular, by grafting technology for a linker including a self-immolative group, the present invention provides a drug-linker-ligand system which allows a drug and/or toxin to stably reach a target cell and effectively exhibit a drug effect while having substantially reduced toxicity.

## BRIEF DESCRIPTION OF THE DRAWINGS

Fig. 1 is a diagram confirming the efficacy of the ADC according to the present invention in a patient-derived tumor animal model.

## BEST MODE(S) FOR CARRYING OUT THE INVENTION

In the following, the present invention is described in further detail through embodiments and experimental examples.

The following embodiments and experimental examples are intended to aid in understanding the present invention, and are not intended to limit the scope of the present invention thereto.

<Example 1> Preparation of compounds 1, 2, 3 and 4

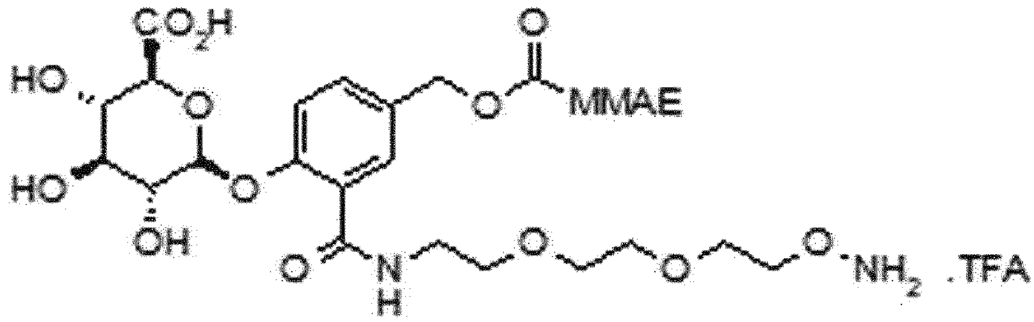

1

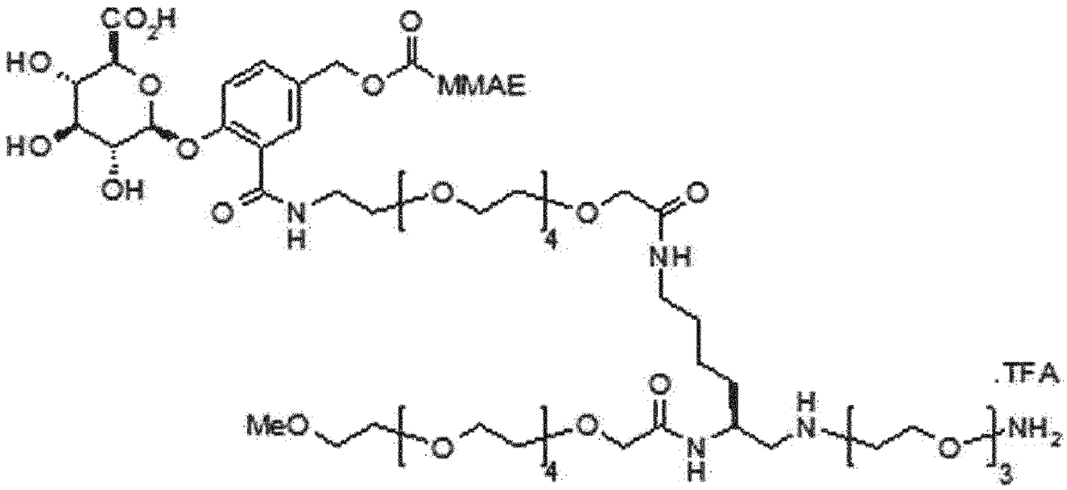

2

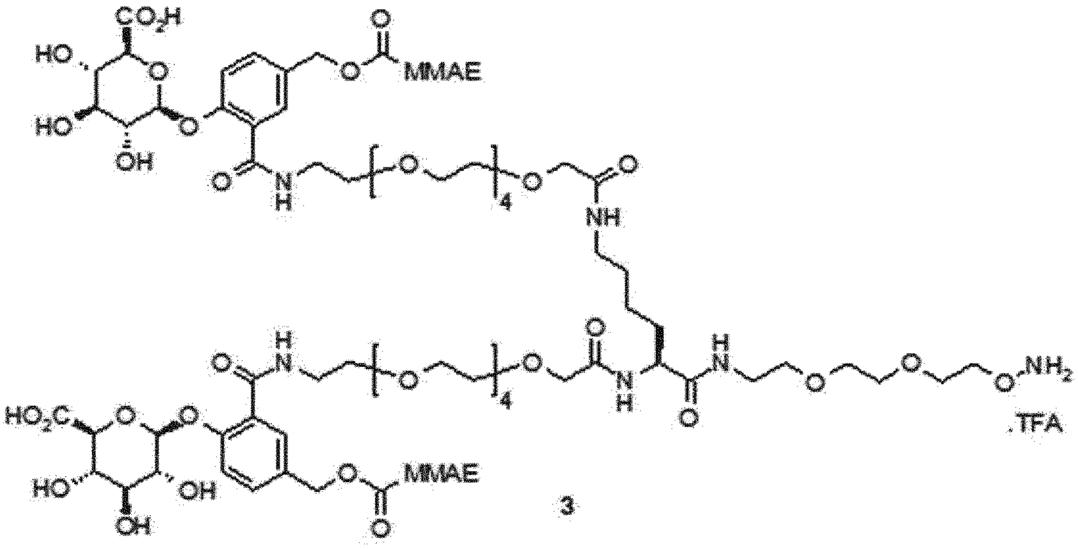

3

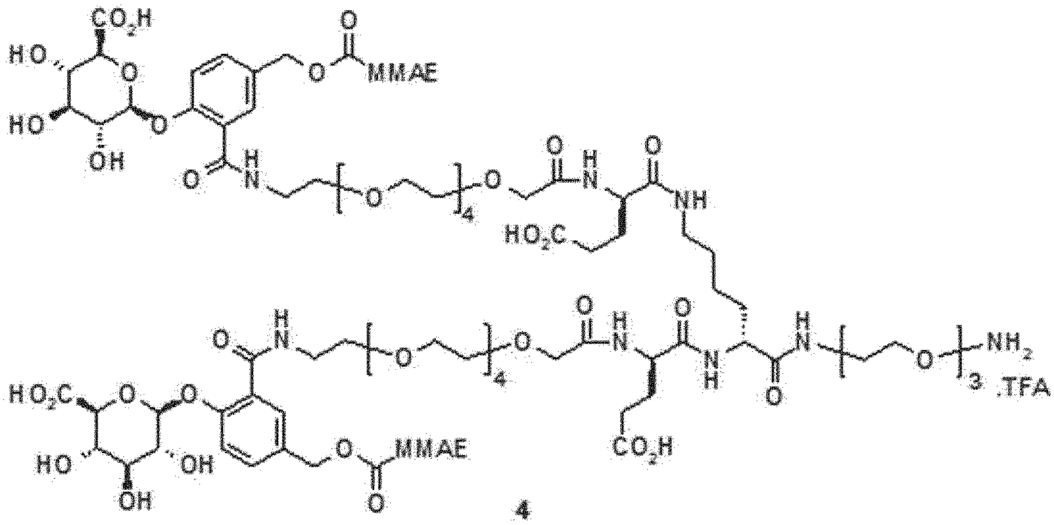

4

Compounds 1, 2, 3 and 4 were prepared using the method stated in International Patent Publication WO2017-089895.

In compounds 1, 2, 3 and 4, the structure of MMAE is as follows.

MMAE

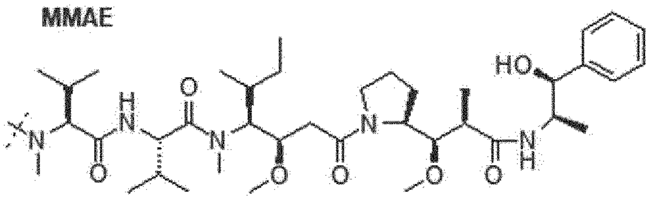

<Example 2> Preparation of compounds 5, 6, 7 and 8

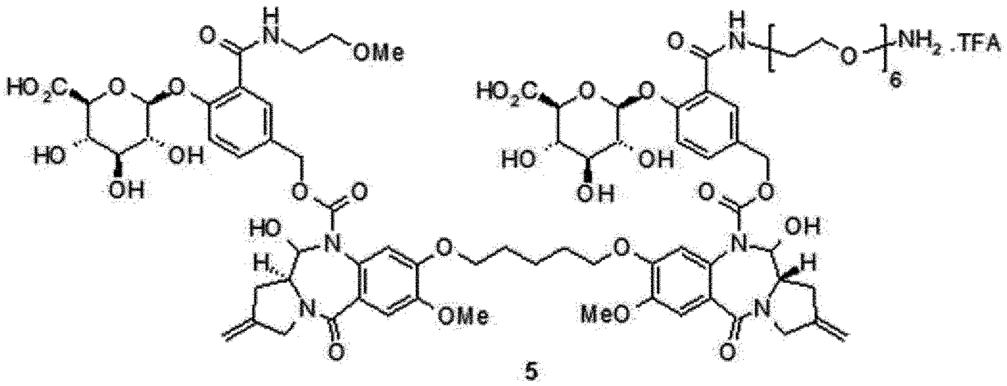

5

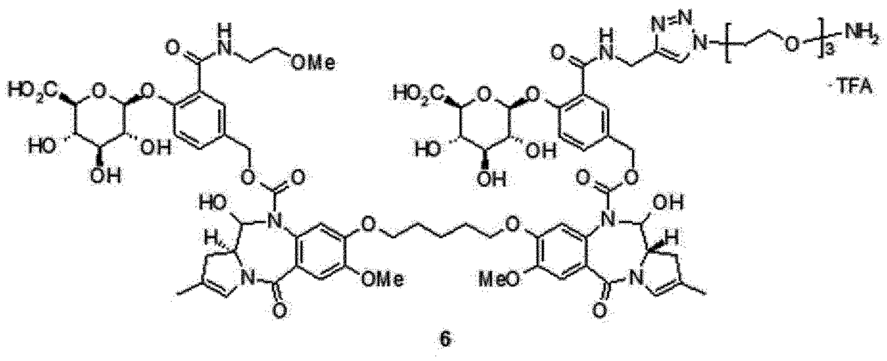

6

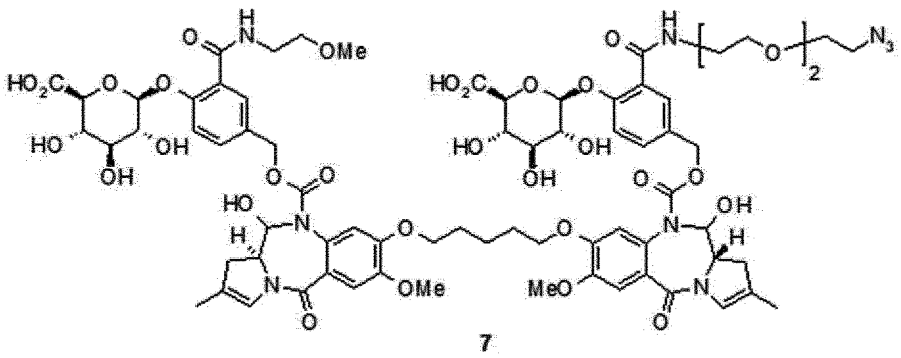

7

Compounds 5, 6 and 7 were prepared using the method stated in Korean Laid-open Patent No. 10-2018-0110645.

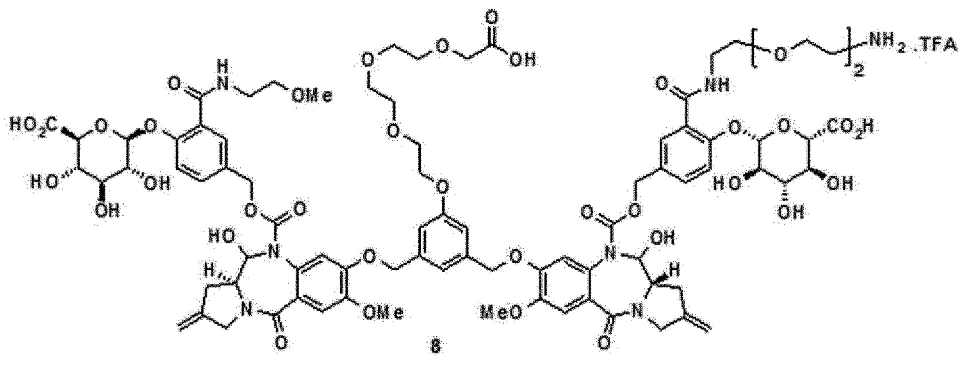

8

Compound 8 was prepared using the method stated in Korean Patent Application No. 10-2019-0000514.

<Example 3> Preparation of ADC

ADC was prepared through the following two steps, and the LCB14-0511 and LCB14-0606 used commonly were prepared using the method stated in Korean Laid-open Patent No. 10-2014-0035393. The structural formulae of LCB14-0511 and LCB14-0606 are as follows:

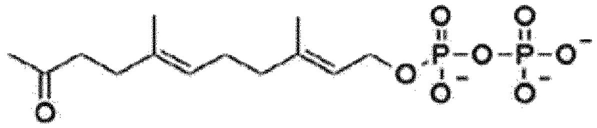

3($NH_4^+$)

**LCB14-0606**

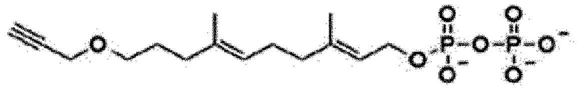

3($NH_4^+$)

**LCB14-0511**

Step 1: Preparation of prenylated antibody

A prenylation reaction mixture of an antibody (18A5) was prepared using the method stated in Korean Patent Application No. 10-2018-0107639 and reacted for 16 hours at 30°C. The reaction mixture was comprised of 24μM antibody (18A5), 200nM FTase (Calbiochem #344145) and a buffer solution (50 mM Tris-HCl (pH 7.4), 5 mM $MgCl_2$, 10 μM $ZnCl_2$, 0.144 mM DTT) containing 0.144 mM LCB14-0511 or LCB14-0606. After the reaction was completed, the prenylated antibody was desalted using a G25 Sepharose column (AKTA purifier, GE healthcare) equilibrated with PBS buffer solution.

Step 2: Method of drug-conjugation

<Conjugation by oxime bond formation>

The mixture for oxime bond-forming reaction between prenylated antibody and linker-drug was prepared by mixing 100mM Na-acetate buffer solution pH 5.2, 10% DMSO, 20μM antibody and 200μM linker-drug (in house, compounds 1, 2, 3, 4, 5, 7 and 8 from Embodiments 1 and 2), and stirred lightly at 30°C. After reacting for 6 or 24 hours, an FLPC (AKTA purifier, GE healthcare) process was carried out to remove the surplus small compounds used. The protein fraction was collected and concentrated.

<Conjugation by click reaction>

The mixture for oxime bond-forming reaction between prenylated antibody and linker-drug was 10% DMSO, 20μM antibody and 200μM linker-drug (in house, compound 6 from Embodiment 2), 1mM copper (II) sulfate pentahydrate, 2mM $(BimC_4A)_3$ (Sigma-Aldrich 696854), 10mM sodium ascorbate and 10mM aminoguanidine hydrochloride, reacted for 3 hours at 25°C, then treated with 2.0mM EDTA and reacted for 30 minutes. After reacting, an FLPC (AKTA purifier, GE healthcare) process was carried out to remove the surplus small compounds used. The protein fraction was collected and concentrated.

[Table 2] List of ADCs prepared

| ADCs | Antibody | Linker-toxin |
|---|---|---|
| ADC1 | 18A5 | Compound 1 |
| ADC2 | | Compound 2 |
| ADC3 | | Compound 3 |
| ADC4 | | Compound 4 |
| ADC5 | | Compound 5 |
| ADC6 | | Compound 6 |
| ADC7 | | Compound 7 |
| ADC8 | | Compound 8 |

Experimental Example 1: In vitro cytotoxicity assessment

The cancer cell line cell proliferation inhibition activity of the drugs stated in Table 3 below and the ADCs prepared in <Embodiment 3> above was measured. For this purpose, commercially available human pancreatic cancer cell line MIA-PaCa2 (DLK1 negative or normal) and MIA-PaCa-2-DLKl over-expressing cell line were used. As the drug, MMAF-OMe was used as the ADC, and the ADCs of Table 2 were used. In a 96-well plate, each well was seeded with 4,000 to 5,000 of the respective cancer cell lines for the 72-hour treatment group, 2,000 to 3,000 cells for the 96-hour treatment group, and 800 to 1,000 cells for the 168-hour treatment group. After culturing for 24 hours, they were treated with the drug and ADC at a concentration of 0.0015 to 10.0 nM (serially diluted threefold). 72, 96 and 168 hours later, respectively, the number of live cells was measured using SRB (Sulforhodamine B)dye.

[Table 3]

| Test samples | IC50 (nM) | | | | | |
|---|---|---|---|---|---|---|
| | MA-PaCa2-DLK1 negative | | | MA-PaCa2-DLK1 positive | | |
| | 72hr | 96h | 168hr | 72hr | 96h | 168hr |
| MMAF-OMe | 0.41 | 0.12 | 0.12 | 0.30 | 0.13 | 0.06 |
| ADC1 | >10 | N.T | >10 | 0.54 | N.T | 0,09 |
| ADC2 | >10 | >10 | >10 | 0.57 | 0.26 | 0.09 |
| ADC3 | >10 | >10 | >10 | 0.19 | 0.11 | 0.03 |
| ADC4 | N.T | >10 | N.T | N.T | 0.05 | N.T |
| ADC5 | >10 | N.T | >10 | 0.22 | 0.01 | 0.003 |
| ADC6 | N.T | N.T | >10 | N.T | N.T | 0.21 |
| ADC7 | >10 | N.T | >10 | 0.008 | N.T | 0.004 |
| ADC8 | N.T | >10 | N.T | N.T | 0.008 | N.T |
| *NT: not tested. | | | | | | |

Most of the antibody-drug complexes were confirmed to have substantially improved cytotoxicity in pancreatic cancer cell lines with DLK1 over-expression than in pancreatic cancer cell lines without DLK1 expression. In pancreatic cancer cell lines over-expressing DLK1, pyrrolobenzodiazepine based antibody-drug complexes (ADCs 5, 7 and 8) were confirmed to exhibit stronger cytotoxicity than auristatin-based antibody-drug complexes (ADCs 1, 2, 3 and 4). Further,, pyrrolobenzodiazepine based antibody-drug complexes was confirmed to exhibit stronger cytotoxicity compared to auristatin-based antibody-drug complexes with longer reaction time.

Experimental Example 2: In vivo efficacy assessment

In a patient-derived tumor animal model, the efficacy of the ADCs prepared in <Embodiment 3> was assessed.

Specifically, the patient-derived small cell lung cancer mouse model (PDX model) was provided by Champions Oncology. The control group of the PDX model was intravenously administered PBS in the tail, while the experimental groups were administered ADC4 at 6mpk/Q4D*4, 6 mpk/QW*4 and 9 mpk/QW*4, followed by measurement of tumor size.

In the results, as shown in Fig. 1, tumors disappeared from all 8 individual experimental animals in each of the 6 mpk/Q4D*4 group and the 9 mpk/QW*4 group (CR, complete remission), while tumors disappeared in 6 of the 8 experimental animals in the 6 mpk/QW*4 group.

## INDUSTRIAL APPLICABILITY

The new antibody-drug conjugates (ADCs) targeting DLK1 and pharmaceutical compositions comprising the same may be used in production of drugs for treatment and/or prevention of diseases proliferative and/or angiogenetic diseases, for example, cancer.

## Claims

**1.** An antibody conjugate of General Formula I, or a pharmaceutically acceptable salt or solvate thereof:

[General Formula I] Ab - (X)y

where, in the formula:

Ab is an antibody binding specifically to DLK1 (delta-like 1 homolog (Drosophila)) comprising a heavy chain variable region and a light chain variable region, or an antigen-binding fragment thereof; wherein the antibody which binds to DLK1 or antigen-binding fragment of the same comprises:

a heavy chain variable region comprising at least one heavy chain CDR1 selected from the group comprising SEQ ID NO. 2, 16, 30, 44, 58, 72 and 86,
at least one heavy chain CDR2 selected from the group comprising SEQ ID NO. 4, 18, 32, 46, 60, 74 and 88, and at least one heavy chain CDR3 selected from the group comprising SEQ ID NO. 6, 20, 34, 48, 62, 76 and 90; and
a light chain variable region comprising at least one light chain CDR1 selected from the group comprising SEQ ID No. 9, 23, 37, 51, 65, 79, 93, 115 and 121,
at least one light chain CDR2 selected from the group comprising SEQ ID No. 11, 25, 39, 53, 67, 81 and 95, and at least one light chain CDR3 selected from the group comprising SEQ ID No. 13, 27, 41, 55, 69, 83, 97, 116 and 125;

X is independently a chemical residue comprising at least one active agent and a linker, wherein the linker links the antibody and the active agent, and
y is an integer of 1 through 20.

**2.** The antibody which binds with specificity to DLK1 or an antigen-binding fragment thereof according to Claim 1, comprising:

at least one heavy chain FR1 selected from the group comprising SEQ ID No. 1, 15, 29, 43, 57, 71, 85 and 119;
at least one heavy chain FR2 selected from the group comprising SEQ ID No. 3, 17, 31 , 45, 59, 73 and 87;
at least one heavy chain FR3 selected from the group comprising SEQ ID No. 5, 19, 33, 47, 61, 75 and 89;
at least one heavy chain FR4 selected from the group comprising SEQ ID No. 7, 21, 35, 49, 63, 77 and 91;
at least one light chain FR1 selected from the group comprising SEQ ID No. 8, 22, 36, 50, 64, 78, 92, 117 and 120;
at least one light chain FR2 selected from the group comprising SEQ ID No. 10, 24, 38, 52, 66, 80, 94 and 122;
at least one light chain FR3 selected from the group comprising SEQ ID No. 12, 26, 40, 54, 68, 82, 96, 118 and 123; and
at least one light chain FR4 selected from the group comprising SEQ ID No. 14, 28, 42, 56, 70, 84, 98 and 125.

**3.** The antibody which binds with specificity to DLK1 or an antigen-binding fragment thereof according to Claim 1, **characterized in that** it comprises a heavy chain variable region comprising a sequence that has at least 90% sequence homology with a sequence selected from the group comprising SEQ ID No. 99, 101, 103, 105, 107, 109, 111 and 127.

**4.** The antibody which binds with specificity to DLK1 or an antigen-binding fragment thereof according to Claim 1,

**characterized in that** it comprises a light chain variable region comprising a sequence that has at least 90% sequence homology with a sequence selected from the group comprising SEQ ID No. 100, 102, 104, 106, 108, 110, 112, 126 and 128.

**5.** The antibody which binds with specificity to DLK1 according to Claim 1 or an antigen-binding fragment thereof, **characterized in that** the linker has the structure of General Formula II:

[General Formula II]

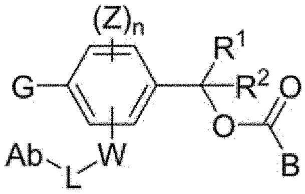

Where in the formula, the formula:

G is a glucuronic acid moiety or

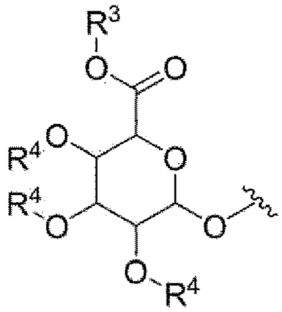

;

$R^3$ is a hydrogen or carboxyl protective group, and the respective
$R^4$ are independently hydrogen or hydroxyl protective groups;
Ab is an antibody binding with specificity to DLK1 or an antigen-binding fragment thereof:

B is an active agent;
$R_1$ and $R_2$ are, respectively and independently, hydrogen, (C1-C8) alkyl or (C3-C8) cycloalkyl;
W is -C(O)-, -C(O)NR'-, -C(O)O-, -SO2NR'-, -P(O)R"NR'-, -SONR'- or -$PO_2$NR'-; the C, S or P directly bonds to a phenyl ring; the NR' bonds to L; and R' and R" are respectively and independently, hydrogen, (C1-C8) alkyl, (C3-C8) cycloalkyl, (C1-C8) alkoxy, (C1-C8) alkylthio, mono- or di-(C1-C8) alkylamino, (C3-C20) heteroaryl, or (C6-C20) aryl;

each Z is, independently, ($C_1$-$C_8$) alkyl, halogen, cyano or nitro;
n is an integer of 1 through 3;
L is any one of the following:

A) Is a $C_1$-$C_{50}$ alkylene or a 1 to 50 atom heteroalkylene, and satisfies at least one of the following:

(i) L comprises at least one unsaturated bond
(ii) 2 atoms within L are substituted with a divalent substituent the same as in a substituent, which completes heteroarylene;
(iii) L is a 1 to 50 atom heteroalkylene, and;
(iv) The alkylene is substituted by at least one $C_{1-20}$ alkyl;

B) comprises at least one isoprenyl derivative unit of General Formula III below, which can be recognized by isoprenoid transferase;

[General Formula III]

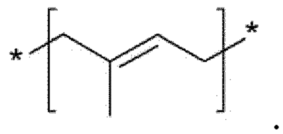

6. The antibody conjugate or a pharmaceutically acceptable salt or solvate thereof according to Claim 5, characterized in thatL is a nitrogen-containing 1 to 50 atom heteroalkylene ,
the linker comprising at least 2 atoms of a hydrophilic amino acid; and the nitrogen forms a peptide bond with a carbonyl of a hydrophilic amino acid selected from the group comprising arginine, aspartate, asparagine, glutamate, glutamine, histidine, lysine, ornithine, proline, serine or threonine.

7. The antibody conjugate or a pharmaceutically acceptable salt or solvate thereof according to Claim 5, **characterized in that** W is -C(O)NR'-, and the nitrogen of W is a nitrogen atom of a hydrophilic amino acid selected from the group comprising arginine, aspartate, asparagine, glutamate, glutamine, histidine, lysine, ornithine, proline, serine or threonine.

8. The antibody conjugate or a pharmaceutically acceptable salt or solvate thereof according to Claim 6 or Claim 7, **characterized in that** the amino acid covalently bonds an oxime of a linker to a polyethylene glycol unit of the linker.

9. The antibody conjugate or a pharmaceutically acceptable salt or solvate thereof according to Claim 6 or Claim 7, **characterized in that** the hydrophilic amino acid is an amino acid selected from the group consisting of aspartate, glutamate, ornithine, lysine, and arginine comprising a side chain having a moiety representing a charge at neutral pH in an aqueous solution.

10. The antibody conjugate or a pharmaceutically acceptable salt or solvate thereof according to Claim 5, **characterized in that** the linker comprises a peptide, the peptide comprising 2 to 20 amino acids and comprising at least one hydrophilic amino acid selected from the group comprising alanine, aspartate, asparagine, glutamate, glutamine, glycine, lysine, ornithine, proline, serine and threonine, and comprising at least one hydrophilic amino acid selected from the group consisting of aspartate, glutamate, ornithine, lysine and arginine comprising a side chain having a moiety that exhibits charge at neutral pH in an aqueous solution.

11. The antibody conjugate or a pharmaceutically acceptable salt or solvate thereof according to Claim 10, **characterized in that** W is -C(O)NR'-, and the nitrogen of W is a nitrogen atom of an N-terminal amino acid of the peptide.

12. The antibody conjugate or a pharmaceutically acceptable salt or solvate thereof according to Claim 5, **characterized in that** the peptide covalently bonds an oxime of a linker to a polyethylene glycol unit of the linker.

13. The antibody conjugate according or a pharmaceutically acceptable salt or solvate thereof to Claim 5, **characterized in that** the L is covalently bonded to an antibody by a thioether bond, and the thioether bond comprises a sulfur atom of the cysteine of the antibody.

14. The antibody conjugate or a pharmaceutically acceptable salt or solvate thereof according to Claim 13, **characterized in that** the antibody comprises an amino acid which can be recognized by isoprenoid transferase at the C-terminal of the antibody, and the thioether bond comprises a sulfur atom of the cysteine of the antibody.

15. The antibody conjugate or a pharmaceutically acceptable salt or solvate thereof according to Claim 14, **characterized in that** the amino acid motif is a CYYX sequence;

C is cysteine;
Y is an aliphatic amino acid selected from the group consisting of alanine, isoleucine, leucine, methionine and valine;
X is any one selected from the group consisting of glutamine, glutamate, serine, cysteine, methionine, alanine and leucine; and
the thioether bond comprises a sulfur atom of cysteine of the amino acid motif.

**16.** The antibody conjugate or a pharmaceutically acceptable salt or solvate thereof according to Claim 15, **characterized in that** the amino acid motif is a CVIM or CVLL sequence.

**17.** The antibody conjugate or a pharmaceutically acceptable salt or solvate thereof according to any one of Claim 14 through Claim 16, **characterized in that** at least one of the 1 to 10 amino acids preceding the amino acid motif is selected from among glycine, proline, aspartic acid, arginine and serine.

**18.** The antibody conjugate or a pharmaceutically acceptable salt or solvate thereof according to Claim 17, **characterized in that** the 1 to 10 amino acids preceding the amino acid motif are respectively glycine.

**19.** The antibody conjugate or a pharmaceutically acceptable salt or solvate thereof according to Claim 5, **characterized in that** the L comprises the amino sequence GGGGGGGCVIM at the C-terminal.

**20.** The antibody conjugate or a pharmaceutically acceptable salt or solvate thereof according to Claim 5, **characterized in that** L comprises at least one isoprenyl derivative unit of General Formula III below, which can be recognized by isoprenoid transferase:

[General Formula III]

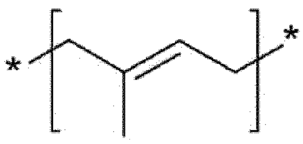

**21.** The antibody conjugate or a pharmaceutically acceptable salt or solvate thereof according to Claim 5, **characterized in that** L is a 3 to 50 heteroalkylene comprising an oxime, the oxygen atom of the oxime is on the side of L linked to W and the carbon atom of the oxime is on the side of L linked to Ab, or; the carbon atom of the oxime is on the side of L linked to W and the oxygen atom of the oxime is on the side of L linked to Ab.

**22.** The antibody conjugate or a pharmaceutically acceptable salt or solvate thereof according to Claim 20 or Claim 21, **characterized in that** L comprises an oxime, and **in that** at least one isoprenyl unit covalently links the oxime to Ab.

**23.** The antibody conjugate or a pharmace

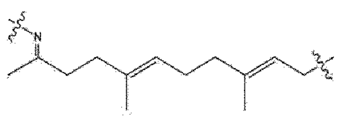

solvate thereof according

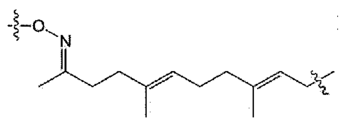

in that L comprises or

**24.** The antibody conjugate or a pharmaceutically acceptable salt or solvate thereof according to Claim 5, **characterized in that** L further comprises a connecting unit represented by General Formula VIII or General Formula IX:

[General Formula VIII] $-(CH_2)_r(V(CH_2)_p)_q-$

[General Formula IX] $-(CH_2CH_2X)_w-$

where: V is a single bond, -O-, -S-, $-NR^{21}-$, $-C(O)NR^{22}-$, $NR^{23}C(O)-$, $NR^{24}SO_2-$, or $-SO_2NR^{25}-$;

X is -O-, $C_1$-$C_8$ alkylene or -$NR^{21}$-;
$R^{21}$ to $R^{25}$ are, independently and respectively hydrogen, ($C_1$-$C_6$) alkyl, ($C_1$-$C_6$) alkyl ($C_6$-$C_{20}$) aryl, or ($C_1$-$C_6$) alkyl ($C_3$-$C_{20}$) heteroaryl;
r is an integer of 0 to 10;
p is an integer of 0 to 10;

q is an integer of 1 to 20; and
w is an integer of 1 to 20.

**25.** The antibody conjugate or a pharmaceutically acceptable salt or solvate thereof according to Claim 24, **characterized in that** L comprises 1 to 20 polyethylene glycol units represented by

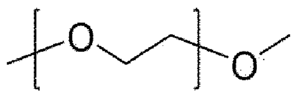 or 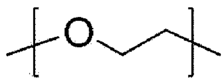.

**26.** The antibody conjugate or a pharmaceutically acceptable salt or solvate thereof according to Claim 25, **characterized in that** L comprises 1 to 12 -$OCH_2CH_2$- units.

**27.** The antibody conjugate or a pharmaceutically acceptable salt or solvate thereof according to Claim 25, **characterized in that** L comprises an oxime; and 1 to 20 polyethylene glycol units covalently bond the oxime to the active agent.

**28.** The antibody conjugate or a pharmaceutically acceptable salt or solvate thereof according to Claim 5, **characterized in that** L further comprises a binding unit formed by a 1,3-dipolar cycloaddition reaction, hetero-diels reaction, nucleophilic substitution reaction, non-aldol type carbonyl reaction, addition to carbon-carbon multiple bond, oxidation reaction or click reaction.

**29.** The antibody conjugate or a pharmaceutically acceptable salt or solvate thereof according to Claim 28, **characterized in that** the binding unit is formed by a reaction between acetylene and azide, or a reaction between aldehyde or a ketone group and hydrazine or alkoxyamine.

**30.** The antibody conjugate or a pharmaceutically acceptable salt or solvate thereof according to Claim 5, **characterized in that** L further comprises binding units represented by General Formulae IV, V, VI or VII below:

[General Formula IV]

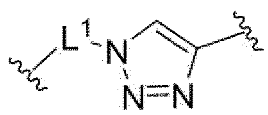

[General Formula V]

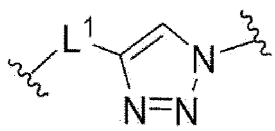

[General Formula VI]

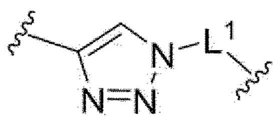

[General Formula VII]

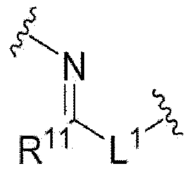

where, in the formula,

the $L^1$ is a single bond or a $C_1$-$C_{30}$ alkylene; and
$R^{11}$ is hydrogen or a $C_1$-$C_{10}$ alkyl.

**31.** The antibody conjugate or a pharmaceutically acceptable salt or solvate thereof according to Claim 30, $L^1$ is a single bond or a $C_{11}$ or $C_{12}$ alkylene.

**32.** The antibody conjugate or a pharmaceutically acceptable salt or solvate thereof according to Claim 30, **characterized in that** L comprises

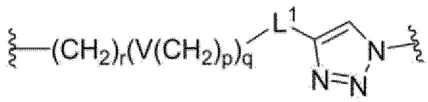

or

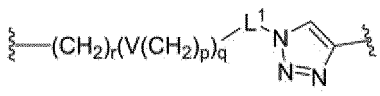

,

V is a single bond, -O-, -S-, -$NR^{21}$-, -C(O)$NR^{22}$-, $NR^{23}$C(O)-, $NR^{24}SO_2$-, or -$SO_2NR^{25}$-;
$R^{21}$ to $R^{25}$ are, independently, hydrogen, ($C_{1-6}$) alkyl, ($C_{1-6}$) alkyl ($C_{6-20}$) aryl, or ($C_{1-6}$) alkyl ($C_{3-20}$) heteroaryl;
r is an integer of 0 to 10;
p is an integer of 0 to 10;
q is an integer of 1 to 20; and
$L^1$ is a single bond.

**33.** The antibody conjugate or a pharmaceutically acceptable salt or solvate thereof according to Claim 32, **characterized in that** the L comprises any one selected from the group comprising

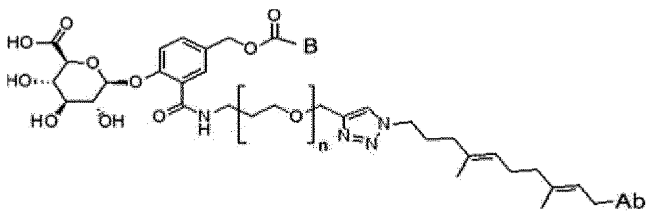

,

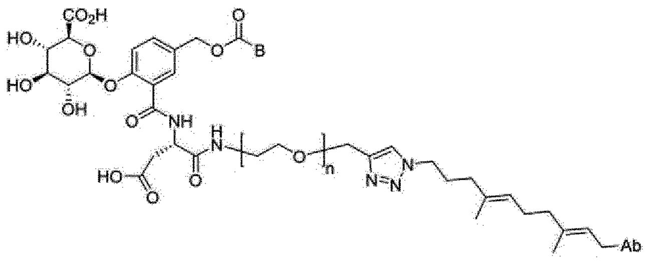

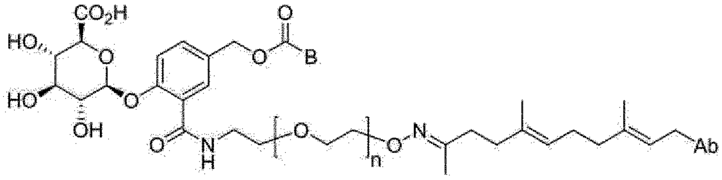

and;

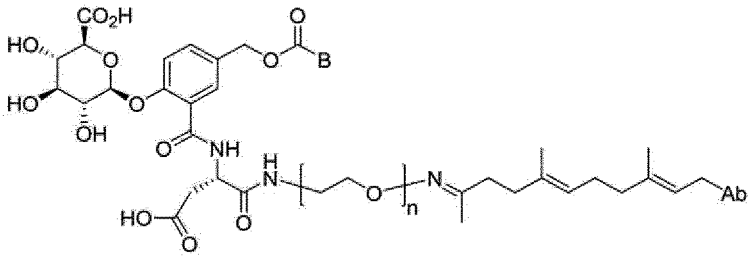

the Ab is an antibody which bonds with specificity to DLK1 or an antigen-binding fragment thereof;
B is an active agent; and
n is an integer of 0 to 20.

**34.** The antibody conjugate or a pharmaceutically acceptable salt or solvate thereof according to Claim 5, **characterized in that** the isoprenoid transferase is a farnesyl protein transferase (FTase) or a geranylgeranyl transferase (GGTase).

**35.** The antibody conjugate or a pharmaceutically acceptable salt or solvate thereof according to Claim 5, **characterized in that** L comprises at least one branch linker covalently bonded to Ab,

i) the respective branch linkers comprise a branched unit (BR) covalently bonded to Ab by a primary linker (PL);
ii) the respective branch linkers bind a first active agent to a branched unit, and comprise a first branch (B1) comprising a second linker (SL) and a cleaving group (CG); and
iii) the respective branch linkers comprise a) a second branch (B2) wherein a second active agent is covalently bonded to a branched unit by a second linker (SL) and a cleaving group (CG), or b) a second branch wherein a polyethylene glycol moiety is covalently bonded to a branched unit;
iv) and the respective cleaving groups are hydrolyzed to release an active agent from an antibody conjugate.

**36.** The antibody conjugate or a pharmaceutically acceptable salt or solvate thereof according to Claim 35, **characterized in that** the branch linker is

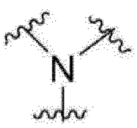

,

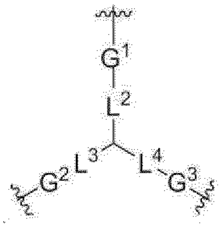

,

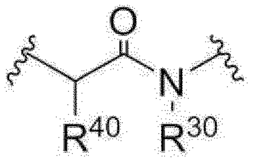 or 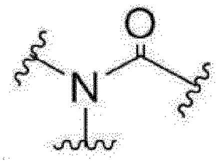 ;

the $L^2$, $L^3$ and $L^4$ are, respectively and independently, direct bonds or $-C_nH_{2n}-$; n is an integer of 1 to 30; the $G^1$, $G^2$ and $G^3$ are, independently, a direct bond

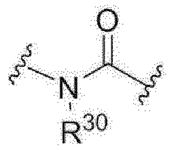 , 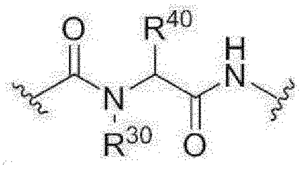 ,

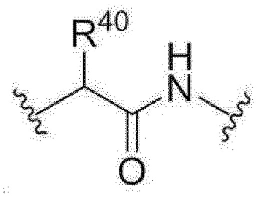 or 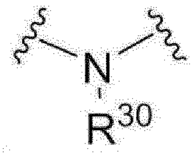

; and the $R^{30}$ is hydrogen or $C_{1-30}$ alkyl.

**37.** The antibody conjugate or a pharmaceutically acceptable salt or solvate thereof according to Claim 36, **characterized in that** it comprises

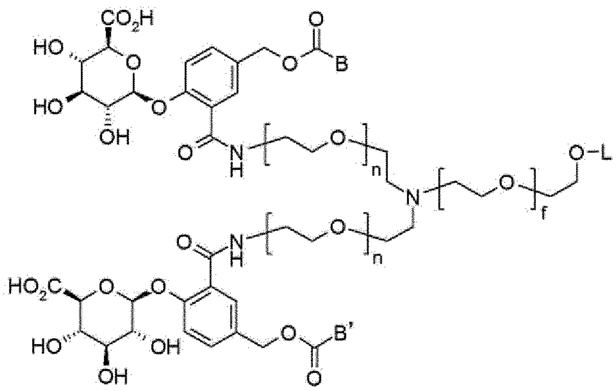

,

wherein the B and B' indicate active agents which may be different or identical;
n indicates, respectively and independently, an integer of 0 to 30;
f indicates, respectively and independently, an integer of 0 to 30; and
L indicates a bond to Ab.

**38.** The antibody conjugate or a pharmaceutically acceptable salt or solvate thereof according to Claim 37, **characterized in that** L comprises an oxime and 1 to 20 polyethylene glycol unit(s) which covalently binds the oxime to the active agent.

**39.** The antibody conjugate or a pharmaceutically acceptable salt or solvate thereof according to Claim 35, **characterized in that** the cleavage group may be cleaved within a target cell, and the cleavage group may release one or more active agents.

**40.** The antibody conjugate or a pharmaceutically acceptable salt or solvate thereof according to Claim 5, **characterized**

**in that** L comprises 1 to 6 branched linkers covalently bonded to Ab; and 2 to 6 identical or different active agents covalently bonded to the branched linkers.

**41.** The antibody conjugate or a pharmaceutically acceptable salt or solvate thereof according to Claim 40, **characterized in that** the respective active agents are bonded to the branched linker by a cleavable bond.

**42.** The antibody conjugate or a pharmaceutically acceptable salt or solvate thereof according to Claim 40, **characterized in that** the respective branched linkers comprise a branched unit, the respective active agents are bonded to the branched unit through a second linker, and the branched units are bonded to Ab by a first linker.

**43.** The antibody conjugate or a pharmaceutically acceptable salt or solvate thereof according to Claim 42, **characterized in that** the branched unit is a nitrogen unit or a lysine unit.

**44.** The antibody conjugate or a pharmaceutically acceptable salt or solvate thereof according to Claim 42, **characterized in that** the branched unit is an amide, and the first linker or the second linker comprises a carbonyl of the amide.

**45.** The antibody conjugate or a pharmaceutically acceptable salt or solvate thereof according to Claim 5, **characterized in that** the active agent is a chemotherapeutic agent or a toxin.

**46.** The antibody conjugate or a pharmaceutically acceptable salt or solvate thereof according to Claim 5, **characterized in that** the active agent is an immunoregulatory compound, anti-cancer agent, antiviral, antibacterial, antifungal, antiparasitic or a combination thereof.

**47.** The antibody conjugate or a pharmaceutically acceptable salt or solvate thereof according to Claim 5, **characterized in that** the active agent is selected from the following:

(a) erlotinib, bortezomib, fulvestrant, sutent, letrozole, imatinib mesylate, PTK787/ZK 222584, oxaliplatin, 5-fluorouracil, leucovorin, rapamycin, lapatinib, lonafarnib, sorafenib, gefitinib, AG1478, AG1571, thiotepa, cyclophosphamide, busulfan, improsulfan, piposulfan, benzodopa, carboquone, meturedopa, uredopa, ethylenimine, altretamine, triethylenemelamine, triethylenephosphoramide, triethylenethiophosphoramide, teimethylolomelamine, bullatacin, bullataciionone, camptothecin, topotecan, bryostatin, callystatin, CC-1065, adozelesin, carzelesin, bizelesin, cryptophycin 1, cryptophycin 8, dolastatin, duocarmycin, KW-2189, CB1-TM1, eleutherobin, pancratistatin, sarcodictyin, spongistatin, chlorambucil, chlomaphazine, cholophosphamide, estramustine, ifosfamide, mechlorethamine, melphalan, novembichin, phenesterine, prednimustine, trofosfamide, uracil mustard, carmustine, chlorozotoxin, fotemustine, lomustine, nimustine, ranimustine, calicheamicin, calicheamicin gamma 1, calicheamicin omega 1, dynemicin, dynemicin A, clodronate, esperamicin, neocarzinostatin chromophore, aclacinomysins, actinomycin, antrymycin, azaserine, bleomycins, catcinomycin, carabicin, carninomycin, carzinophilin, chromomycins, dactinomycin, daunorubicin, detorubucin, 6-diazo-5-oxo-L-norleucine, doxorubicin, morpholino-doxorubicin, cyanomorpholino-doxorubicin, 2-pyrrolino-doxorubucin, liposomal doxorubicin, deoxydoxorubicin, epirubicin, esorubicin, marcellomycin, mitomycin C, mycophenolic acid, nogalamycin, olivomycins, peplomycin, potfiromycin, puromycin, quelamycin, rodorubicin, streptomigrin, streptozocin, tudercidin, ubenimex, zinostatin, zorubicin, 5-fluoDLKacil, denopterin, methotrexate, pteropterin, trimetrexate, fludarabine, 6-mercaptopurine, thiamiprine, thiguianine, ancitabine, azacytidine, 6-azauridine, carmofur, cytarabine, dideoxyuridine, doxifluridine, enocitabine, floxuridine, calusterone, dromostanolone, propionate, epitiostanol, mepitiostane, testolactone, aminoglutethimide,mitotane, trilostane, folinic acid, aceglatone, aldophosphamide glycoside, aminolevulinic acid, eniluracil, amsacrine, bestrabucil, bisantrene, edatrexate, defofamine, democolcine, diaziquone, elfornithine, elliptinium acetate, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, polysaccharide-k, razoxane, rhizoxin, sizofiran, spirogermanium, tenuazonic acid, triaqizuone, 2,2', 2''-trichlorotriethylamine, T-2 toxin, verracurin A, DLKidin A, anguidine, urethane, vindesine, dacarbazine, mannomustine, mitobronitol, mitolactol, pipobroman, gacytosine, arabinoside, cyclophosphamide, thiotepa, paclitaxel, albumin-engineered nanoparticle formulation of paclitaxel, docetaxel, chlorambucil, gemcitabine, 6-thioguanine, mercaptopurine, cisplatin, carboplatin, vinblastine, platinum, etoposide, ifosfamide, mitoxantrone, vincristine, vinorelbine, novantrone, teniposide, edatrexate, daunomycin, aminopterin, xeloda, ibandronate, CPT-11, topoisomerase inhibitor RFS 2000, difluoromethylornithine, retinoic acid, capecitabine, or pharmaceutically acceptable salts, solvates or acids thereof;
(b) monokine, lympokine, traditional polypeptide hormone, parathyroid hormone, thyroxine, relaxin, pDLKelaxin, glycoprotein hormone, follicle stimulating hormone, thyroid stimulating hormone, luteinizing hormone, hepatic

growth factor fibroblast growth factor, prolactin, placental lactogen, tumor necrosis factor, tumor necrosis factor-a, tumor necrosis factor-β, Mullerian inhibiting substance, mouse gonadotropin associated peptide, inhibin, activin, vascular endothelial growth factor, thrombopoietin, erythropoietin, osteoinductive factor, interferon, interferon-a, interferon-β, interferon-y, colony stimulating factor (CSF), macrophage-CSF, granulocyte-macrophage-CSF), granulocyte-macrophage-CSF, granulocyte-CSF, interleukin (IL), IL-1, IL-1α, IL-2, IL-3, IL-4, IL-5, IL-6, IL-7, IL-8, IL-9, IL-10, IL-11, I L-12, tumor necrosis factor, TNF-α, TNF-β, polypeptide factor, LIF, kit ligand, or mixtures thereof;
(c) diphtheria toxin, botulinum toxin, tetanus toxin, dysentery toxin, cholera toxin, amanitin, α-amatinin, pyrrolobenzodiazepine, pyrrolobenzodiazepine derivative, indolinobenzodiazepine, pyridobenzodiazepine, tetrodotoxin, brevetoxin, ciguatoxin, ricin, AM toxin, auristatin, tubulysin, geldanamycin, maytansinoid, calicheamicin, daunomycin, doxorubicin, methotrexate, vindesine, SG2285, dolastatin, dolastatin analog, auristatin, cryptophycin, camptothecin, rhizoxin, rhizoxin derivatives, CC-1065, CC-1065 analogs or derivatives, duocarmycin, enediyne antibiotic, esperamicin, epothilone, toxoid, or mixtures thereof;
(d) affinity ligand, where the affinity ligand is a substrate, inhibitor, active agent, neurotransmitter, radioactive isotope, or mixtures thereof;
(e) radioactive label, 32P, 35S, fluorescent dye, electron dense reagent, enzyme, biotin, streptavidin, dioxigenin, hapten, immunogenic protein, nucleic acid molecule with a sequence complementary to a target, or mixtures thereof;
(f) immunomodulatory compound, anti-cancer agent, anti-viral agent, anti-bacterial agent, antifungal agent, anti-parasitic agent, or mixtures thereof;
(g) tamoxifen, raloxifene, droloxifene, 4-hydroxytamoxifen, trioxifene, keoxifene, LY117018, onapristone or toremifene;
(h) 4(5)-imidazole, aminoglutethimide, megestrol acetate, exemestane, letrozole or anastrozole
(i) flutamide, nilutamide, bicalutamide, leuprolide, goserelin, or troxacitabine;
(j) aromatase inhibitor;
(k) protein kinase inhibitor;
(l) lipid kinase inhibitor;
(m) antisense oligonucleotide;
(n) ribozyme;
(o) vaccine; and
(p) anti-angiogenic agent.

**48.** The antibody conjugate or a pharmaceutically acceptable salt or solvate thereof according to Claim 1, **characterized in that** the the active agent is a pyrrolobenzodiazepine dimer, wherein the linker links Ab to the N10 or N'10 position of the pyrrolobenzodiazepine dimer.

**49.** The antibody conjugate or a pharmaceutically acceptable salt or solvate thereof according to Claim 48, **characterized in that** the activator is a pyrrolobenzodiazepine dimer;

the pyrrolobenzodiazepine dimer is substituted by X at the N10 position or by X' at the N'10 position, where X or X' links the pyrrolobenzodiazepine dimer to the linker;
X and X' are, respectively and independently, -C(O)O*, -S(O)O-*, -C(O)-*, -C(O)NR$^X$-*, - S(O)$_2$NR$^X$-*, -P(O)R'NR$^X$-*, -S(O)NR$^x$-*, or -PO$_2$NR$^X$-*;
R$^X$ is C$_{1-8}$ alkyl, C$_{3-8}$ cycloalkyl, C$_{3-20}$ heteroaryl or C$_{5-20}$ aryl;
R$^{X'}$+ is OH, N$_3$, CN, SH, C$_{1-8}$ alkyl, C$_{3-8}$ cycloalkyl, C$_{1-8}$ alkoxy, C$_{1-8}$ alkylthio, C$_{3-20}$ heteroaryl, C$_{5-20}$ aryl or amino; and
* is a binding site between the pyrrolobenzodiazepine dimer and the linker.

**50.** The antibody conjugate or a pharmaceutically acceptable salt or solvate thereof according to Claim 49, **characterized in that** the pyrrolobenzodiazepine dimer is represented by General Formula X or General Formula XI below:

[General Formula X]

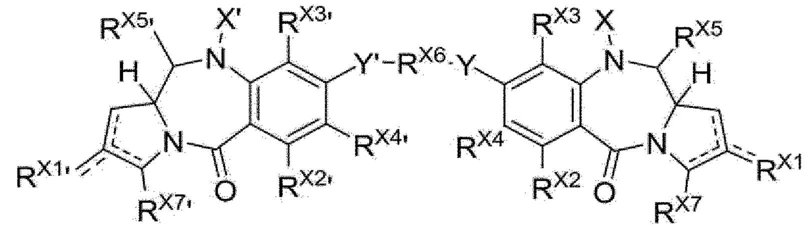

[General Formula XI]

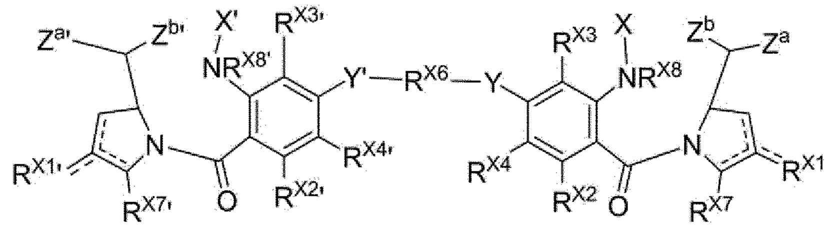

where, in the formula:

the dotted lines denote the selective existence of double bonds between C1 and C2, between C2 and C3, between C'1 and C'2, or between C'2 and C'3;

$R^{X1}$ and $R^{X1'}$ are independently selected from H, OH, =O, $=CH_2$ CN, $R^m$ $OR^m$, $=CH-R^{m'}$, $=C(R^{m'})_2$, $O-SO_2-R^m$, $CO_2R^m$, $COR^m$, halo and dihalo;

$R^{m'}$ is selected from among $R^m$, $CO_2R^m$, $COR^m$, CHO, $CO_2H$ and halo;

each $R^m$ is independently selected from a group comprised of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl and 5 to 7 membered heteroaryl;

$R^{X2}$, $R^{X2'}$, $R^{X3}$, $R^{X3'}$, $R^{X5}$ and $R^{X5'}$ are independently selected from among H, $R^m$, OH, $OR^m$, SH, $SR^m$, $NH_2$, $NHR^m$, $NR^m{}_2$, $NO_2$, $Me_3Sn$ and halo;

$R^{X4}$ and $R^{X4'}$ are independently selected from among H, $R^m$, OH, $OR^m$, SH, $SR^m$, $NH_2$, $NHR^m$, $NR^m{}_2$, $NO_2$, $Me_3Sn$, halo, $C_{1-6}$ alkyl, $C_{1-6}$ alkenyl, $C_{2-6}$ alkynyl, $C_{2-6}$ aryl, $C_{3-6}$ heteroaryl, $C_{3-6}$ cycloalkyl, 3 to 7 membered heterocycloalkyl, $C_{5-12}$ aryl, 5 to 7 membered heteroaryl, -CN, -NCO, $-OR^n$, $-OC(O)R^n$, $-OC(O)NR^nR^{n'}$, $-OS(O)R^n$, $-OS(O)_2R^n$, $-SR^n$, $-S(O)R^n$, $-S(O)_2R^n$, $-S(O)NR^nR^{n'}$, $-S(O)_2NR^nR^{n'}$, $-OS(O)NR^nR^{n'}$, $-OS(O)_2NR^nR^{n'}$, $-NR^nR^{n'}$, $-NR^nC(O)R^o$, $-NR^nC(O)OR^o$, $-NR^nC(O)NR^oR^{o'}$, $-NR^nS(O)R^o$, $-NR^nS(O)_2R^o$, $-NR^nS(O)NR^oR^{o'}$, $-NR^nS(O)_2NR^oR^{o'}$, $-C(O)R^n$, $-C(O)OR^n$ and $-C(O)NR^nR^{n'}$;

$R^X$ and $R^{X'}$ are independently selected from among H, OH, $N_3$, CN, $NO_2$, SH, $NH_2$, $ONH_2$, $NHNH_2$, halo, $C_{1-8}$ alkyl, $C_{3-8}$ cycloalkyl, $C_{1-8}$ alkoxy, $C_{1-8}$ alkylthio, $C_{3-20}$ heteroaryl, $C_{5-20}$ aryl or mono- or di-$C_{1-8}$ alkylamino;

Y and Y' are independently selected from O, S and N(H);

$R^{x6}$ is independently selected from $C_{3-12}$ alkylene, $C_{3-12}$ alkenylene, or $C_{3-12}$ heteroalkylene;

$R^{X7}$ and $R^{X7'}$ are independently selected from among H, $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, 5 to 7- membered heteroaryl, $-OR^r$, $-OC(O)R^r$, $-OC(O)NR^rR^{r'}$, $-OS(O)R^r$, $-OS(O)_2R^r$, $-SR^r$, $-S(O)R^r$, $-S(O)_2R^r$, $-S(O)NR^rR^{r'}$, $-S(O)_2NR^rR^{r'}$, $-OS(O)NR^rR^{r'}$, $-OS(O)_2NR^rR^{r'}$, $-NR^rR^{r'}$, $-NR^rC(O)R^s$, $-NR^rC(O)OR^s$, $-NR^rC(O)NR^sR^{s'}$, $-NR^rS(O)R^s$, $-NR^rS(O)_2R^s$, $-NR^rS(O)NR^sR^{s'}$, $-NRr^S(O)_2NR^sR^s$, $-C(O)R^r$, $-C(O)OR^s$ or $-C(O)NR^rR^{r'}$;

each $R^r$, $R^{r'}$, $R^s$ and $R^{s'}$ is independently selected from H, $C_{1-7}$ alkyl, $C_{2-7}$ alkenyl, $C_{2-7}$ alkynyl, $C_{3-13}$ cycloalkyl, 3 to 7 membered heterocycloalkyl, $C_{5-10}$ aryl and 5 to 7 membered heteroaryl;

each $R^{X8}$ and $R^{X8'}$ is independently selected from H, $C_{1-6}$ alkyl, $C_{2-6}$ alkenyl, $C_{2-6}$ alkynyl, $C_{3-6}$ heteroalkyl, 3 to 7 membered heterocycloalkyl, $C_{5-10}$ aryl, 5 to 7 membered heteroaryl, - $S(O)R^m$, $-S(O)_2R^m$, $-S(O)NR^mR^{m'}$, $-S(O)_2NR^mR^{m'}$, $-NR^mR^{m'}$, $-NR^mC(O)R^m$, - $NR^mC(O)OR^n$, $-NR^mC(O)NR^nR^{n'}$, $-NR^mS(O)R^n$, $-NR^mS(O)_2R^n$, $-NR^mS(O)NR^nR^{n'}$, - $NR^mS(O)_2NR^nR^{n'}$, $-C(O)R^m$, $-C(O)OR^m$ and $-C(O)NR^mR^{m'}$;

$Z^a$ is selected from among $OR^{X12a}$, $NR^{X12a}R^{X12a}$, or $SR^{X12a}$;

$Z^b$ is selected from among $OR^{X13a}$, $NR^{X13a}R^{X13a}$, or $SR^{X13a}$;

$Z^{a'}$ is selected from among $OR^{X12a}$, $NR^{X12a}R^{X12a}$, or $SR^{X12a}$;

$Z^{b'}$ is selected from among $OR^{X13a'}$, $NR^{X13a'}R^{X13a'}$, or $SR^{X13a'}$;

each $R^{X12a}$, $R^{X12a'}$, $R^{X13a'}$ and $R^{x13a'}$ is independently selected from H, $C_{1-6}$ alkyl, $C_{2-6}$ alkenyl, $C_{2-6}$ alkynyl, $C_{3-6}$ cycloalkyl, 3 to 7 membered heterocycloalkyl, $C_{5-10}$ aryl, 5 to 7 membered heteroaryl, -C(O)$R^{X15a+}$, -C(O)O$R^{X15a}$ and -C(O)N$R^{X15a}R^{X15a'}$; and

each $R^{X15a}$ and $R^{x15a'}$ is independently selected from $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl, and 5 to 7 membered heteroaryl; and

the $R^{X13a}$ and $R^{X14a}$ optionally join atoms to which they are attached to form a 3 to 7 membered heterocyclyl, 3 to 7 membered heterocycloalkyl, or 3 to 7 membered heteroaryl, and the $R^{X13a'}$ and $R^{X14a'}$ optionally join the atoms to which they are attached to form 3 to 7 membered heterocyclyl, 3 to 7 membered heterocycloalkyl, or 3 to 7 membered heteroaryl; and

each of the $R^n$, $R^{n'}$, $R^o$, $R^{o'}$, $R^p$ and $R^{p'}$ is independently selected from among H, $C_{1-7}$ alkyl, $C_{2-7}$ alkenyl, $C_{2-7}$ alkynyl, $C_{3-13}$ cycloalkyl, 3 to 7 membered heterocycloalkyl, $C_{5-10}$ aryl, and 5 to 7 membered heteroaryl.

**51.** The antibody conjugate or a pharmaceutically acceptable salt or solvate thereof according to Claim 50, **characterized in that** the $R^m$ is independently selected from a group comprised of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl and 5 to 7 membered heteroaryl; and the $R^m$ is additionally substituted with of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3 to 7 membered heterocyclyl, 3 to 7 membered heterocycloalkyl or 5 to 7 membered heteroaryl.

**52.** The antibody conjugate or a pharmaceutically acceptable salt or solvate thereof according to Claim 50, **characterized in that** the $R^{x4}$ and $R^{x4'}$ are independently selected from among H, $R^m$, OH, $OR^m$, SH, $SR^m$, $NH_2$, $NHR^m$, $NR^mR^{m'}$, $NO_2$, $Me_3Sn$, halo, $C_{1-6}$ alkyl $C_{1-6}$ alkoxy, $C_{2-6}$ alkenyl, $C_{2-6}$ alkynyl, $C_{3-6}$ cycloalkyl, 3 to 7 membered heterocycloalkyl, $C_{5-12}$ aryl, 5 to 7 membered heteroaryl, -CN, -NCO, -$OR^n$, -OC(O)$R^n$, -OC(O)$NR^nR^{n'}$, -OS(O)$R^n$, -OS(O)$_2R^n$, - $SR^n$, -S(O)$R^n$, -S(O)$_2R^n$, -S(O)$NR^nR^{n'}$, -S(O)$_2NR^nR^{n'}$, -OS(O)$NR^nR^{n'}$, -OS(O)$_2NR^nR^{n'}$, -$NR^nR^{n'}$, - $NR^n$C(O)$R^o$, -$NR^n$C(O)$OR^o$, -$NR^n$C(O)$NR^oR^{o'}$, -$NR^n$S(O)$R^o$, -$NR^n$S(O)$_2R^o$, -$NR^n$S(O)$NR^oR^{o'}$,-$NR^n$S(O)$_2NR^oR^{o'}$, -C(O)$R^n$, -C(O)$OR^n$ and -C(O)$NR^nR^{n'}$; and the $R^{X4}$ or $R^{X4'}$ is $C_{1-6}$ alkyl, $C_{1-6}$ alkoxy, $C_{2-6}$ alkenyl, $C_{2-6}$ alkynyl, $C_{3-6}$ cycloalkyl, 3 to 7 membered heterocycloalkyl, $C_{5-12}$ aryl or 5 to 7 membered heteroaryl, and additionally is substituted with at least one $C_{1-6}$ alkyl, $C_{1-6}$ alkoxy, $C_{2-6}$ alkenyl, $C_{2-6}$ alkynyl, $C_{3-6}$ cycloalkyl, 3 to 7 membered heterocycloalkyl, $C_{5-10}$ aryl, 5 to 7 membered heteroaryl, -$OR^p$, -OC(O)$R^p$, -C(O)$NR^pR^{p'}$, -OS(O)$R^p$, -OS(O)$_2R^p$, -$SR^p$,-S(O)$R^p$, -S(O)$_2R^p$, -S(O)$NR^pR^{p'}$, -S(O)$_2NR^pR^{p'}$, -OS(O)$NR^pR^{p'}$, -OS(O)$_2NR^pR^{p'}$, -$NR^pR^{p'}$,-$NR^p$C(O)$R^q$, -$NR^p$C(O)$OR^q$, -$NR^p$C(O)$NR^qR^{q'}$, -$NR^p$S(O)$R^q$, -$NR^p$S(O)$_2R^q$, -$NR^p$S(O)$NR^qR^{q'}$, - $NR^p$S(O)$_2NR^qR^{q'}$, -C(O)$R^p$, -C(O)$OR^p$ or -C(O)$NR^pR^p$.

**53.** The antibody conjugate or a pharmaceutically acceptable salt or solvate thereof according to Claim 50, **characterized in that** $R^{X7}$ and $R^{X7'}$ are independently selected from H, $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, 5 to 7 membered heteroaryl, -$OR^r$, -OC(O)$R^r$, -OC(O)$NR^rR^{r'}$, -OS(O)$R^r$, -OS(O)$_2R^r$, -$SR^r$, -S(O)$R^r$, - S(O)$_2R^r$, -S(O)$NR^rR^{r'}$, -S(O)$_2NR^rR^{r'}$, -OS(O)$NR^rR^{r'}$, -OS(O)$_2NR^rR^{r'}$, -$NR^rR^{r'}$, -$NR^r$C(O)$R^s$, - $NR^r$C(O)$OR^s$, -$NR^r$C(O)$NR^sR^{s'}$, -$NR^r$S(O)$R^s$, -$NR^r$S(O)$_2R^s$, -$NR^r$S(O)$NR^sR^{s'}$, -$NR^r$S(O)$_2NR^sR^s$, - C(O)$R^r$, -C(O)$OR^s$ or -C(O)$NR^rR^{r'}$; $R^{X7}$ and $R^{X7'}$ are independently selected from among H, $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, or 5 to 7 membered heteroaryl, and are additionally substituted by $C_{1-6}$ alkyl, $C_{2-6}$ alkenyl, $C_{2-6}$ alkynyl, $C_{3-6}$ cycloalkyl, 3 to 7 membered heterocycloalkyl, $C_{6-10}$ aryl, 5 to 7 membered heteroaryl, -$OR^t$, -OC(O)$R^t$, -OC(O)$NR^tR^{t'}$, -OS(O)$R^t$, -OS(O)$_2R^t$, -$SR^t$, -S(O)$R^t$, -S(O)$_2R^t$, - S(O)$NR^tR^{t'}$, -S(O)$_2NR^tR^{t'}$, -OS(O)$NR^tR^{t'}$, -OS(O)$_2NR^tR^{t'}$, -$NR^tR^{t'}$, -$NR^t$C(O)$R^u$, -$NR^t$C(O)$OR^u$,-$NR^t$C(O)$NR^uR^{u'}$, -$NR^t$S(O)$R^u$, -$NR^t$S(O)$_2R^u$, -$NR^t$S(O)$NR^uR^{u'}$, -$NR^t$S(O)$_2NR^uR^{u'}$, -C(O)$R^t$, - C(O)$OR^t$ or -C(O)$NR^tR^{t'}$; and the $R^r$, $R^{r'}$, $R^s$, $R^{s'}$, $R^t$, $R^{t'}$, $R^u$ and $R^{u'}$ are independently selected from among H, $C_{1-7}$ alkyl, $C_{2-7}$ alkenyl, $C_{2-7}$ alkynyl, $C_{3-13}$ cycloalkyl, 3 to 7 membered heterocycloalkyl, $C_{5-10}$ aryl, and 5 to 7 membered heteroaryl.

**54.** The antibody conjugate or a pharmaceutically acceptable salt or solvate thereof according to Claim 50, **characterized in that** the $R^{x6}$ is $C_{3-12}$ alkylene, $C_{3-12}$ alkenylene or $C_{3-12}$ heteroalkylene, with the $R^{x6}$ substituted by -NH2, -$NHR^m$, -NHC(O)$R^m$, -NHC(O)$CH_2$, $[OCH_2CH_2]_n$-$R^{XX}$, or -$[CH_2CH_2O]_n$-$R^{XX}$;the $R^{XX}$ is H, OH, N3, CN, $NO_2$, SH, $NH_2$, $ONH_2$, $NHNH_2$, halo, $C_{1-8}$ alkyl, $C_{3-8}$ cycloalkyl, $C_{1-8}$ alkoxy, $C_{1-8}$ alkylthio, $C_{3-20}$ heteroaryl, $C_{5-20}$ aryl or mono- or di-$C_{1-8}$ alkyl amino; and n is an integer of 1 through 6.

**55.** The antibody conjugate or a pharmaceutically acceptable salt or solvate thereof according to Claim 50, **characterized in that** the active agent is a pyrrolobenzodiazepine dimer denoted by General Formula XII or General Formula XIII;

[General Formula XII]

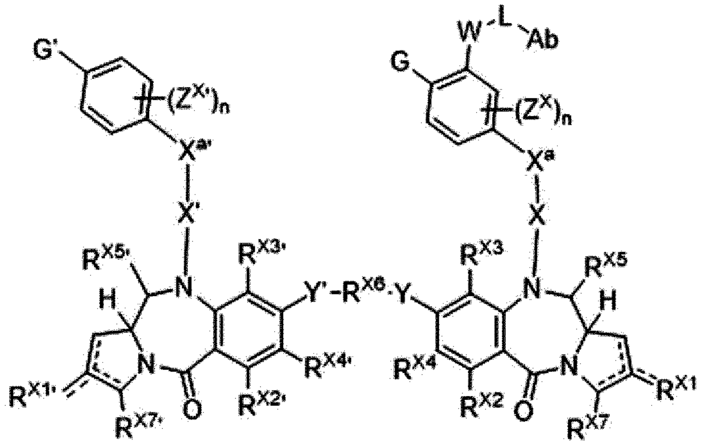

[General Formula XIII]

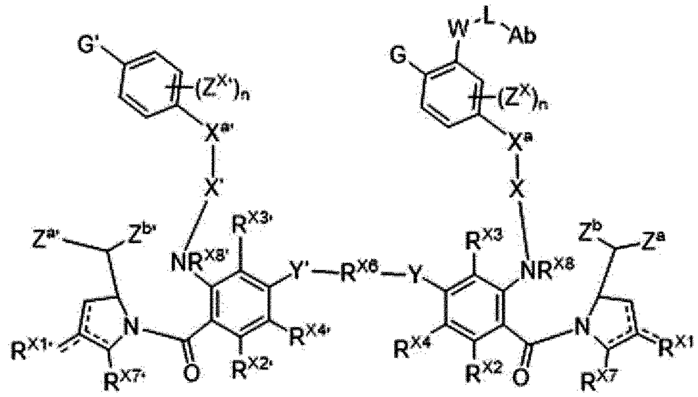

$X^a$ and $X^{a'}$ are independently selected from a bond or $C_{1-6}$ alkylene;
$Z^{X'}$ and $Z^X$ are independently selected from among hydrogen, $C_{1-8}$ alkyl, halogen, cyano, nitro,

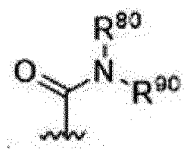,

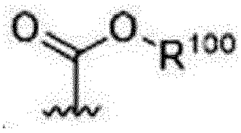

or $-(CH_2)_m-OCH_3$;

the respective $R^{80}$, $R^{90}$ and $R^{100}$ are selected from among hydrogen, $C_{1-8}$ alkyl, $C_{2-6}$ alkenyl and $C_{1-6}$ alkoxy; and m is an integer of 0 to 12.

**56.** The antibody conjugate or a pharmaceutically acceptable salt or solvate thereof according to Claim 1, **characterized in that** the active agent is selected from among the group comprising:

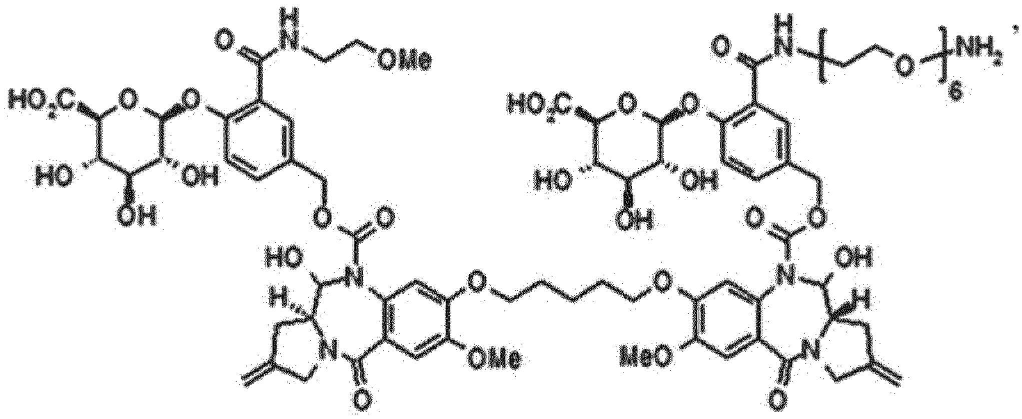

,

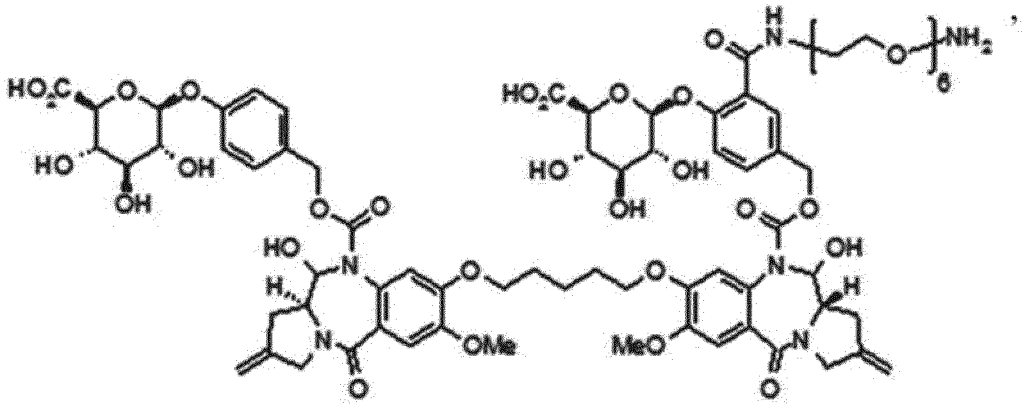

,

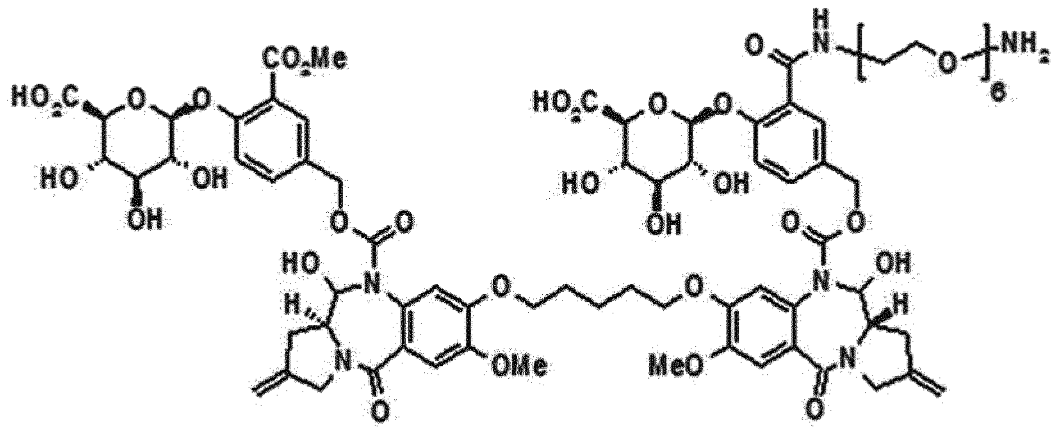

,

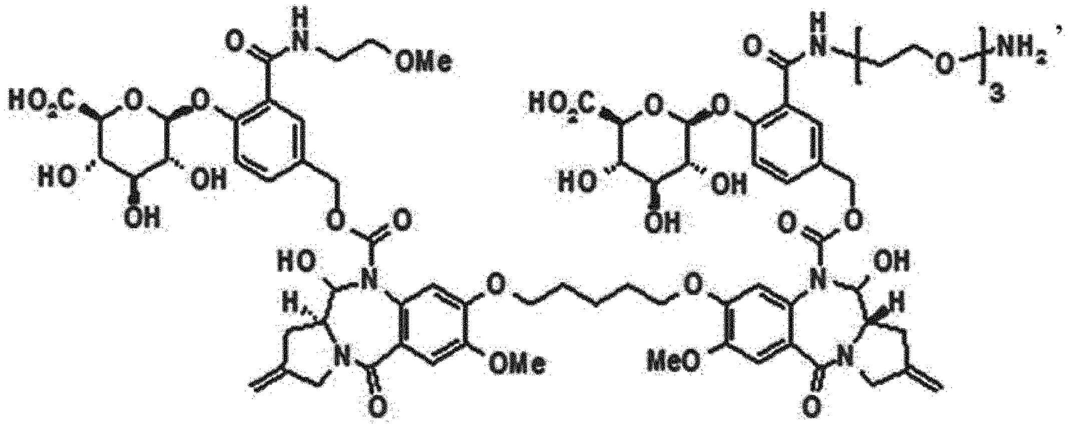

,

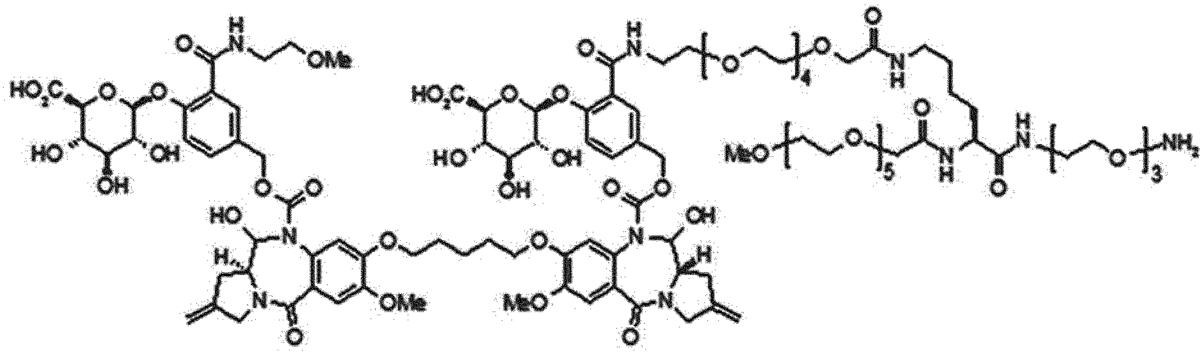

,

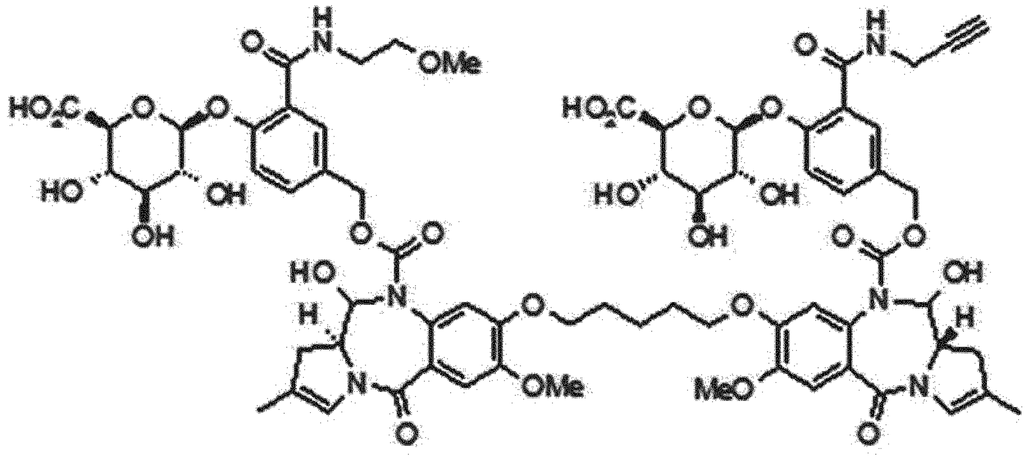

,

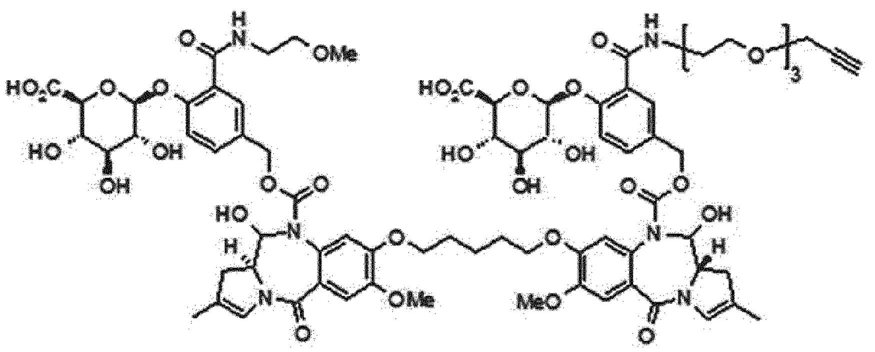

,

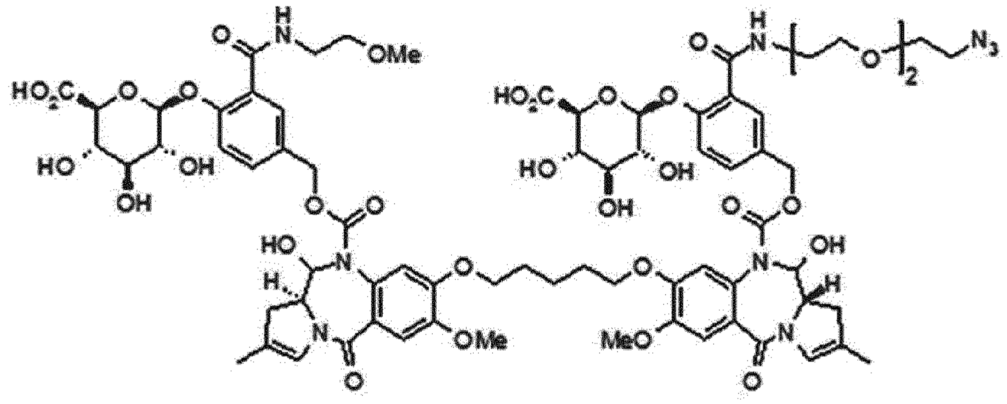

,

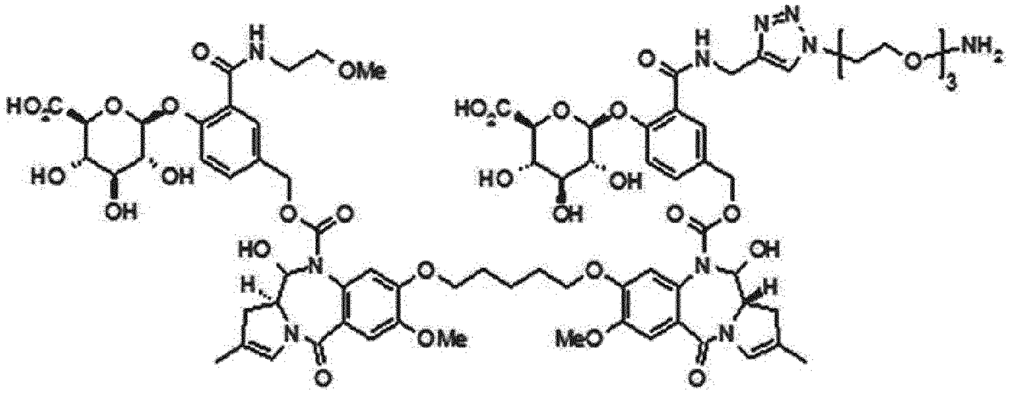

-

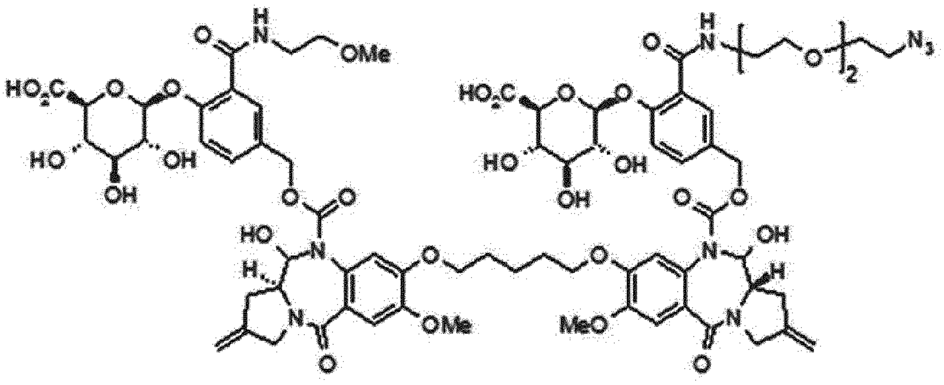

,

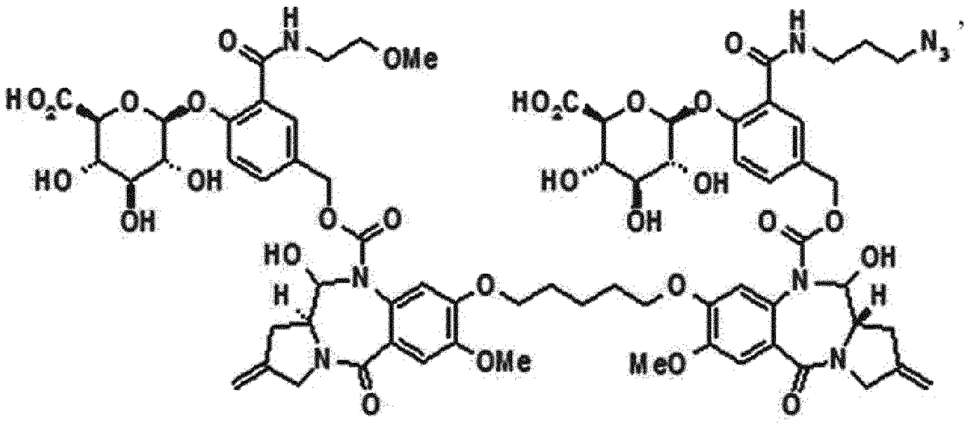,

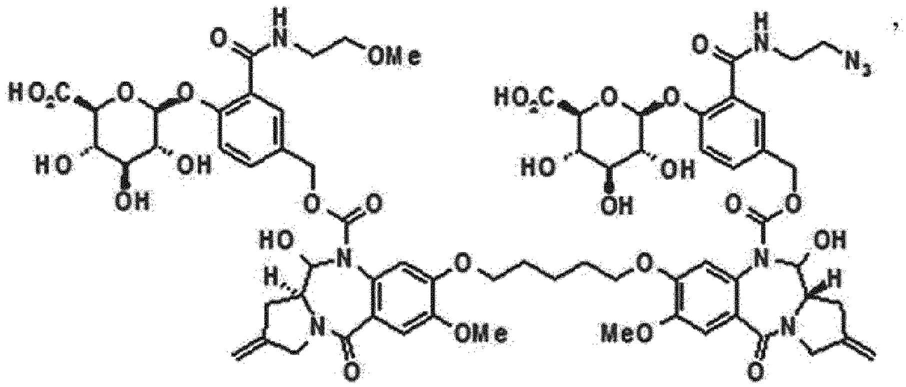,

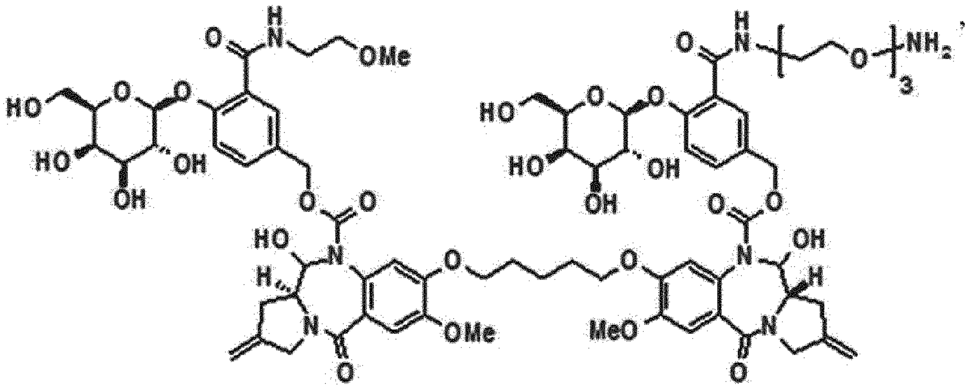,

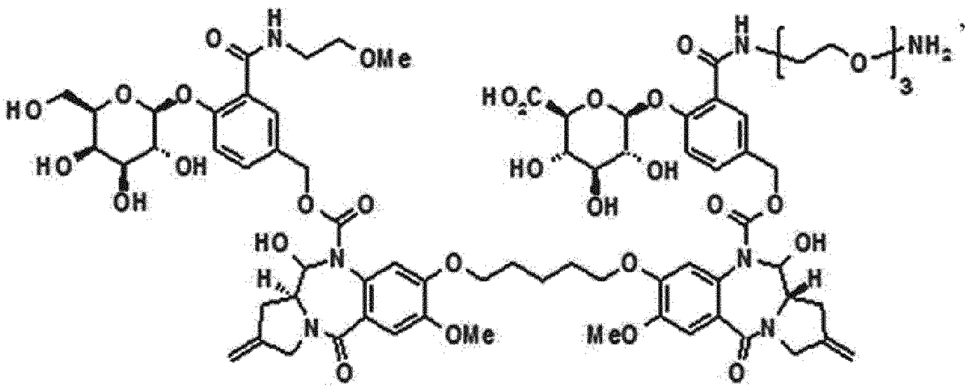,

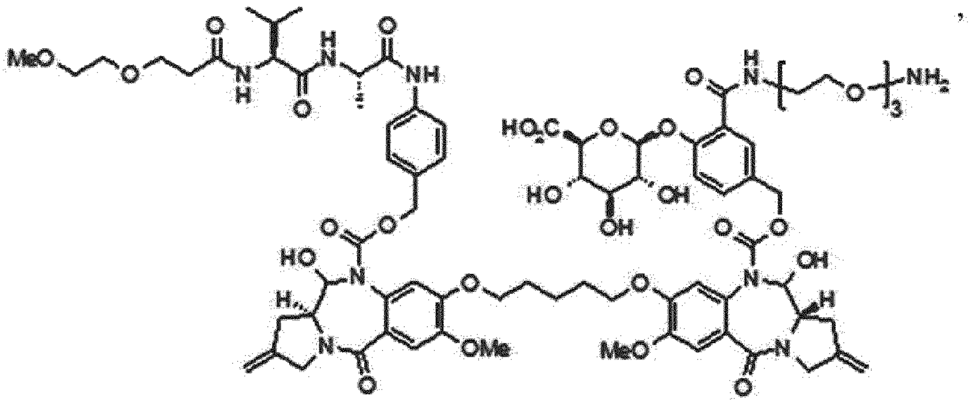

,

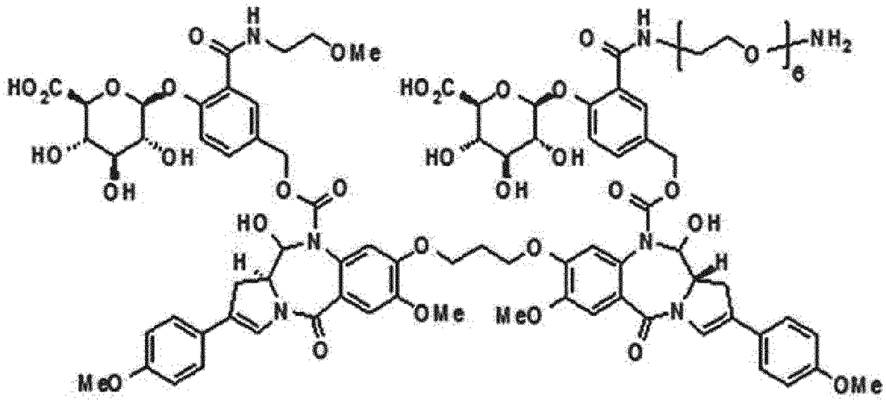

,

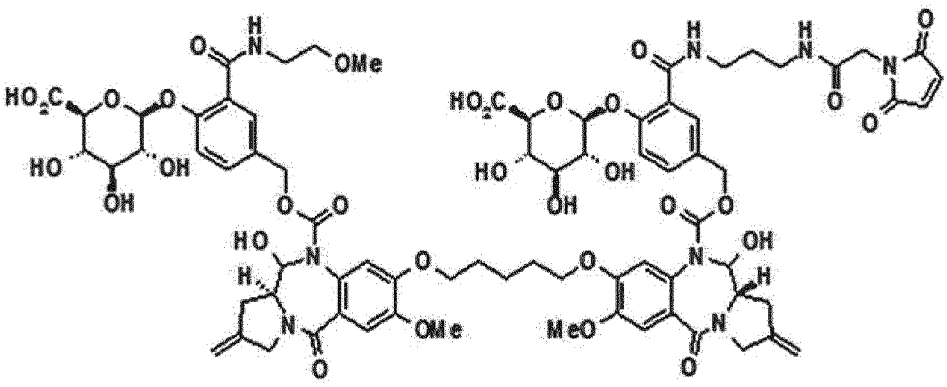

,

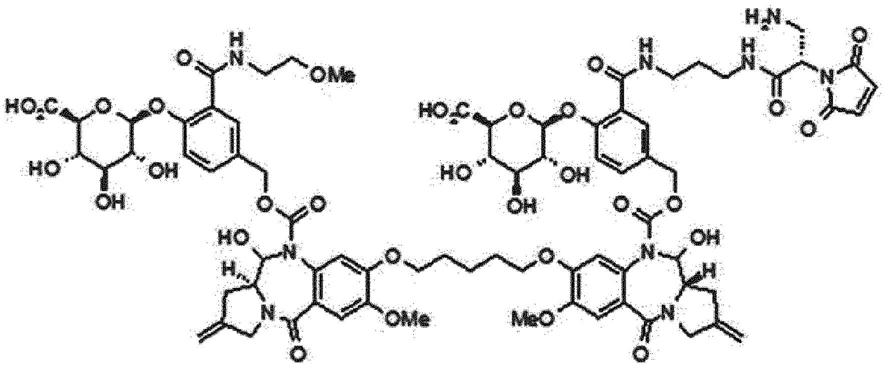

,

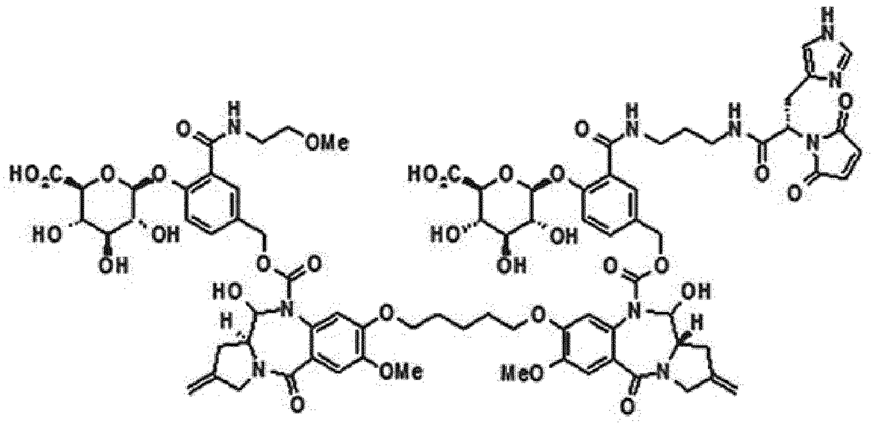

,

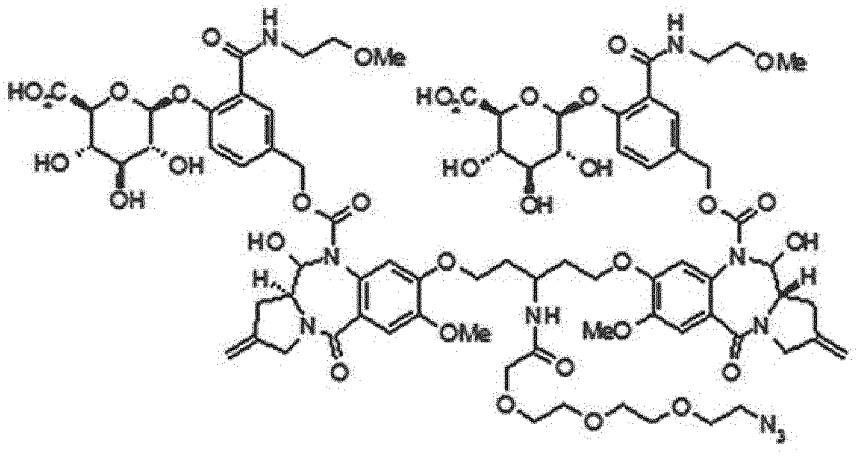

,

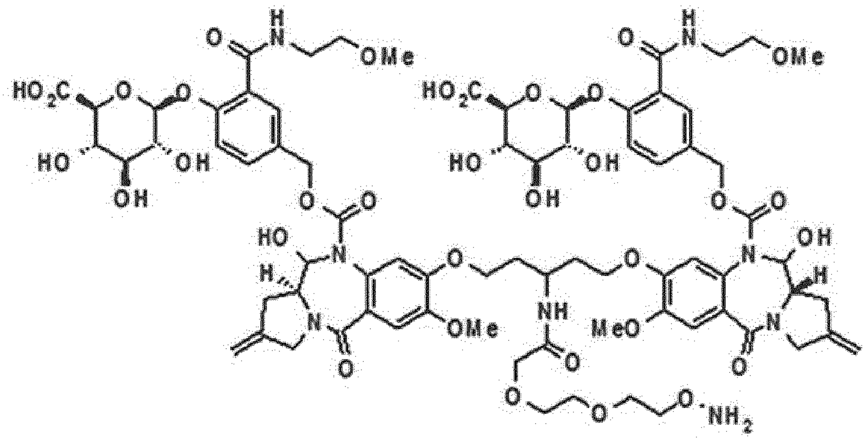

,

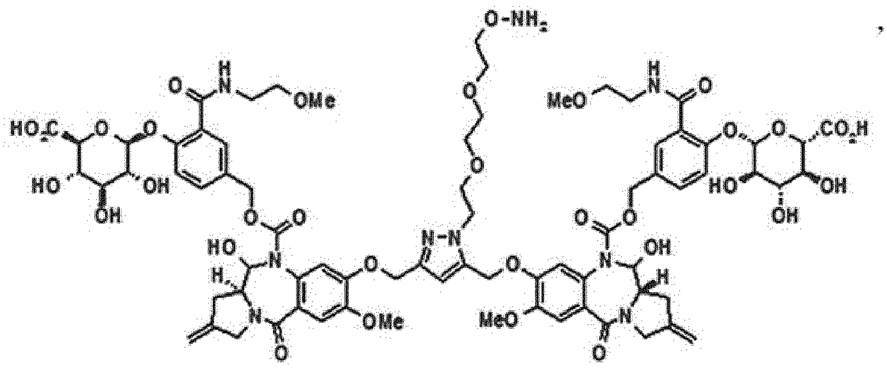

,

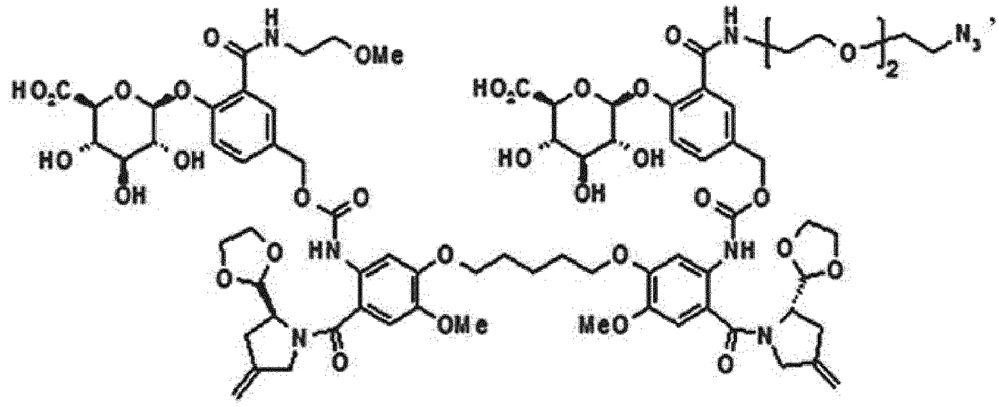
,

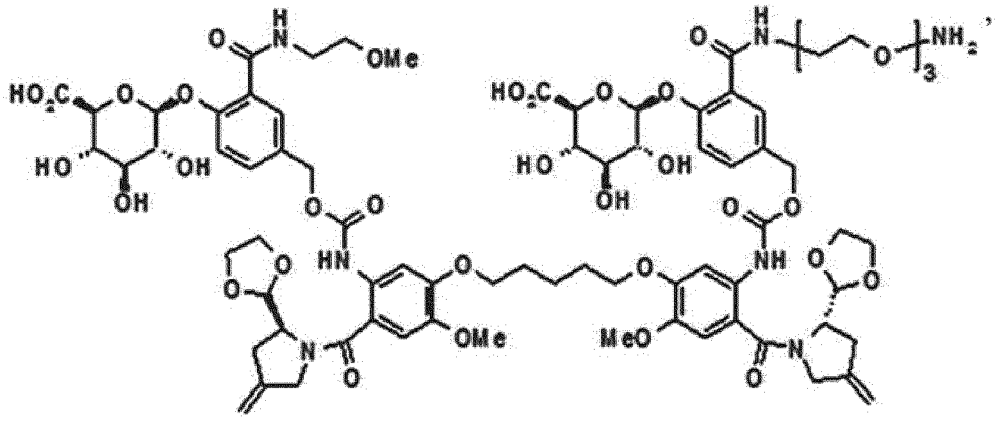
,

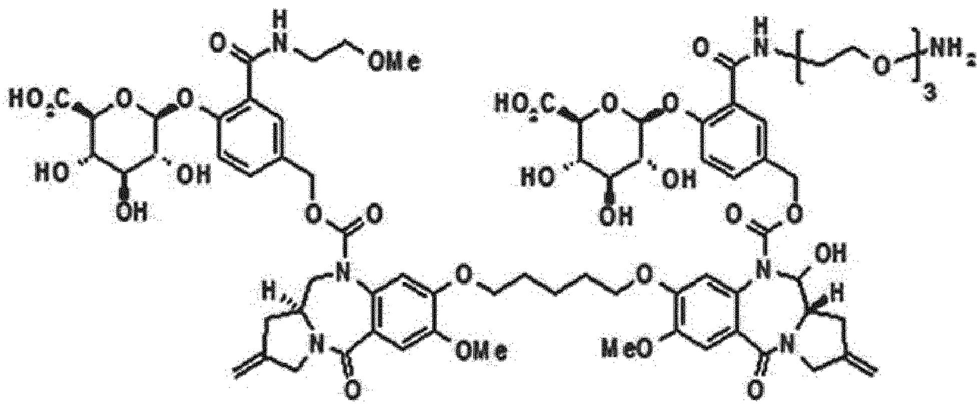

and

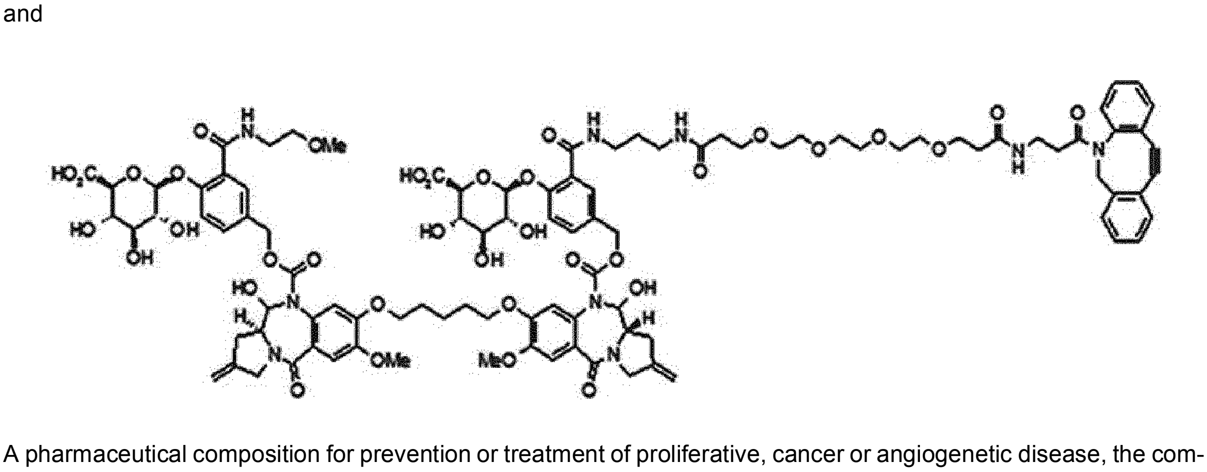

**57.** A pharmaceutical composition for prevention or treatment of proliferative, cancer or angiogenetic disease, the composition comprising the antibody conjugate of Claim 1.

**58.** The pharmaceutical composition of Claim 57, further comprising a pharmaceutically efficacious amount of chemotherapeutic agent.

**59.** The pharmaceutical composition of Claim 57, **characterized in that** the cancer is selected from the group comprising lung cancer, small cell lung carcinoma, gastrointestinal cancer, colon cancer, intestinal cancer, breast cancer, ovarian cancer, prostate cancer, testicular cancer, liver cancer, kidney cancer, bladder cancer, pancreatic cancer, brain cancer, sarcoma, osteosarcoma, Kaposi’s sarcoma and melanoma.

**60.** A method for prevention of proliferative, cancer or angiogenetic disease, the method comprising a step of administering an individual with a pharmaceutically efficacious amount of the conjugate of Claim 1.

**61.** A method for treatment of proliferative, cancer or angiogenetic disease, the method comprising a step of administering an individual with a pharmaceutically efficacious amount of the conjugate of Claim 1.

**62.** A use for the conjugate of Claim 1 as a pharmaceutical composition for prevention or treatment of proliferative, cancer or angiogenetic disease.

FIG. 1

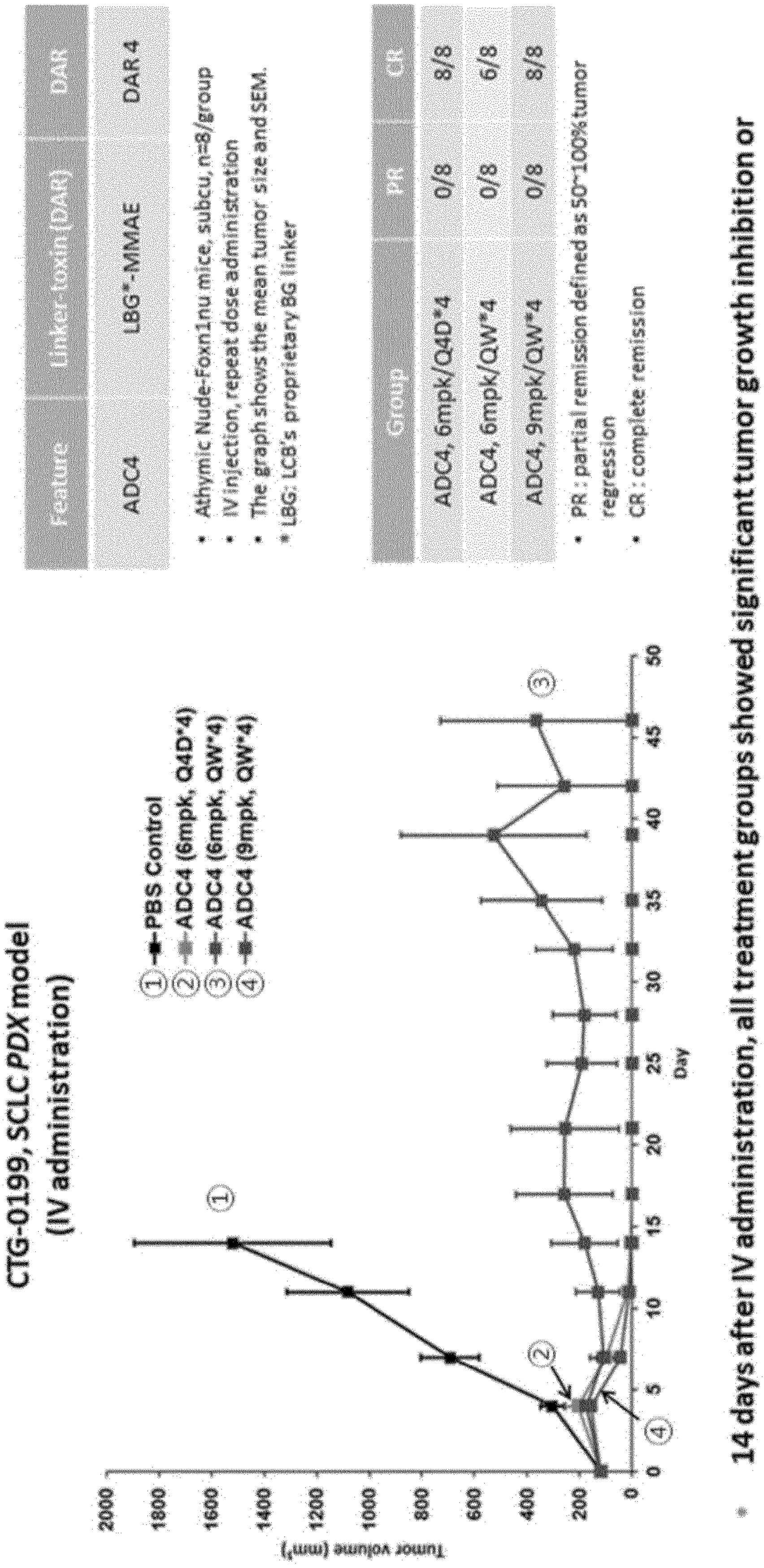

CTG-0199, SCLC PDX model
(IV administration)
① PBS Control
② ADC4 (6mpk, Q4D*4)
③ ADC4 (6mpk, QW*4)
④ ADC4 (9mpk, QW*4)
Tumor volume (mm³)
Day
Feature | Linker-toxin (DAR) | DAR
ADC4 | LBG*-MMAE | DAR 4
Athymic Nude-Foxn1nu mice, subcu, n=8/group
IV injection, repeat dose administration
The graph shows the mean tumor size and SEM.
* LBG: LCB's proprietary BG linker
Group | PR | CR
ADC4, 6mpk/Q4D*4 | 0/8 | 8/8
ADC4, 6mpk/QW*4 | 0/8 | 6/8
ADC4, 9mpk/QW*4 | 0/8 | 8/8
PR : partial remission defined as 50~100% tumor regression
CR : complete remission
14 days after IV administration, all treatment groups showed significant tumor growth inhibition or complete remission (CR)

## INTERNATIONAL SEARCH REPORT

International application No.
PCT/KR2020/003100

A. CLASSIFICATION OF SUBJECT MATTER

*A61K 47/68(2017.01)i, A61K 47/65(2017.01)i, A61K 31/5517(2006.01)i, A61P 35/00(2006.01)i, C07K 16/28(2006.01)i, A61K 45/06(2006.01)i*

According to International Patent Classification (IPC) or to both national classification and IPC

B. FIELDS SEARCHED

Minimum documentation searched (classification system followed by classification symbols)

A61K 47/68; A61K 31/5517; A61K 39/395; C07D 487/04; C07H 7/06; C07K 16/18; C07K 16/28; C12N 15/13; A61K 47/65; A61P 35/00; A61K 45/06

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal), STN (Registry, Caplus) & Keywords: DLK1, antibody-drug conjugate, heavy chain, light chain, linker

C. DOCUMENTS CONSIDERED TO BE RELEVANT

| Category* | Citation of document, with indication, where appropriate, of the relevant passages | Relevant to claim No. |
|---|---|---|
| A | KR 10-2019-0018400 A (LEGOCHEM BIOSCIENCES, INC. et al.) 22 February 2019<br>See claims 1-12; paragraphs [0304]-[0306], [0308], [0309], [0311]-[0318]. | 1-59,62 |
| A | KR 10-2012-0113175 A (KOREA RESEARCH INSTITUTE OF BIOSCIENCE AND BIOTECHNOLOGY) 12 October 2012<br>See claims 1-23; paragraph [0026]. | 1-59,62 |
| A | KR 10-2018-0110645 A (LEGOCHEM BIOSCIENCES, INC.) 10 October 2018<br>See claims 1-28. | 1-59,62 |
| A | KR 10-2009-0088893 A (LIVTECH INC.) 20 August 2009<br>See the entire document. | 1-59,62 |
| A | WO 2018-069490 A1 (MEDIMMUNE LIMITED) 19 April 2018<br>See the entire document. | 1-59,62 |
| PX | KR 10-2019-0028350 A (Y-BIOLOGICS INC.) 18 March 2019<br>See claims 1-4, 12, 13; paragraph [0106]. | 1-4,57-59,62 |

☐ Further documents are listed in the continuation of Box C.    ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 JUNE 2020 (24.06.2020) | **24 JUNE 2020 (24.06.2020)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**

International application No.
**PCT/KR2020/003100**

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **60, 61**
because they relate to subject matter not required to be searched by this Authority, namely:
Claims 60 and 61 pertain to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

INTERNATIONAL SEARCH REPORT
Information on patent family members

International application No.
PCT/KR2020/003100

| Patent document cited in search report | Publication date | Patent family member | Publication date |
|---|---|---|---|
| KR 10-2019-0018400 A | 22/02/2019 | WO 2019-035649 A1 | 21/02/2019 |
| KR 10-2012-0113175 A | 12/10/2012 | KR 10-1438265 B1 | 05/09/2014 |
| | | KR 10-1461706 B1 | 18/11/2014 |
| | | KR 10-2012-0113174 A | 12/10/2012 |
| | | US 2014-0072558 A1 | 13/03/2014 |
| | | WO 2012-138100 A2 | 11/10/2012 |
| | | WO 2012-138100 A3 | 10/01/2013 |
| | | WO 2012-138102 A2 | 11/10/2012 |
| | | WO 2012-138102 A3 | 10/01/2013 |
| KR 10-2018-0110645 A | 10/10/2018 | AU 2018-246806 A1 | 17/10/2019 |
| | | CA 3058360 A1 | 04/10/2018 |
| | | CN 109790171 A | 21/05/2019 |
| | | EP 3604311 A1 | 05/02/2020 |
| | | KR 10-2018-0110649 A | 10/10/2018 |
| | | WO 2018-182341 A1 | 04/10/2018 |
| KR 10-2009-0088893 A | 20/08/2009 | AU 2007-318483 A1 | 15/05/2008 |
| | | AU 2007-318483 B2 | 02/05/2013 |
| | | CA 2669731 A1 | 15/05/2008 |
| | | CA 2669731 C | 19/01/2016 |
| | | CN 101631802 A | 20/01/2010 |
| | | CN 101631802 B | 05/03/2014 |
| | | CN 103073641 A | 01/05/2013 |
| | | CN 103073641 B | 21/01/2015 |
| | | EP 2096122 A1 | 02/09/2009 |
| | | EP 2096122 B1 | 16/07/2014 |
| | | ES 2484842 T3 | 12/08/2014 |
| | | JP 5219827 B2 | 26/06/2013 |
| | | US 2009-0326205 A1 | 31/12/2009 |
| | | US 8227578 B2 | 24/07/2012 |
| | | WO 2008-056833 A1 | 15/05/2008 |
| WO 2018-069490 A1 | 19/04/2018 | AU 2017-343793 A1 | 18/04/2019 |
| | | AU 2017-343793 B2 | 10/10/2019 |
| | | BR 112019007612 A2 | 02/07/2019 |
| | | CA 3039832 A1 | 19/04/2018 |
| | | CL 2019000992 A1 | 23/09/2019 |
| | | CN 109843335 A | 04/06/2019 |
| | | CO 2019003709 A2 | 28/06/2019 |
| | | EA 201990615 A1 | 30/08/2019 |
| | | EP 3525830 A1 | 21/08/2019 |
| | | JP 2020-503250 A | 30/01/2020 |
| | | JP 6678821 B2 | 08/04/2020 |
| | | KR 10-2019-0064619 A | 10/06/2019 |
| | | MX 2019004292 A | 14/10/2019 |
| | | US 2019-0282705 A1 | 19/09/2019 |
| | | ZA 201902081 B | 27/11/2019 |

Form PCT/ISA/210 (patent family annex) (January 2015)

**INTERNATIONAL SEARCH REPORT**
Information on patent family members

International application No.
**PCT/KR2020/003100**

| Patent document cited in search report | Publication date | Patent family member | Publication date |
|---|---|---|---|
| KR 10-2019-0028350 A | 18/03/2019 | WO 2019-050362 A2<br>WO 2019-050362 A3 | 14/03/2019<br>02/05/2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1628872
- WO 8810649 A
- WO 88106630 A
- WO 8807085 A
- WO 8807086 A
- WO 8809344 A
- US 4816567 A
- KR 1020180107639
- US 2016063564 W
- US 2016063595 W
- WO 2017089895 A
- KR 1020180110645
- KR 1020190000514
- KR 1020140035393


### Non-patent literature cited in the description


- *Discovery Medicine,* 2010, vol. 10 (53), 329-39
- **HOLLINGER ; HUDSON.** *Nature Biotechnology,* 2005, vol. 23 (9), 1126-1136